# EUROPEAN PATENT APPLICATION

(11) **EP 3 909 944 A1**
(43) Date of publication of application: **17.11.2021**
(21) Application number: 20174828.2
(22) Date of filing: 14.05.2020
(51) Int. Cl.: C07D 257/08, C07F 7/22, A61K 51/00

(54) **NUCLIDE LABELLED H-TETRAZINES AND USE THEREOF FOR PET AND SPECT PRETARGETED IMAGING AND RADIONUCLIDE THERAPY**

(71) Applicant: Rigshospitalet, 2100 Copenhagen Ø (DK); University of Copenhagen, 1165 Copenhagen K (DK)
(72) Inventor: KJÆR, Andreas, 2100 Copenhagen Ø (DK); HERTH, Matthias Manfred, 1165 Copenhagen K (DK); BATTISTI, Umberto Maria, 1165 Copenhagen K (DK); GARCIA, Rocio, 1165 Copenhagen K (DK)
(74) Representative: Budde Schou A/S

(57) **Abstract**

The present invention relates to novel tetrazine compounds for use in pretargeted in vivo imaging and in therapy and to the precursors of the tetrazine compounds. The compounds are suitable for use in click chemistry, i.e. reactions that join a targeting molecule and a reporter molecule. The compounds comprise a radionuclide of F, I or At and on or more polar groups providing that the compounds can efficiently react with extracellularly located pretargeting vectors and as such used for example for pretargeted cancer diagnostics and cancer therapy.

## Description

### FIELD OF THE INVENTION

The present invention is within the field of bioorthogonal chemistry and relates to novel tetrazine compounds for use in pretargeted in vivo imaging and therapy. The compounds are suitable for use in click chemistry, i.e. reactions that join a targeting molecule and a reporter molecule. The compounds comprise a covalently bound radionuclide of F, I or At and are high polar compounds that will not enter cell membranes and are thus particularly useful in relation to cancer diagnostics and cancer therapy using non-internalizing pretargeting vectors.

### BACKGROUND OF THE INVENTION

Click chemistry has emerged as a versatile tool for pretargeted imaging, radiotherapy and recently also for specific drug release in vivo. Click chemistry is of particular interest in bioorthogonal chemistry.

Bioorthogonal chemistry refers to any chemical reaction that can occur inside living systems without interfering with native biochemical processes. A pretargeting strategy makes use of bioorthogonal chemistry and proceeds in two steps. A first step is where a substrate is modified with a bioorthogonal functional group (denoted chemical reporter or target vector) and introduced to the patient. Normally, a substrate can be a metabolite, an enzyme inhibitor, monoclonal antibody, nanomedicine, polymer, nanoparticle, etc. The second step is where a probe, that contains the complementary functional group, is introduced and reacts and labels the substrate. The probe is a small effector molecule carrying the label.

Recently, bioorthogonal chemistry has emerged as a versatile tool for nuclear pretargeted imaging of slow accumulating targeting vectors (nanomedicines) such as monoclonal antibodies (mAbs) or other nanomedicines.^{[1]} Improved target-to-background ratios and lower radiation burden to healthy tissue can be reached using pretargeting compared to conventional targeting strategies. These improved target-to-background ratios are a result of the temporal separation of the slow targeting process of nanomedicines from the actual imaging or therapeutic step. Consequently, the exquisite target specificity of nanomedicines as well as the optimal pharmacokinetics of small molecules for molecular imaging, e.g. rapid target accumulation and blood clearance, can be exploited using pretargeted imaging.^{[2,3]} So far, the most prominent reaction for pretargeted imaging is the tetrazine ligation. Excellent chemoselectivity, metabolic stability, high reaction rates and low toxicity make this ligation exceptional.^{[4,5]} The tetrazine ligation itself is based on an Inverse-Electron-Demand Diels-Alder (IEDDA) cycloaddition reaction followed by a retro-Diels-Alder elimination of nitrogen between an electron-deficient tetrazine (Tz) and often a strained trans-cyclooctene (TCO) derivative.^{[6,7]} Many efforts have been made in recent years to utilize the tetrazine ligation for pretargeted PET imaging or radionuclide therapy. Initially, TCOs have been used to develop a ¹⁸F-PET pretargeting imaging agent. However, the rapid plasma isomerization of TCOs make them non-ideal.^{[2]} Thus, the labeling of Tzs gradually emerged as a more feasible approach.^{[3]}

Positron-Emission-Tomography (PET) and Single-photon emission computed tomography (SPECT) are powerful and routinely used non-invasive imaging tools in precision medicine or drug development.^{[4]} Its high sensitivity and isotropism are in combination unmatched compared to any other in vivo molecular imaging technique.^{[5]} Fluorine-18 (¹⁸F) is considered as the "gold standard" PET radionuclide for clinical applications as it provides almost ideal physical characteristics for PET molecular imaging. A relatively short positron range (2.4 mm max. range in water), a good branching ratio (96.7 % positron decay) and a half-life of approx. 110 min results in good resolution, a relatively low radiation burden for patients and in the ability to distribute a ¹⁸F-labeled tracer within a several hundred kilometers range.^{[12, 13]} The PET radionuclide Iodine-124 (¹²⁴I) possesses a half-life of 4.18 d and allows as such for longer timeframes. Iodine-123 (¹²³I) is a standardly used radionuclide for SPECT and is as such useful for SPECT imaging. Recently, targeted radionuclide therapy with alpha-emitters has emerged as a versatile and effective tool to treat cancers, even micrometastases, one of the main reasons of reoccurrence of cancer after initial treatment. Astatine-211 (²¹¹At) is one of the most interesting radionuclide in this respect since it can be simultaneously imaged and per decay only one alpha-particle is emitted and not several as it is the case for many other alpha-emitters such as ²²⁵Ac or ²¹³Bi.

Labeling approaches using radiometals such as ¹¹¹In, ⁶⁴Cu, ⁸⁹Zr, ⁴⁴Sc, ²¹²Pb, ²²⁵Ac, ²¹³Bi or ⁶⁸Ga have been used for pretargeting strategies within the last decade. ^{[14-17]} It would, however, be useful if labeling approaches for other radionuclides such as ¹⁸F, ¹²³I, ¹²⁴I, ¹³¹I and ²¹¹At could be provided. These radionuclides are of particular interest because they are covalently bound to their targeting molecule and allow as such for a bigger flexibility in respect to finetuning the pharmacokinetic profile of the radiopharmaceutical. Size, lipophilicity and rate constants can easily be manipulated. Moreover, from a synthetic point of view, all radionuclides can be introduced from the same or similar precursors and as such, a theranostic pair is easily accessible. A theranostic pair in the context of radiopharmacy allows to accompany a therapy with a diagnosis with the aim of a patient-specific treatment. In this respect, a diagnostic radiopharmaceutical can be used to adequately estimate the effectiveness of a therapeutic radiopharmaceutical and allows dose estimation and to determine the maximum tolerated dose.

In 2013, the first successful attempt to label a Tz moiety with a covalently bound PET radionuclide, i.e. with carbon-11, was reported by the groups of the present inventors.^{[7]} Despite the great progresses in the field, no highly reactive ¹⁸F-Tzs were reported until recently, most likely due to the insufficient stability of Tzs during standard aromatic nucleophilic ¹⁸F-fluorination conditions.^{[19-22]} Only relatively base insensitive and less reactive Tzs with rate constants of approx. 86 M⁻¹ s⁻¹ (measured in 1,4-dioxane, 37 °C) could be radiolabeled via an ¹⁸F-aliphatic substitution (S_{N}2) strategy. Radiochemical yields (RCYs) up to 18% were reached.^{[8]} More recently, the preparation of a highly reactive ¹⁸F-labeled glycosylated Tz by Keinänen et al. and an Al[¹⁸F]NOTA-labeled Tz radioligand by Jan-Philip et al. were reported.^{[19, 21]} The last strategy described exploited the azide-alkyne Huisgen cycloaddition to efficiently label highly reactive Tzs.^{[9]} However, none of the aforementioned ¹⁸F-labeling procedures seem optimal for clinical applications, since multi-step procedures are usually challenging to set up for clinical routine and Al¹⁸F-labeling procedures are still in its infancy in respect to its scalability.

The most exploited reaction for the synthesis of radiolabeled aryl fluorides is the Nucleophilic Aromatic Substitution reaction (S_{N}Ar). Typically, this type of reaction requires relatively basic conditions and high temperature and as such, they are not ideally applicable to ¹⁸F-label structures containing Tz moieties. ^{[20, 23]} Recently, however, several milder aromatic ¹⁸F-labeling strategies have been reported that proceed faster, at lower basicity as well as at lower temperatures. Especially, Cu-mediated oxidative fluorination of tin- and boronic ester/acid species ^{[24-27]}, concerted Nucleophilic Aromatic Substitution of uronium or iodonium salts ^{[28-30]}, hypervalent iodononium based precursors ^{[11]} and minimalistic labeling strategies have proven their potential in this respect.^{[30, 32]}.

For the above reasons, it would be beneficial to develop a simple, scalable and reliable direct aromatic radionuclide labeling procedure that can be applied to label highly reactive Tzs providing radionuclide-Tz based pretargeted imaging agents with fast reaction kinetics, good metabolic stability and favorable pharmacokinetic profile needed for bioorthogonal chemistry and with the possibility for being provided in clinically relevant amounts.

Iodine labeling and ²¹¹At-radiolabeling of tetrazines have been successfully carried out.^{[33-35]} None of these tetrazines were applied to pretargeted strategies since their lipophilicity is too high for any reasonable in vivo ligation, at least in any standardly applied pretargeting tumor model.

Thus, a direct radionuclide labeling strategy with radionuclides such as ¹⁸F, ¹²³I, ¹²⁴I, ¹³¹I and ²¹¹At for highly reactive tetrazines suitable for pretargeted approaches in vivo would be greatly beneficial if not essential for clinical translation of such labeled tetrazines.

### SUMMARY OF THE INVENTION

Accordingly, in a first aspect, the present invention relates to tetrazine compounds having the following formula I: 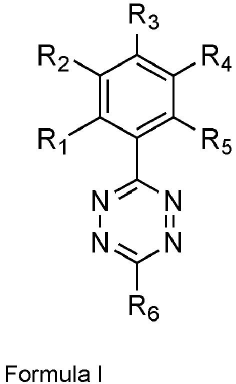 wherein R₁-R₅ are independently selected from: a radionuclide selected from ¹⁸F, ¹²³I, ¹²⁴I, ¹³¹I or ²¹¹At; one or more group(s) providing a lipophilicity of clogD_{7.4} < -0.5 to the compound of Formula I independently selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine; and
wherein R₆ is H;
wherein one of R₁-R₅ is a radionuclide and one or more of the remaining R₁-R₅ is a/are group(s) providing a lipophilicity of clogD_{7.4} < - 0,5, and any R₁-R₅ remaining thereafter is/are H.

In some embodiments, the radionuclide is ¹⁸F, situated at position R₁, R₂, R₃, R₄ or at R₅.

In other embodiments, the radionuclide is ¹²³I, situated at position R₁, R₂, R₃, R₄ or at R₅.

In other embodiments, the radionuclide is ¹²⁴I, situated at position R₁, R₂, R₃, R₄ or at R₅.

In other embodiments, the radionuclide is ¹³¹I situated at position R₁, R₂, R₃, R₄ or at R₅.

In other embodiments, the radionuclide is ²¹¹At situated at position R₁, R₂, R₃, R₄ or at R₅.

In a preferred embodiment, the radionuclide is situated at position R₄ in Formula I. Thus, in one preferred embodiment, the radionuclide is ¹⁸F, situated at position R₄. In another preferred embodiment, the radionuclide is ¹²³I, situated at position R₄. In another preferred embodiment, the radionuclide is ¹²⁴I, situated at position R₄. In another preferred embodiment, the radionuclide is ¹³¹I situated at position R₄. In another preferred embodiment, the radionuclide is ²¹¹At situated at position R₄.

The tetrazine compounds according Formula I comprises at least one group that provides the compounds according to Formula I with a lipophilicity of clogD_{7.4} < -0.5. It has been found that when the tetrazine compounds of Formula I have a sufficiently high polarity, they will reach pretargeting vectors which do no internalize. This is beneficial for their use as imaging agents, as diagnostic agents and as therapeutic agents in relation to certain diseased tissue in animals and humans, such as cancer tissue, cancerous cells, infected cells or pathogens.

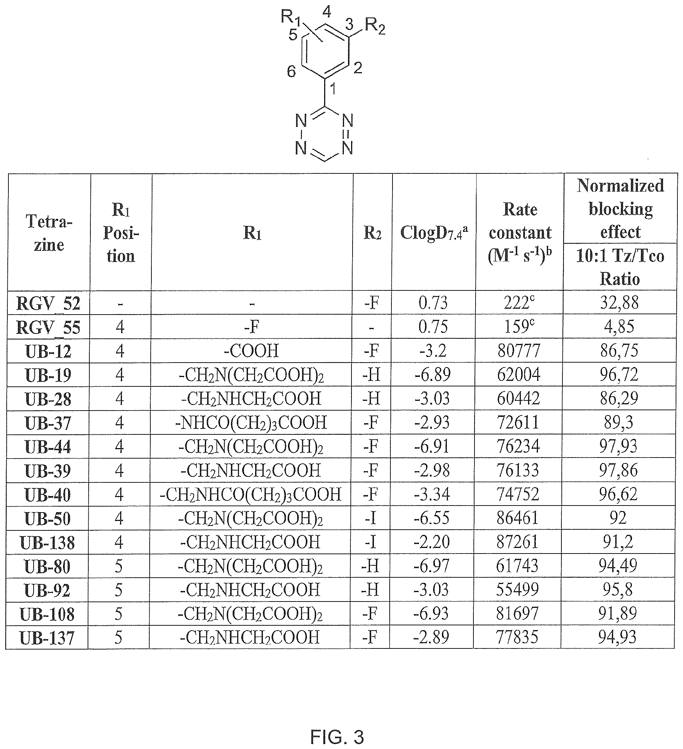

The lipophilicity of compounds of formula 1 suited for such purposes was found to have a lipophilicity clogD_{7.4} of approximately less than -0.5. As shown in Figure 3, the blocking effect increased the lower the lipophilicity value. Thus, the compounds of Formula I preferably have a lipophilicity clogD_{7.4} of less than -2. Even more preferred, the compounds of Formula I preferably have a lipophilicity clogD_{7.4} of less than less than -3 and most preferred, the compounds of Formula I have a lipophilicity clogD_{7.4} of less than -6.

In order for the compounds of Formula I to have a lipophilicity of clogD_{7.4} < -0.5 or less, one or more of R₁, R₂, R₃, R₄ or R₅ is a group that provides for such a polarity. It has been found that the tetrazines of Formula I will have a clogD_{7.4} value of -0.5 or less, when one or more of R₁, R₂, R₃, R₄ or R₅ is selected from -OH, NR₇R₈, CH₂N(CH₂COOH)₂, CH₂NHCH₂COOH, CH₂NRCH₂COOH, COOH, CONR₇R₈, SO₃H, SO₂NH₂, and SO₂NH, wherein R is H, CH₃, CH₂CH₃, CH₂CH₂CH₃ or CH₂COOH, R₇ is H, CH₃, CH₂CH₃, CH₂CH₂CH₃ or CH₂COOH; and R₈ is H, CH₃, CH₂CH₃, CH₂CH₂CH₃ or CH₂COOH.

In a preferred embodiment, one of R₂ or R₄ is CH₂N(CH₂COOH)₂, CH₂NHCH₂COOH, CH₂NRCH₂COOH or COOH wherein R is H, CH₃, CH₂CH₃, CH₂CH₂CH₃ or CH₂COOH; while the other is a radionuclide selected from ¹⁸F, ¹²³I, ¹²⁴I, ¹³¹I or ²¹¹At.

In preferred embodiments, the one or more group(s) providing a lipophilicity of clogD_{7.4} < -0.5 to the compound of Formula I is selected from the polar groups (PG):

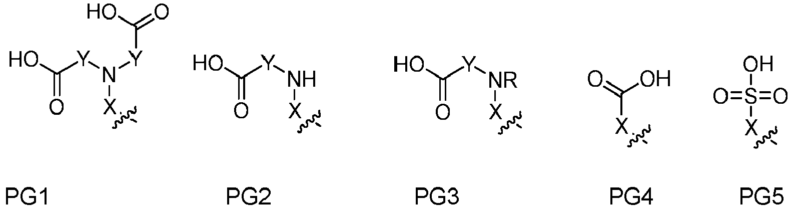

Wherein X is CO(CH₂)ₙ, SO₂(CH₂)ₙ, (CH₂)ₙ or (OCH₂CH₂)ₙ and n = 0, 1, 2 or 3; and wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)nOCH₂COOH and n = 0, 1, 2 or 3; and wherein the curly sign indicates the link to the aromatic ring. In a preferred embodiment, the compounds of Formula I is selected from the following compounds Ia or Ib: 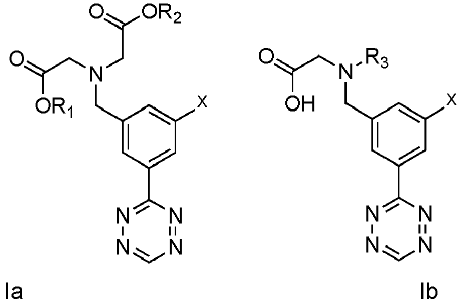 wherein X is selected from ¹⁸F, ¹²³I, ¹²⁴I, ¹³¹I or ²¹¹At, and R₁, R₂ and R₃ is independently selected from H, (CH₂)ₙCH₃, (OCH₂CH₂)ₙOH, CO(CH₂)ₙCOOH, (OCH₂CH₂)ₙOCH₂COOH and n = 0, 1, 2 or 3.

In another preferred embodiment, the compounds of Formula I is selected from the following compounds Ic, Id and Ie: 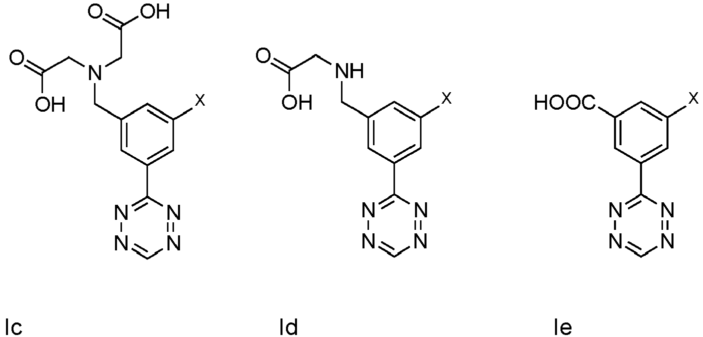 wherein X is selected from ¹⁸F, ¹²³I, ¹²⁴I, ¹³¹I or ²¹¹At.

In another aspect, the invention provides tetrazine precursors having the general formula II: 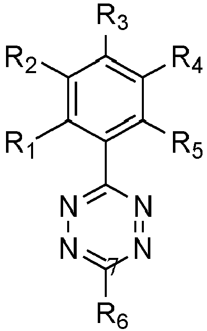 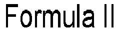 wherein R₁-R₅ are independently selected from the group consisting of: SnR₃, B(OR)₂, I⁺-Ar, I double-bonded to R or SiR₃, wherein R is a linear or branched C1-C6 alkyl, cyclic C1-C6 alkyl, optionally substituted with -OH, -NH₂ or halogen; a group providing a lipophilicity of clogD_{7.4} < -0.5 to the compound of Formula II selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, an hydroxy group, a sulfonamide, a carboxyl group, a sulfonyl group, amine, a substituted amine with 1-5 polyethylene glycol unit(s), a -(O-CH₂-CH₂)₁₋₅-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, and
wherein R₆ is a H; and
wherein one of R₁-R₅ is selected from the group consisting of: SnR₃, B(OR)₂, I⁺-Ar, I double-bonded to R or SiR₃; and one or more of the remaining R₁-R₅ is a/are group(s) providing a lipophilicity of clogD_{7.4} < - 0,5; and any R₁-R₅ remaining thereafter is/are H.

All the precursors of formula II can be used to provide the compounds of formula I. However, some precursors are more suited than others to provide the compound of formula I in high efficiency depending on the specific radionuclide.

In the most preferred embodiments, one of R₁-R₅ in the tetrazine precursor of formula II is SnR₃ or B(OR)₂. These precursors are suitable for providing all compounds according to formula I regardless of the specific radionuclide selected.

In one preferred embodiment, when one of R₁-R₅ in the precursor of formula II is SiR₃ the radionuclide in the final compound of formula I provided by this precursor is ²¹¹At.

In another preferred embodiment, when one of R₁-R₅ in the precursor of formula II is I⁺-Ar, I double-bonded to R, the radionuclide in the final compound of formula I provided by this precursor is ¹⁸F.

In preferred embodiments, when one of R₁-R₅ in the precursor of formula II is SnR₃ or SiR₃, R is a linear methyl, ethyl, propyl or butyl and all R's are the same.

In preferred embodiments the tetrazine precursor of Formula II the SnR₃, B(OR)₂, I⁺-Ar, I double-bonded to R (I=R) or SiR₃, wherein R is a linear or branched C1-C6 alkyl, cyclic C1-C6 alkyl, optionally substituted with -OH, -NH₂ or halogen is situated at position R₄, and one or more of the remaining R₁-R₅ is a/are group(s) providing a lipophilicity of clogD_{7.4} < - 0,5; and any R₁-R₅ remaining thereafter is/are H.

In preferred embodiments, the tetrazine precursor of Formula II is selected from the following structures: 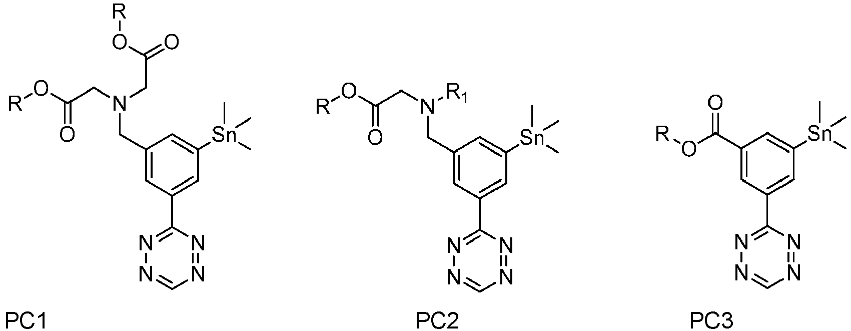 wherein the protective group R is selected from butyl groups, methylthiomethyl (MTM) groups, tetrahydropyranyl (THP) groups or benzyloxymethyl (BOM) esters, and R₁ is selected from a Boc group, trityl group, acetamide, carbamate group, (CH₂)ₙCH₃, (OCH₂CH₂)ₙOH, CO(CH₂)ₙCOOtBu, (CH₂CH₂O)ₙCH₂CO(CH₂)ₙCOOtBu wherein n = 0, 1, 2 or 3.

In another preferred embodiment, the tetrazine precursor of Formula II is selected from the following structures: 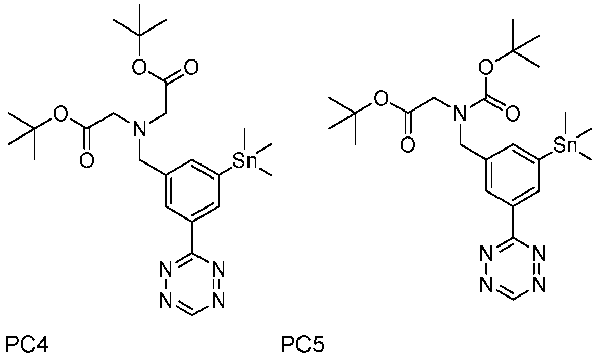 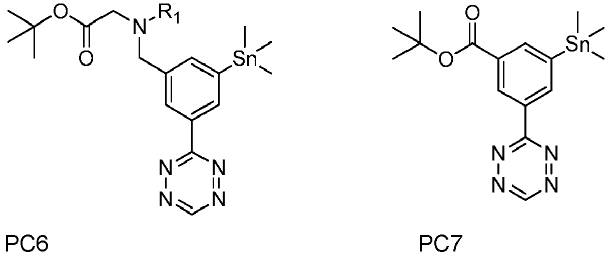 wherein R₁ is selected from a Boc group, trityl group, acetamide, carbamate group, (CH₂)ₙCH₃, (OCH₂CH₂)ₙOH, CO(CH₂)ₙCOOtBu, or (CH₂CH₂O)ₙCH₂CO(CH₂)ₙCOOtBu wherein n = 0, 1, 2 or 3.

As shown herein, the tetrazine compounds of Formula I A compound are excellent for use in bioorthogonal chemistry. All if the tetrazines of Formula I tested showed a TCO click ability close to 100%.

It is moreover shown in the present Examples, that the compounds of Formula I provides very good imaging results in mice being infested with a human cancer type as shown with PET scanning. The applicability of the tetrazine compounds of Formula I in various imaging techniques such as PET and SPECT will depend on the specific radionuclide selected. Accordingly, in one aspect of the invention, the compounds of Formula I is for use in biorthogonal chemistry for pretargeted strategies.

In another aspect of the invention, the compounds of Formula I is for use in diagnostics.

In a preferred embodiment, the diagnostics is cancer diagnostics. The cancer can be any kind of cancer.

In one aspect of the invention, the radionuclide of the tetrazine derivative of Formula I is ¹⁸F or ¹²⁴I use in PET imagining.

In one aspect of the invention, the radionuclide of the tetrazine derivative of Formula I is ¹²³I for use in SPECT imagining.

In a preferred embodiment, the PET or SPECT imaging according to the above is of cancer tissue.

In one aspect of the invention, the radionuclide of the tetrazine derivative of Formula I is ²¹¹At or ¹³¹I for use in radionuclide therapy.

In a preferred embodiment of that aspect, the radionuclide therapy is of a cancer disease.

In another preferred embodiment of that aspect, the radionuclide therapy is applied to kill extracellular pathogens or any kind of unwanted cell, virus, microorganism which is extracellularly deposited.

Due to the one or more polar groups in Formula I, these compounds can be uses in imaging, diagnostics and therapy wherein it is desired that the compound does not penetrate cell membranes.

Another aspect of the invention provides a method for preparing a compound of Formula I as detailed in general procedure a, b, c and d.

In one embodiment the method comprises reacting 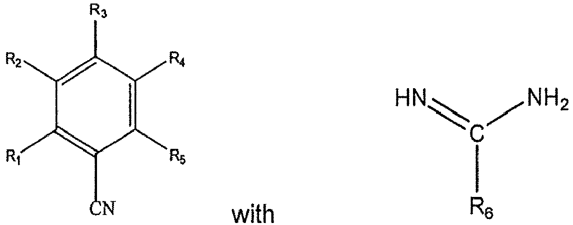 wherein R₁-R₅ are independently selected from the group consisting of: SnR₃, B(OR)₂, I⁺-Ar, I double-bonded to R or SiR₃, wherein R is a linear or branched C1-C6 alkyl, cyclic C1-C6 alkyl, optionally substituted with -OH, -NH₂ or halogen; one or more group(s) providing a lipophilicity of clogD_{7.4} < -0.5 to the compound of Formula II selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy,
(C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine; with 1-5 polyethylene glycol unit(s), a -(O-CH₂-CH₂)₁₋₅-OCH₂-COOH, H, Methyl, Ethyl, Propyl, optionally substituted heteroaryl,and optionally substituted arylalkyl; wherein optionally substituted in relation to said substituted amine means one or more substituents selected from a halogen, a hydroxy group, a sulfonamide, a carboxyl group, a sulfonyl group, amine,
and wherein R6 is a H;
and wherein one of R₁-R₅ is selected from: SnR₃, B(OR)₂, I⁺-Ar, I double-bonded to R or SiR₃; and one or more of the remaining R₁-R₅ is a/are group(s) providing a lipophilicity of clogD_{7.4} < - 0,5; and any R₁-R₅ remaining thereafter is/are H.

In another preferred embodiment, the method comprises reacting 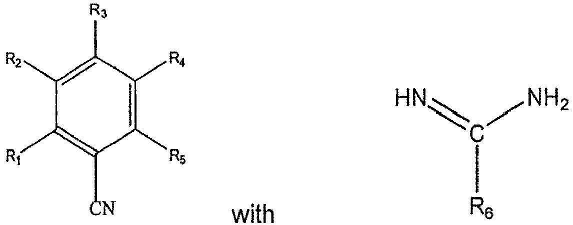 wherein R₁-R₅ are independently selected from I or F and one or more group(s) providing a lipophilicity of clogD_{7.4} < -0.5 to the compound of Formula II, selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene,
(C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, and wherein R6 is a H,
and wherein one of R₁-R₅ is selected from: I or F; and one or more of the remaining R₁-R₅ is a/are group(s) providing a lipophilicity of clogD_{7.4} < - 0,5; and any R₁-R₅ remaining thereafter is/are H

In a preferred embodiment, the method is carried out at a temperature range of from 50° to 70°C.

In another preferred embodiment, the method is carried out by adding water after cooling to room temperature followed by addition of HCI and extraction with EtOAc.

### BRIEF DESCRIPTION OF THE DRAWINGS

Figure 1 Scheme showing the principle of labelling tetrazines with ¹⁸F, ¹²³I, ¹²⁴I, ¹³¹I or ²¹¹At
Figure 2 Scheme showing labeling of Tz-1 as a model compound and table with optimization data
Figure 3 Scheme showing the ability of ¹⁹F-tetrazines to block ¹¹¹In-DOTA-tetrazine (Q)
Figure 4 Structure of compound ¹⁸F-UB108, ¹⁸F-UB137 and ¹¹¹In-DOTA-tetrazine (Q)
Figure 5 Scheme showing the synthesis UB108 and UB137
Figure 6 Synthesis of the tin-precursor of UB108 and UB137and radiolabeling of ¹⁸F-UB108 and ¹⁸F-UB137
Figure 7 NMR of UB108
Figure 7a NMR of UB108 precursor (UB161)
Figure 8 NMR of UB137
Figure 8a NMR of UB137 precursor (UB266)
Figure 9 HPLC of reference compound UB108 and radio-HPLC of purified ¹⁸F-UB108.
Figure 10 HPLC of reference compound UB108 and radio-HPLC of purified ¹⁸F-UB137.
Figure 11 UB-108 click-ability to a TCO derivative
Figure 11a UB-137 click-ability to a TCO derivative
Figure 12 PET scan and biodistribution of ¹⁸F-UB108 one 1h after injection of pretreated tumor bearing mice with TCO-CC49 three days before ¹⁸F-UB108 injection
Figure 13 PET scan and biodistribution of ¹⁸F-UB137 one 1h after injection of pretreated tumor bearing mice with TCO-CC49 three days before ¹⁸F-UB137 injection.

### DETAILED DESCRIPTION OF THE INVENTION

The present invention provides tetrazine compounds labelled with a ¹⁸F, ¹²³I, ¹²⁴I, ¹³¹I or ²¹¹At radionuclide and having one or more polar groups that provide a H-tetrazine compound with a lipophilicity of clogD_{7.4} < -0.5. This lipophilicity is needed to obtain reasonable tumor accumulation (normalized blocking effect > 70), in standardly applied tumor models for pretargeted strategies. A simple, scalable and reliable direct labeling method for labeling tetrazines with the ¹⁸F, ¹²³I, ¹²⁴I, ¹³¹I or ²¹¹At is also provided together with tin- or boronic species precursors or silyl, iodonium or ylide precursors, especially for ²¹¹At labeling. The tetrazine compounds of the invention are shown to be suitable for use in biorthogonal chemistry including imaging-based diagnostics, such as PET and SPECT and in radionuclide therapy. Due to the high polarity of the tetrazine compounds disclosed herein, the tetrazine compounds cannot penetrate cell membranes and are thus particularly suitable for pretargeted strategies of non-internalizing pretargeting vectors. The tetrazine compounds of the invention display characteristics in mice that qualifies their use in one or more of PET imaging, SPECT imaging and radionuclide therapy in humans.

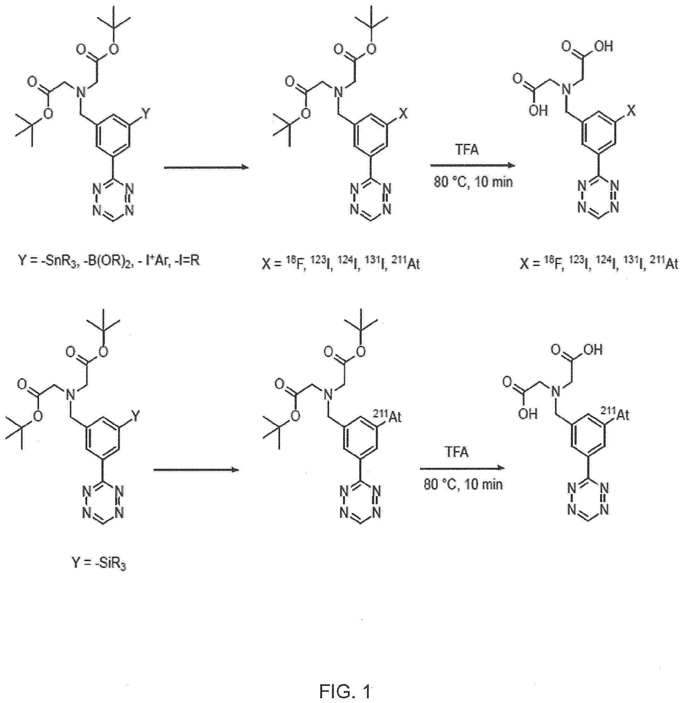

The H-tetrazine compounds labelled with a ¹⁸F, ¹²³I, ¹²⁴I, ¹³¹I or ²¹¹At radionuclide and having one or more polar groups that provide the tetrazine compound with a lipophilicity of clogD_{7.4} < -0.5. The compounds as disclosed herein were prepared from tin- and boronic precursors or silyl, iodonium or ylide precursors and utilized to label tetrazines with ¹⁸F, ¹²³I, ¹²⁴I, ¹³¹I or ²¹¹At. The principle of the labelling is shown in Figure 1.

In the context of the present invention, an "H-tetrazine" is defined as: 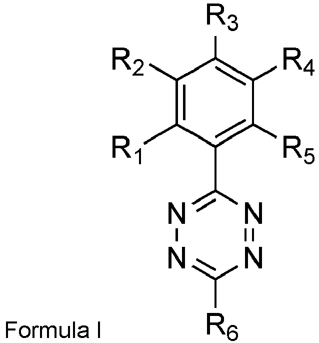 wherein one of R₁-R₅ is a radionuclide selected from ¹⁸F, ¹²³I, ¹²⁴I, ¹³¹I or ²¹¹At; and
wherein one or more R₁-R₅ is a group providing a lipophilicity of clogD_{7.4} < -0.5 to the compound of Formula I; and wherein the remaining R₁-R₅ are H; and R₆ H.

The "H" in H-tetrazine refers to the H at position R6 in formula I.

It was found that compounds of formula I having a high polarity as determined by their lipohilicity value clogD_{7.4} (Figure 3) provide high blocking effects in tumor blocking assays (Fig 3). These compounds have rate constants of approximately 50.000-90.000 M⁻¹ s⁻¹ (determined with standard TCO at 37 °C in PBS).

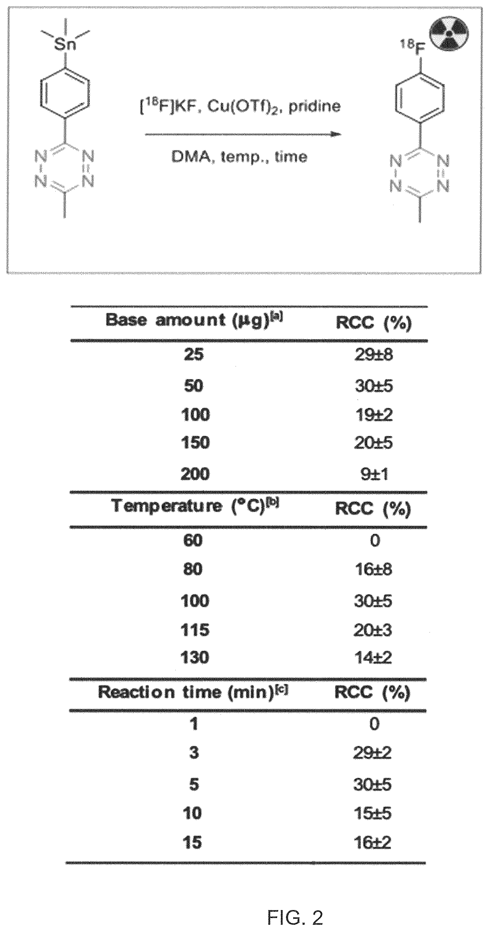

Tz 1 (Figure 2) was selected as a model compound for ¹⁸F-labeling, due its easy accessibility and good stability against hard bases. Precursors and reference compounds were synthesized as described in Examples 1 to 7. It was found that commonly used ¹⁸F-labeling strategies resulted in decomposition products. On the contrary, for the Cu-mediated radionuclide reaction through the stannate precursor (Figure 2), labeling attempts resulted in a radiochemical conversion (RCC) of approximately 20%. In a next step, we optimized the RCC of the Cu-mediated radionuclide. Parameters such as temperature, time and base amount were screened and led to a further RCC increase of circa 10% (Figure 2).

It was then studied if the identified labeling conditions could be applied to more reactive Tzs. In this respect, Tzs with stepwise increased reactivity were chosen and tested to investigate the product scope of the suggested labeling procedure. Precursors and reference were synthesized using known procedures ^{[35-38]} and radiolabeling was conducted using the best conditions identified for our model compound. Moderate RCCs (10-30%) as well as sufficient decay-corrected (d.c.) RCYs (10-24%) could be isolated at the end of synthesis (EOS) for methyl-, phenyl- and H-Tzs. The automated synthesis including [¹⁸F]fluoride collection, azeotropic drying, labeling and HPLC separation was carried out within 90 minutes (Example 9). Radiochemical purity (RCP) and molar activity (Am) were good and within the area that we usually observe for other tracers. A typical activity yield was 215 MBq starting from 1.60 GBq fluoride-18. As expected, high reactive Tz resulted in the lowest RCY. However, observed RCYs are in the range of many clinically applied PET tracers. Table 1, annexed to this description, displays the found trend.

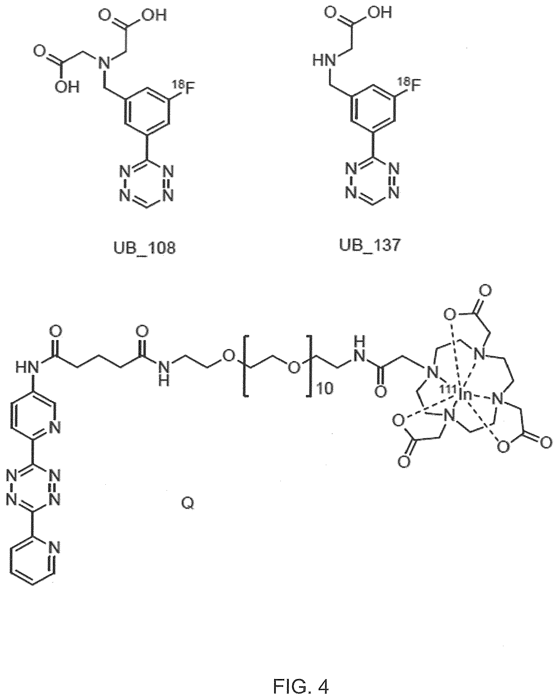

To study the effect of different substituents, [¹⁸F]-9 (Figure 4) was selected for further analysis since it displayed the highest reaction rate, which is one of the most crucial factors for pretargeted in vivo applications. In this respect, the substitution pattern was correlated with its synthetic accessibility and RCCs (as a predictor for RCYs). Necessary iodine Tz intermediates were obtained from the corresponding nitriles. 6-substituted stannate analogues could only be synthesized as methyl or methoxy derivatives. Similar stannate precursor formation at the 4-position succeeded only for the methoxy analogue. Both observations can most likely be explained by steric hindrance effects.^{[24-26]} As such, a 3,5-disubstitution pattern is best suited for oxidative ¹⁸F-florination.

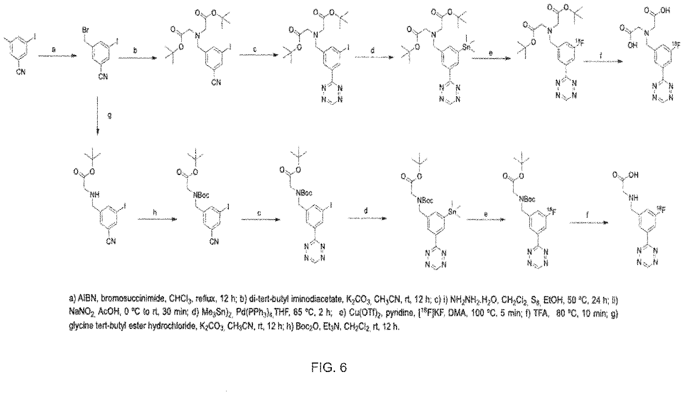

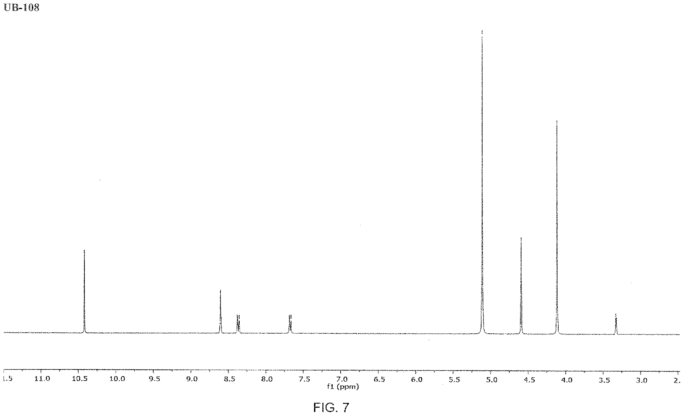

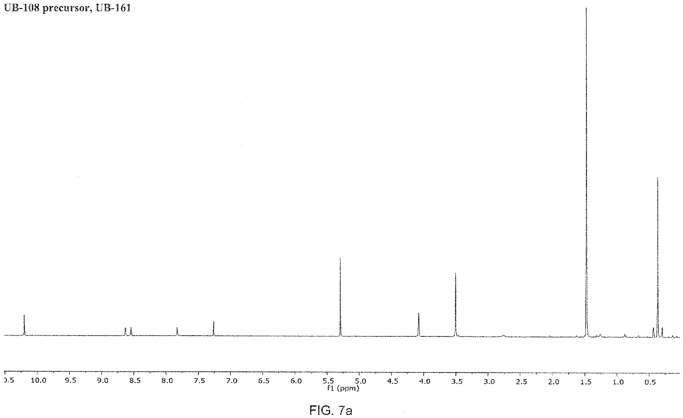

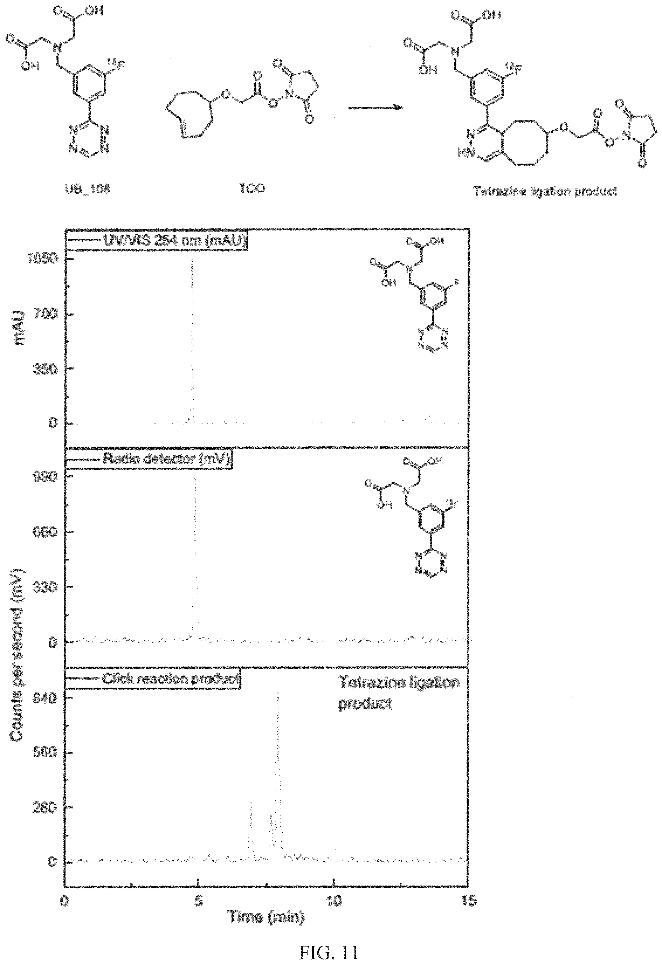

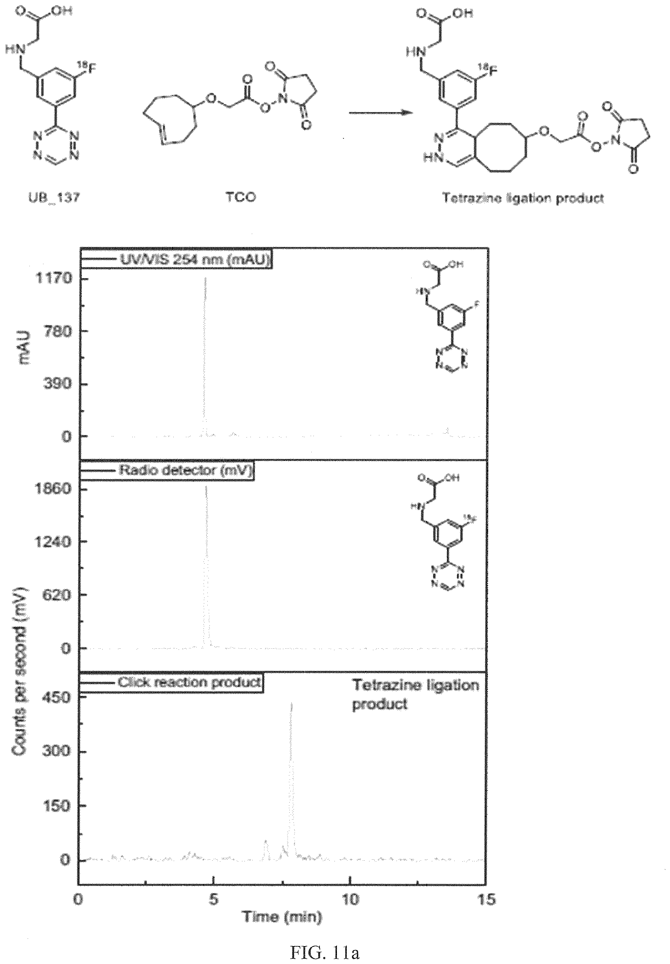

It is demonstrated herein that success of pretargeted imaging is strongly dependent on the polarity and the rate constant of the applied Tz (Figure 3). Low polarity (a lipophilicity of clogD_{7.4} < -0.5) and rate constants between 50.000-90.000 M⁻¹s⁻¹ measured in PBS and using TCO resulted in target-to-background ratios (Figure 3). In this respect, we designed two highly reactive Tzs, which allowed ¹⁸F-labeling and respective introduction of polar groups (Figure 4-8). UB137 and UB108 were synthesized in sufficient yields via a Pinner-like synthesis as described in Example 97 and 106 and their ability to be used in pretargeted imaging were evaluated. The results were compared with the performance of compounds UB-65 and RGV-52 (Figure 3). Efficiency in vivo was tested in an ex vivo screening assay lately published by our group and is inspired by traditional receptor blocking studies. In short, tumour-bearing mice are injected with a validated TCO-modified pretargeting vector 72h before a test ¹⁹F-Tz is administered. Subsequently after 2 hours, a pretargeted established ¹¹¹In-DOTA-Tz (Q)^{[6]} is administered to the same mouse. An ex vivo biodistribution study is subsequently conducted 22h later. In this manner, the blocking ability of the cold ¹⁹F-Tz can be evaluated. Higher blocking capacity is correlated with better in vivo imaging performance of the respective Tz. Polar Tz's, UB108 and UB137 resulted in the best blocking effect (Figure 3) and was as such selected for further development. The in vitro stability of UB108 and UB137 was assessed by analytical HPLC in Phosphate-buffered saline (PBS), Example 13 for UB108. UB108 and UB137 did not show degradation in PBS after 12 h at 37 °C. Consequently, the organotin precursors were synthesized with an unoptimized synthetic strategy (Figure 6, Examples 101 and 110). Radiolabeling succeeded in a one-pot, two step sequence with a RCY (d.c.) of approx. 10% and an overall synthesis time of ca. 90 minutes including synthesis, separation and formulation steps. An activity yield of 636 MBq starting from 11 GBq fluoride-18, and RCP of 98% at EOS was observed (Figure 6 and 11). UB137 could be labeled with similar results (Figure 6 and 7). The tetrazine ligation of ¹⁸F-UB108 with s-TCO occurred rapidly, forming ligation products after click reaction (Figure 11). Stability evaluation was completed for the formulated compound ¹⁸F-UB108 in PBS at room temperature. These results showed no significant decomposition after 4 h post-injection and importantly maintained their reactivity with TCO at all the time points tested (96% recovery). UB137 could be labeled with similar results.

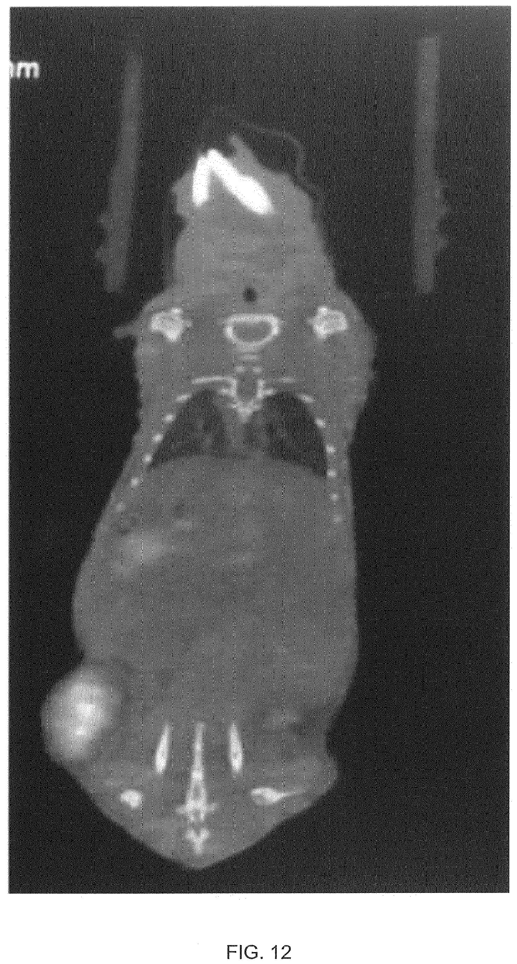

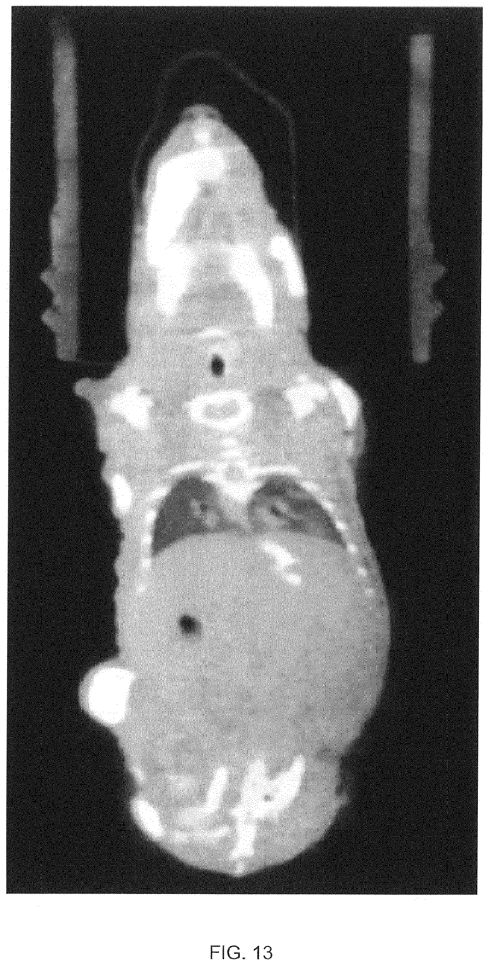

To test the tetrazine ligation in living animals, we administered CC49-TCO to mice bearing colon cancer xenografts overexpressing TAG72, followed by the injection of ¹⁸F-UB108 or ¹⁸F-UB137 two days later. We selected the TAG72 antigen as it is overexpressed in a wide range of solid tumours, including, colorectal cancer and its limited internalization and as CC49 binds to the target and is not internalized afterwards. This pretargeting model is considered as the "Gold standard".^{[15, 39]} Figure 12 and 13 displays the gained image 1h after ¹⁸F-UB108 or ¹⁸F-UB137 administration. The contrast is thus far the highest observed of all tetrazines after 1 h.

In conclusion, this work showed the first ¹⁸F-direct labeling strategy of highly reactive and polar Tzs, starting from organotin precursors via a Cu-mediated approach. This strategy allows us to synthesize and radiolabel two promising ¹⁸F-Tz, [¹⁸F]-UB108 and [¹⁸F]-UB137, as a tetrazine ligation based radiotracer for pretargeted in vivo imaging. The developed procedure is simple, short, reproducible as well as scalable and as such, superior to previously used ¹⁸F-multistep labeling strategies with regard to clinical applications. The compounds surprisingly result in very good target-to-background ratios already 1 h after injection unreached with any other tetrazines thus far.

### EXAMPLES

### General Procedures

### General Procedure A. Synthesis of 3- substituted -6-substituted-1,2,4,5-tetrazine.

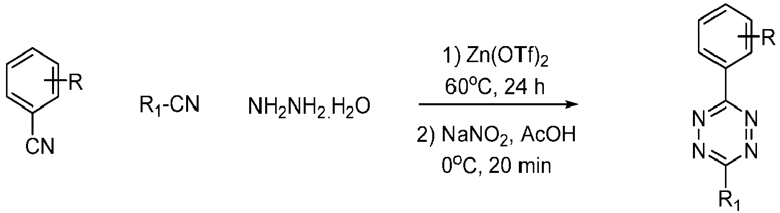

The preparation of this intermediates, were performed using a method described previously.^{[13]} The selected aromatic halogenated nitrile (1 mmol, 1 equiv.), Zn(OTf)₂ (182 mg, 0.50 mmol, 0.5 equiv.) and hydrazine monohydrate (2.43 mL, 50 mmol, 50 equiv.), along with the appropriate second nitrile (5 mmol, 5 equiv.), were added to a microwave vial equipped with a stir bar and sealed. The reaction was allowed to stir at 60 °C for 24 hours before being allowed to cool to room temperature and unsealed. NaNO₂ (1.35 g, 20 mmol, 20 equiv.) in water (30 mL) was added to the now yellow mixture followed by dropwise addition of acetic acid (14 mL), producing a mixture red in colour. The mixture was then extracted with EtOAc, washed with brine, dried over MgSO₄, filtered and concentrated under reduced pressure. The tetrazine was then purified via automatic flash chromatography utilising in various mixtures as the eluent.

### General Procedure B. Synthesis of 3- substituted -6-substituted-1,2-dihydro-1,2,4,5-tetrazine

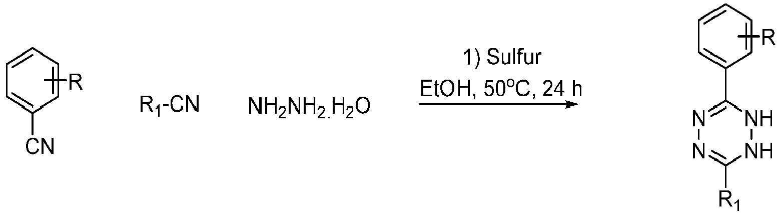

The preparation of this intermediates, was performed using a method described previously.^{[15]} The selected aromatic halogenated nitrile (1 mmol, 1 equiv.), sulfur (513 mg, 2.00 mmol, 2 equiv.), hydrazine monohydrate (804 uL, 16.5 mmol, 16.5 equiv.) and ethanol (2.0 mL), along with the appropriate second nitrile (4.5 mmol, 4.5 equiv.), were added to a microwave vial equipped with a stir bar and sealed. The reaction mixture was heated to 125 °C for 2 hours before being allowed to cool to room temperature, unsealed and dry under vacuum. The mixture was suspended in 10 mL water and extracted with CH₂Cl₂(2x10 mL), washed with brine, dried over MgSO₄, filtered and concentrated under reduced pressure. The tetrazine was then purified via automatic flash chromatography utilising in various mixtures as the eluent.

### General Procedure C. Synthesis of 3-substituted-6-H-1,2,4,5-tetrazine

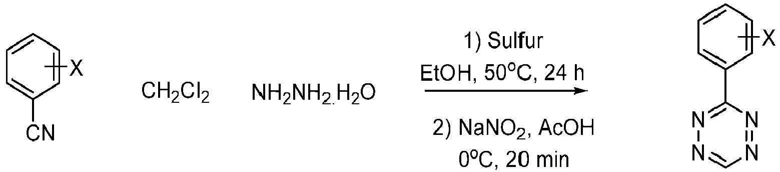

The preparation of this intermediates, was performed using a method described previously.^{[15]} CH₂Cl₂ (0.256 mL, 4.00 mmol, 1 equiv.), sulfur (0.257 g, 1.00 mmol, 0.25 equiv.), hydrazine monohydrate (1.6 mL, 32.00 mmol, 8 equiv.) and ethanol (4.0 mL) along with the appropriate nitrile (4 mmol, 1 equiv.) were added to a microwave vial equipped with a stir bar. The vessel was sealed, and the reaction mixture was heated to 50 °C for 24 hours, before being allowed to cool to room temperature and unsealed. Then 3 ml of CH₂Cl₂ and NaNO₂ (2.8 g, 40.00 mmol, 10 equiv.) in water (40 ml) were added to the now yellow mixture followed by dropwise addition of acetic acid (14 mL), producing a mixture red in colour. The reaction mixture was extracted with CH₂Cl₂, washed with brine, dried with MgSO₄ and filtered before concentrating *in vacuo.* The tetrazine was then purified via flash chromatography utilising n-Heptane and EtOAc in various mixtures as the eluent and recrystallized in n-Heptane.

### General Procedure D.1. Synthesis of organotin compounds

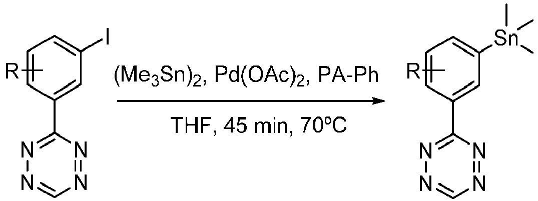

The preparation of these intermediates, was performed using a method described previously with minor modifications.^{[14]} Palladium acetate (4.5 mg, 12%) and 1,3,5,7-tetramethyl-2,4,8-trioxa-(2,4-dimethoxyphenyl)-6-phosphaadamantane (PA-Ph) (9.8mg, 20%) dry THF (1.5 mL) and hexamethylditin (75 µL, 137 mg, 0.42 mmol, 2.5 equiv.) were successively added to a microwave vial equipped with a stir bar which was then sealed and purged with N₂. A solution of the appropriate *iodo-phenyl-1,2,4,5-tetrazine* (0.17 mmol) in dry THF (1 mL) was added via a syringe and the reaction allowed to stir at 70 °C in a microwave for 45 minutes. The reaction was allowed to cool to room temperature and unsealed before being quenched with saturated aqueous KF (1 mL). The solution was extracted with CH₂Cl₂ washed with brine, dried over MgSO₄, filtered and concentrated under reduced pressure. The tetrazine was then purified via automatic flash chromatography utilising n-Heptane and EtOAc as the eluent.

### General Procedure D.2. Synthesis of organotin compounds.

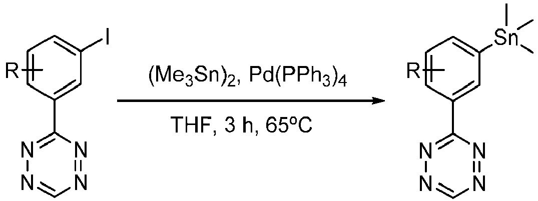

The preparation of these intermediates, was performed using a method described previously with minor modifications.^{[14]} Pd(PPh₃)₄ (19.4 mg, 10%) and Hexamethylditin (87 µL, 0.42 mmol, 2.5 equiv.) were successively added to a microwave vial equipped with a stir bar which was then sealed and purged with N₂. A solution of the appropriate *iodo-phenyl-1,2,4,5-tetrazine* (0.17 mmol) in dry THF (2.5 mL) was added via a syringe and the reaction allowed to stir at 65 °C in a microwave for 3 hours. The reaction was allowed to cool to room temperature and unsealed before being quenched with saturated aqueous KF (1 mL). The solution was extracted with CH₂Cl₂ washed with brine, dried over MgSO₄, filtered and concentrated under reduced pressure. The tetrazine was then purified via automatic flash chromatography utilising n-Heptane and EtOAc as eluent.

### Example 1

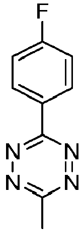

**3-(4-fluorophenyl)-6-methyl-1,2,4,5-tetrazine (RGV_48, Tz-1):** The final compound was obtained from 4-fluorobenzonitrile (121 mg, 1.00 mmol) and acetonitrile (261 uL, 5.00 mmol) following the *General Procedure A.* The crude was purified using flash chromatography (90/10 n-Heptane/EtOAc) to yield 0.57 g (30%) of a pink solid. *Rf* = 0.31 (n-Heptane:10%EtOAc); ¹H NMR (400 MHz, Chloroform-d) δ 8.62 (dd, *J* = 8.7, 5.4 Hz, 2H), 7.27 (t, *J* = 8.7 Hz, 2H), 3.10 (s, 3H); ¹³C NMR (101 Hz, CDCl₃) δ 167.2, 165.7 (d, J= 254.1 Hz) 163.3, 130.2 (d, J=8.9 Hz), 127.9, 116.5 (d, J=21.9 Hz), 21.1; HPLC-MS [M+H]+ m/z calc. for [C₉H₈FN₄]⁺: 191.07; Found: 191.20.

### Example 2

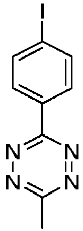

**3-(4-iodophenyl)-6-methyl-1,2,4,5-tetrazine** (RGV_5): The final compound was obtained from 4-iodobenzonitrile (229 mg, 1.00 mmol) and acetonitrile (261 uL, 5.00 mmol) following the *General Procedure A.* The crude was purified using flash chromatography (90/10 n-Heptane/EtOAc) to yield 0.85 g (39%) of a red solid. *Rf* = 0.25 (n-Heptane:10%EtOAc); ¹H NMR (400 MHz, Chloroform-*d*) δ 8.35 - 8.27 (m, 2H), 8.00 - 7.90 (m, 2H), 3.09 (s, 3H).; ¹³C NMR (101 MHz, Chloroform-d) δ 167.65, 163.92, 138.71, 131.44, 129.41, 100.33, 21.35.

### Example 3

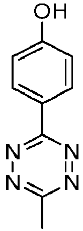

**4-(1,2,4,5-tetrazin-3-yl)phenol (RGV_59).** The final compound was obtained from 4-hydroxybenzonitrile (120 mg, 1.00 mmol) and acetonitrile (261 uL, 5.00 mmol) following the *General Procedure A.* The crude was purified using flash chromatography (90/10 n-Heptane/EtOAc) to yield 0.32 g (20%) of a red solid. *Rf* = 0.35 (n-Heptane:10%EtOAC); ¹H NMR (400 MHz, Methanol-*d*₄) δ 8.42 (d, *J* = 8.8 Hz, 2H), 7.01 (d, *J* = 8.8 Hz, 2H), 3.02 (s, 3H); ¹³C NMR (101 MHz, MeOD) δ 167.84, 165.19, 163.13, 130.73, 124.33, 117.13, 20.88; HPLC-MS [M+H]⁺ m/z calc. for [C₉H₉ON₄]⁺: 189.08; Found: 189.40.

### Example 4

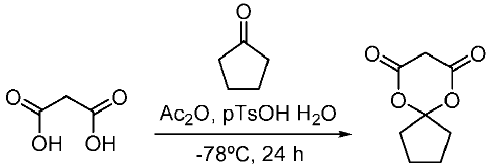

**6,10-dioxaspiro[4.5]decane-7,9-dione.** Malonic acid (2 g) and pTsOH H2o were added toa 25 mL flask and cooled to -75 C. Ac2O (mL) was dded dropwise and then cyclopentanone (mL) under stirring. The reaction was allowed to heat to room temperature overnight. Water was added tot he reaction (10 mL) and it was cooled down, until the formation of a white precipitate was observed. The precipitate was then filreted and washed with water and cold EtOH. The white crystal powder was collected to yield 165 mg.

### Example 4a

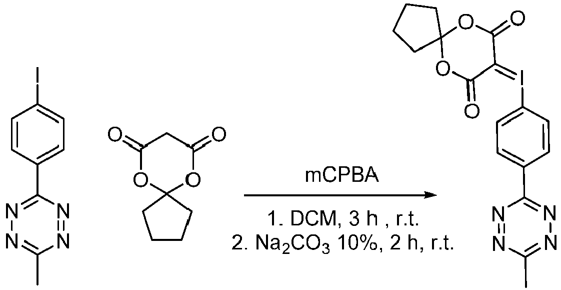

**8-((4-(6-methyl-1,2,4,5-tetrazin-3-yl)phenyl)-I3-iodaneylidene)-6,10-dioxaspiro[4.5]decane-7,9-dione (RGV_8).** 3-(4-iodophenyl)-6-methyl-1,2,4,5-tetrazine (15 mg, 0.05 mmol, 1 equiv) is dissolved CH₂Cl₂ (1mL/1mmol) in a sealed tube before adding mCPBA (13.8 mg, 0.06 mmol, 1.2 equiv), the mixture is sealed and allowed to stir at room temperature for 3 hours. A solution of 6,10-dioxaspiro[4.5]decane-7,9-dione (9.4 mg, 0.05 mmol, 1.1 equiv) in Na₂CO₃ 10% (2.86mL/mmol) is prepared and then added dropwise to the mixture in the sealed tube. The mixture was stirred at room temperature for additional 2 hours. To the reaction mixture 5 mL of water is added and is extracted by CH₂Cl₂, dried over MgSO₄, filtered and concentrated *in vacuo.* The crude was submited to combi flash from 100% CH₂Cl₂ to CH₂Cl₂/10% EtOH. All fractions containing compound were concentrated, disolved in warm methanol and left to crystalized at 4°C, which afforded pink crystals (5.4 mg, 15%). Rf = (CH₂Cl₂:EtOH (:1)); ¹H NMR (400 MHz, Methanol-*d*₄) δ 8.65 (d, *J* = 8.6 Hz, 2H), 8.19 (d, *J* = 8.6 Hz, 2H), 3.11 (s, 3H), 2.16 (t, *J* = 7.4 Hz, 4H), 1.84 (t, *J* = 7.5 Hz, 4H); ¹³C NMR (151 MHz, CDCl₃) δ 168.31, 164.35, 162.97, 138.73, 135.86, 133.86, 131.15, 117.80, 114.58, 37.60, 23.56, 21.46.

### Example 5

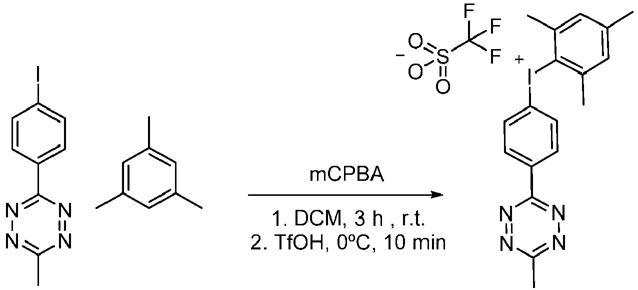

**mesityl(4-(6-methyl-1,2,4,5-tetrazin-3-yl)phenyl)iodonium (RGV_10).**^{[20]} In a sealed tube m-Chloroperbenzoic acid (11.3 mg, 0.05 mmol) and 3-(4-iodophenyl)-6-methyl-1,2,4,5-tetrazine (10 mg, 0.03 mmol) were dissolved in CH₂Cl₂ (1 mL/0.23 mmol) and stirred at r.t. during 3 hours. Mesitulene (5.1 µL, 0.04 mmol) is added and the mixture is cooled to 0°C followed by dropwise addition of TfOH (8.9µL, 0.10mmol). The reaction mixture was stirred at r.t during 10 minutes. The crude reaction was concentrated under vacuum. Diethyl ether was added and the mixture was stirred at r.t. during 20 minutes and then stored in the freezer during 1 hour for ensure complete precipitation, before filtered and washed with diethyl ether. The resulting solid was collected with methanol and dried under vacuum (12mg, 71%). ¹H NMR (600 MHz, MeOD) δ 8.57 (d, *J* = 8.7 Hz, 2H), 8.07 (d, *J* = 8.7 Hz, 2H), 3.00 (s, 3H), 2.64 (s, 6H), 2.31 (s, 3H).

### Example 6

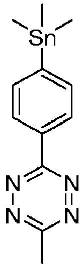

**3-(4-trimethyltin)-6-methyl-1,2,4,5-tetrazine (RGV_6).** The final compound was obtained from 50 mg (0.17 mmol) of the starting material, following the *General Procedure D.1.* The crude was purified using flash chromatography (90/10 n-Heptane/EtOAc) to yield 0.46 g (76%) of a pink solid. *Rf* = 0.39 (n-Heptane:10%EtOAc); ¹H NMR (400 MHz, Chloroform-*d*) δ 8.52 (d, J = 8.2 Hz, 2H), 7.73 (d, J = 8.2 Hz, 2H), 3.09 (s, 3H), 0.36 (s, 9H).; ¹³C NMR (101 MHz, Chloroform-*d*) δ 167.35, 164.55, 149.30, 136.77, 131.62, 126.81, 21.29, -9.35.;HPLC-MS [M+H]⁺ m/z calc. for [C₁₂H₁₇SnN₄]⁺: 337.04; Found: 337.45.

### Example 7 - Radiolabeling of Tz-1:

The radiolabelling of Tz-1 was provided as described in Example 128, 129 and 130 and as shown in Figure 2. In the table in Figure 2, the following conditions were applied: [a] Cu(OTf)₂, pyridine, [¹⁸F]KF, DMA, 100 °C, 5 min.; [b] Cu(OTf)₂, pyridine, [¹⁸F]KF (50 ug K₂CO₃), DMA, 5 min.; [c] Cu(OTf)₂, pyridine, [¹⁸F]KF (50 ug K₂CO₃), DMA, 100 °C. Radiochemical conversion (RCC) was determined by radio-HPLC (n=3). Radiochemical yield (RCY) was decay corrected to the starting amount of radioactivity received from the cyclotron and the isolated product without formulation step (n=3).

### Example 8

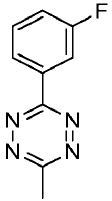

**3-(3-fluorophenyl)-6-methyl-1,2,4,5-tetrazine (RGV_49, (W)):** The final compound was obtained from 3-fluorobenzonitrile (120 mg, 1.00 mmol) and acetonitrile (261 uL, 5.00 mmol) following the *General Procedure A.* The crude was purified using flash chromatography (90/10 n-Heptane/EtOAc) to yield 0.80 g (42%) of a purple solid. *Rf* = 0.28 (n-Heptane:10%EtOAc); ¹H NMR (400 MHz, Chloroform-d) δ 8.49 - 8.35 (m, 1H), 8.35 - 8.22 (m, 1H), 7.67 - 7.49 (m, 1H), 7.43 - 7.28 (m, 1H), 3.11 (s, 3H); ¹³C NMR (101 MHz, Chloroform-d) δ 167.79, 163.50 (d, *J* = 3.3 Hz), 163.45 (d, *J* = 247.1 Hz), 134.15 (d, *J* = 8.3 Hz), 131.08 (d, *J* = 8.0 Hz), 123.77 (d, *J* = 3.1 Hz), 119.72 (d, *J* = 21.3 Hz), 114.95 (d, *J* = 24.0 Hz), 21.34; HPLC-MS [M+H]⁺ m/z calc. for [C₉H₈FN₄]⁺: 191.07; Found: 191.47.

### Example 9

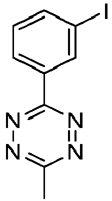

**3-(3-iodophenyl)-6-methyl-1,2,4,5-tetrazine (RGV_50):** The final compound was obtained from 3-iodobenzonitrile (229 mg, 1.00 mmol) and acetonitrile (261 uL, 5.00 mmol) following the *General Procedure A.* The crude was purified using flash chromatography (90/10 n-Heptane/EtOAc) to yield 0.88 g (29%) of a purple solid. *Rf* = 0.26 (n-Heptane:EtOAc=8:1); ¹H NMR (400 MHz, Chloroform-d) δ 8.96 (t, *J* = 1.7 Hz, 1H), 8.57 (dt, *J* = 7.9, 1.4 Hz, 1H), 7.96 (ddd, *J* = 7.9, 1.8, 1.1 Hz, 1H), 7.33 (t, *J* = 7.9 Hz, 1H), 3.11 (s, 3H); ¹³C NMR (101 MHz, Chloroform-d) δ 167.81, 163.12, 141.54, 136.84, 133.89, 130.99, 127.15, 94.95, 21.36.

### Example 9a

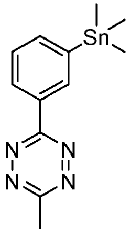

**3-(3-trimethyltin)-6-methyl-1,2,4,5-tetrazine (RGV_51).** The final compound was obtained from 50 mg (0.17 mmol) of the starting material, following the *General Procedure D.1.* The crude was purified using flash chromatography (90/10 n-Heptane/EtOAc) to yield 0.32 g (65%) of a pink solid. *Rf* = 0.38 (n-Heptane:10%EtOAc); ¹H NMR (400 MHz, Chloroform-d) δ 8.78 - 8.62 (m, 1H), 8.51 (ddd, *J* = 7.9, 2.0, 1.4 Hz, 1H), 7.74 (dt, *J* = 7.2, 1.2 Hz, 1H), 7.55 (ddd, *J* = 7.8, 7.1, 0.6 Hz, 1H), 3.10 (s, 3H), 0.37 (s, 9H).; ¹³C NMR (101 MHz, Chloroform-d) δ 167.31, 164.58, 144.07, 140.15, 135.25, 131.30, 128.78, 127.96, 21.29, -9.26.; HPLC-MS [M+H]⁺ m/z calc. for [C₁₂H₁₇SnN₄]⁺: 337.04; Found: 337.38;

### Example 10 - Radiolabeling of RGV_49 (W):

The radiolabelling of RGV_49 was provided as described in Example 128, 129 and 130 and as shown in Table 1 ([18F]W). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 11

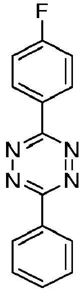

**3-(4-fluorophenyl)-6-phenyl-1,2,4,5-tetrazine (RGV_72 (E)):** The final compound was obtained from 4-fluorobenzonitrile (121 mg, 1.00 mmol) and benzonitrile (477 uL, 5.00 mmol) following the *General Procedure A.* The final compound was isolated by preparative TLC (60/30 Toluene/n-Heptane) to yield a pink solid. *Rf* = 0.65 (Toluene:10%n-Heptane); ¹H NMR (600 MHz, Chloroform-*d*) δ 8.72 - 8.67 (m, 2H), 8.67 - 8.62 (m, 2H), 7.69 - 7.59 (m, 3H), 7.31 (t, *J* = 8.6 Hz, 2H); ¹³C NMR (151 MHz, Chloroform-*d*) δ 166.83, 164.25 (d, *J* = 269.7 Hz), 164.11, 132.90, 131.86, 130.46 (d, *J* = 9.0 Hz), 129.49, 128.19, 128.14, 116.75 (d, *J* = 22.0 Hz); HPLC-MS [M+H]⁺ m/z calc. for [C₁₄H₉FN₄]⁺: 253.09; Found: 253.0.

### Example 12

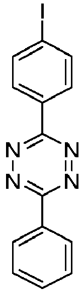

**3-(4-iodophenyl)-6-phenyl-1,2,4,5-tetrazine (RGV_14):** The final compound was obtained from 4-iodobenzonitrile (229 mg, 1.00 mmol) and enzonitrile (477 uL, 5.00 mmol) following the *General Procedure A.* The crude was purified using flash chromatography (90/10 Toluene/n-Heptane) to yield 0.35 g (10%) of a pink solid. *Rf* = 0.5 (Toluene:10%n-Heptane); ¹H NMR (600 MHz, Chloroform-*d*) δ 8.68 - 8.63 (m, 2H), 8.40 - 8.35 (m, 2H), 8.00 - 7.95 (m, 2H), 7.68 - 7.59 (m, 3H); ¹³C NMR (151 MHz, CDCl₃) δ 164.27, 163.80, 138.80, 133.00, 131.80, 131.45, 129.51, 129.43, 128.21, 100.51.

### Example 12a

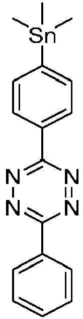

**3-phenyl-6-(4-(trimethylstannyl)phenyl)-1,2,4,5-tetrazine (RGV_26).** The final compound was obtained from 50 mg (0.17 mmol) of the starting material, following the *General Procedure D.1*. The crude was purified using flash chromatography (90/10 n-Heptane/EtOAc) to yield 0.52 mg (95%) of a pink solid. *Rf* = 0.48 (n-Heptane:10%EtOAc). ¹H NMR (400 MHz, Chloroform-*d*) δ 8.70 - 8.63 (m, 2H), 8.63 - 8.53 (m, 2H), 7.85 - 7.68 (m, 2H), 7.68 - 7.57 (m, 3H), 0.37 (s, 9H).; ¹³C NMR (151 MHz, CDCl₃) δ 164.47, 164.13, 149.66, 136.88, 132.80, 132.00, 131.62, 129.46, 128.11, 127.08, -9.32.; HPLC-MS [M+H]⁺ m/z calc. for [C₁₇H₁₈N₄Sn]⁺: 399.06; Found: 399.1.

### Example 13 - Radiolabeling of RGV_72 (E):

The radiolabelling of RGV_72 was provided as described in Example 128, 129 and 130 and as shown in Table 1 ([18F]E). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 14

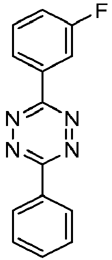

**3-(3-fluorophenyl)-6-phenyl-1,2,4,5-tetrazine (RGV_71 (R)):** The final compound was obtained from 3-fluorobenzonitrile (107 uL, 1.00 mmol) and benzonitrile (477 uL, 5.00 mmol) following the *General Procedure A.* The final compound was isolated by preparative TLC (60/30 Toluene/n-Heptane) to yield a pink solid. Rf= 0.65 (Toluene:10%n-Heptane); ¹H NMR (400 MHz, Chloroform-d) δ 8.64 - 8.58 (m, 2H), 8.40 (dt, *J* = 7.8, 1.3 Hz, 1H), 8.33 - 8.26 (m, 1H), 7.62 - 7.50 (m, 4H), 7.28 (tdd, *J* = 8.3, 2.7, 1.0 Hz, 1H); ¹³C NMR (151 MHz, Chloroform-*d*) δ 164.38, 163.50 (d, *J* = 247.2 Hz), 163.40 (d, *J* = 3.1 Hz), 134.17 (d, *J* = 8.3 Hz), 133.07, 131.76, 131.16 (d, *J* = 8.0 Hz), 129.53, 128.29, 123.84, 119.84 (d, *J* = 21.4 Hz), 114.98 (d, *J* = 24.0 Hz); HPLC-MS [M+H]⁺ m/z calc. for [C₁₄H₉FN₄]⁺: 253.09; Found: 253.0..

### Example 15

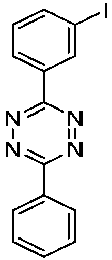

**3-(3-iodophenyl)-6-phenyl-1,2,4,5-tetrazine (RGV_15):** The final compound was obtained from 3-iodobenzonitrile (229 mg, 1.00 mmol) and benzonitrile (477 uL, 5.00 mmol) following the *General Procedure A.* The crude was purified using flash chromatography (90/10 n-Heptane/EtOAc) to yield 0.50 g (14%) of a pink solid. *Rf* = 0.5 (Toluene:10%n-Heptane); ¹H NMR (400 MHz, Chloroform-*d*) δ 9.02 (t, *J* = 1.8 Hz, 1H), 8.65 (dd, *J* = 8.0, 1.6 Hz, 2H), 8.62 (d, *J* = 8.0 Hz, 1H), 7.97 (d, *J* = 7.9 Hz, 1H), 7.66 - 7.57 (m, 3H), 7.35 (t, *J* = 7.9 Hz, 1H).; ¹³C NMR (151 MHz, CDCl₃) δ 164.35, 162.96, 141.60, 136.89, 133.89, 133.07, 131.72, 131.04, 129.52, 128.28, 127.15, 95.03.

### Example 16

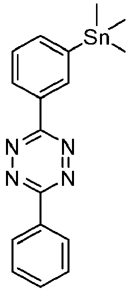

**3-phenyl-6-(3-(trimethylstannyl)phenyl)-1,2,4,5-tetrazine (RGV_25).** The final compound was obtained from 50 mg (0.17 mmol) of the starting material, following the *General Procedure D.1*. The crude was purified using flash chromatography (90/10 n-Heptane/EtOAc) to yield 0.53g (95%) of a pink solid. Rf= 0.48 (n-Heptane:10%EtOAc); ¹H NMR (600 MHz, Chloroform-d) δ 8.78 (dt, *J* = 1.7, 0.7 Hz, 1H), 8.69 - 8.65 (m, 2H), 8.59 (ddd, *J* = 7.9, 2.0, 1.3 Hz, 1H), 7.77 (dt, *J* = 7.2, 1.2 Hz, 1H), 7.65 - 7.61 (m, 3H), 7.58 (ddd, *J* = 7.8, 7.2, 0.6 Hz, 1H), 0.38 (s, 9H).; ¹³C NMR (151 MHz, CDCl₃) δ 164.44, 164.06, 144.19, 140.31, 135.34, 132.82, 131.98, 131.28, 129.47, 128.87, 128.10, 128.01, -9.24; HPLC-MS [M+H]⁺ m/z calc. for [C₁₇H₁₈N₄Sn]⁺: 399.06; Found: 399.1.

### Example 17 - Radiolabeling of RGV_71 (R):

The radiolabelling of RGV_71 was provided as described in Example 128, 129 and 130 and as shown in Table 1 ([¹⁸F]R). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 18

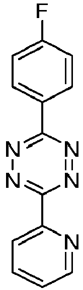

**3-(4-fluorophenyl)-6-(pyridin-2-yl)-1,2,4,5-tetrazine (RGV_3, (T)):** The final compound was obtained from 4-flourbenzonitrile (121 mg, 1.00 mmol) and 2-cyanopyridine (520 mg, 5.00 mmol) following the *General Procedure A.* The crude was purified using flash chromatography (50/50 n-Heptane/EtOAc) to yield 0.40 g (16%) of a pink solid. *Rf* = 0.5 (n-Heptane:50%EtOAC); ¹H NMR (600 MHz, Chloroform-d) δ 8.92 (ddd, *J* = 4.8, 1.8, 0.9 Hz, 1H), 8.69 - 8.63 (m, 2H), 8.63 (dt, *J* = 7.8, 1.1 Hz, 1H), 7.95 (td, *J* = 7.7, 1.8 Hz, 1H), 7.52 (ddd, *J* = 7.6, 4.8, 1.2 Hz, 1H), 7.28-7.19 (m, 2H); ¹³C NMR (151 MHz, Chloroform-d) δ 167.07, 164.57 (d, *J* = 243.4 Hz), 163.51, 151.03, 150.32, 137.69, 130.97 (d, *J* = 9.2 Hz), 127.93 (d, *J* = 3.0 Hz), 126.55, 124.08, 116.82 (d, *J* = 22.1 Hz); HPLC-MS [M+H]⁺ m/z calc. for [C₁₃H₈FN₅]⁺: 254.08; Found: 254.0..

### Example 19

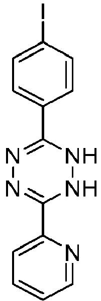

**3-(4-iodophenyl)-6-(pyridin-2-yl)-1,2-dihydro-1,2,4,5-tetrazine (RGV_16):** The final compound was obtained from 4-iodobenzonitrile (229 mg, 1.00 mmol) and 2-cyanopyridine (433 uL, 4.5 mmol) following the *General Procedure B.* The crude was purified using flash chromatography (90/10 Toluene/EtOAc) to yield 0.71 g (19%) of an orange solid. *Rf* = 0.38 (Toluene/10%EtOAc); ¹H NMR (400 MHz, DMSO-*d*₆) δ 9.31 (s, 1H), 8.74 (s, 1H), 8.63 (d, *J* = 4.9 Hz, 1H), 8.00 - 7.86 (m, 2H), 7.85 - 7.75 (m, 2H), 7.61 (d, *J* = 8.4 Hz, 2H), 7.53 (ddd, *J* = 6.9, 4.8, 1.6 Hz, 1H).; ¹³C NMR (151 MHz, CDCl₃) δ 148.43, 147.14, 147.10, 138.15, 137.15, 129.79, 127.59, 125.29, 121.53, 97.03.

### Example 20

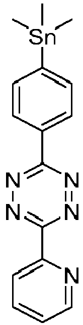

**3-(pyridin-2-yl)-6-(4-(trimethylstannyl)phenyl)-1,2,4,5-tetrazine (RGV_46).** The final compound was obtained from 50 mg (0.17 mmol) of the starting material, following the *General Procedure D.1.* The final compound autooxidised under reaction conditions. The crude was purified using flash chromatography (60/40 n-Heptane/EtOAc) to yield 14 mg (49%) of a pink solid.. *Rf* = 0.31 (EtOAc:50%n-Heptane); ¹H NMR (600 MHz, Chloroform-d) δ 9.00 - 8.94 (m, 1H), 8.69 (dt, *J* = 7.9, 1.0 Hz, 1H), 8.64 - 8.60 (m, 2H), 8.00 (td, *J* = 7.8, 1.8 Hz, 1H), 7.78 - 7.74 (m, 2H), 7.59 - 7.52 (m, 1H), 0.37 (s, 9H); ¹³C NMR (151 MHz, CDCl₃) δ 164.90, 163.61, 151.07, 150.53, 150.25, 137.57, 136.90, 131.37, 127.48, 126.43, 124.02, -9.33.; HPLC-MS [M+H]⁺ m/z calc. for [C₁₆H₁₇N₅Sn]⁺: 400.06; Found: 400.1.

### Example 21 - Radiolabeling of RGV_3 (T):

The radiolabelling of RGV_3 was provided as described in Example 128, 129 and 130 and as shown in Table 1 ([¹⁸F]T). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 22

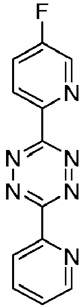

**3-(5-fluoropyridin-2-yl)-6-(pyridin-2-yl)-1,2,4,5-tetrazine (RGV_61, (U)):** The final compound was obtained from 5-fluoropicolinonitrile (122 mg, 1.00 mmol) and 2-cyanopyridine (520 uL, 5.00 mmol) following the *General Procedure A.* The crude was purified using flash chromatography (50/50 n-Heptane/EtOAc) to yield 0.45 g (18%) of a pink solid. *Rf* = 0.5 (n-Heptane:50%EtOAC); HPLC-MS [M+H]⁺ m/z calc. for [C₁₂H₈FN₆]⁺: 255.08; Found: 255.0.

### Example 23

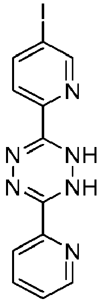

**3-(5-iodopyridin-2-yl)-6-(pyridin-2-yl)-1,2-dihydro-1,2,4,5-tetrazine (CPO_5):**^{[12]} The final compound was obtained from 2-Cyano-5-iodopyridine (231 mg, 1.00 mmol) and 2-Cyanopyridine (433 uL, 4.5 mmol) following the *General Procedure B.* The crude was purified using flash chromatography (95/5 n-Heptane/EtOAc) to yield 0.53 g (15%) of an orange solid. *Rf* = 0.44 (n-Heptane:5%EtOAC); ¹H NMR (600 MHz, Chloroform-d) δ 8.79 (dd, *J* = 2.1, 0.8 Hz, 1H), 8.61 - 8.54 (m, 2H), 8.40 (s, 1H), 8.08 - 8.02 (m, 2H), 7.83 (dd, *J* = 8.3, 0.9 Hz, 1H), 7.75 (td, *J* = 7.7, 1.7 Hz, 1H), 7.35 (ddd, *J* = 7.5, 4.9, 1.2 Hz, 1H).; ¹³C NMR (151 MHz, CDCl₃) δ 154.62, 148.54, 147.50, 146.66, 146.55, 146.21, 145.15, 136.89, 125.09, 122.91, 121.46, 95.02.

### Example 24

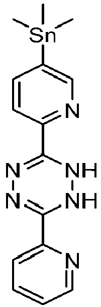

**3-(pyridin-2-yl)-6-(5-(trimethylstannyl)pyridin-2-yl)-1,2-dihydro-1,2,4,5-tetrazine (CPO_6).** The final compound was obtained from 50 mg (0.17 mmol) of the starting material, following the *General Procedure D.1.* The crude was purified using flash chromatography (70/30 n-Heptane/EtOAc) to yield 0.47 g (85%) of an orange solid. *Rf* = 0.50 (n-Heptane:10%EtOAc); ¹H NMR (400 MHz, Chloroform-*d*) δ 8.62 - 8.51 (m, 4H), 8.05 (dt, *J* = 8.0, 1.1 Hz, 1H), 7.97 (dd, *J* = 7.7, 1.1 Hz, 1H), 7.84 (dd, *J* = 7.7, 1.5 Hz, 1H), 7.75 (td, *J* = 7.7, 1.7 Hz, 1H), 7.34 (ddd, *J* = 7.5, 4.9, 1.2 Hz, 1H), 0.36 (s, 9H).; ¹³C NMR (101 MHz, CDCl₃) δ 154.24, 148.52, 147.74, 147.18, 147.10, 146.81, 144.22, 139.76, 136.82, 124.95, 121.41, 121.02, -9.35.

### Example 25

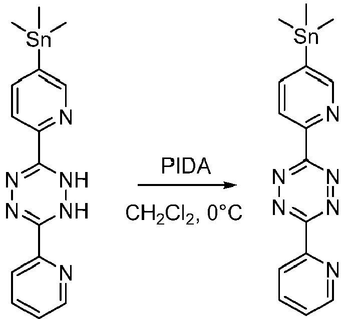

**3-(pyridin-2-yl)-6-(5-(trimethylstannyl)pyridin-2-yl)-1,2,4,5-tetrazine (RGV_60).** The 1,2-dihydro-1,2,4,5-tetrazine stannate was dissolved in dry CH₂Cl₂ and cooled to 0°C, followed by the portion wise addition of (Diacetoxyiodo)benzene (1.2 equiv.). The reaction was allowed to warm to r.t. and stirred for 3h. The crude was purified using flash chromatography. HPLC-MS [M+H]⁺ m/z calc. for [C₁₅H₁₆N₆Sn]⁺: 401.05; Found: 401.1..

### Example 26 - Radiolabeling of RGV_61 (U):

The radiolabelling of RGV_61 was provided as described in Example 128, 129 and 130 and as shown in Table 1 ([¹⁸F]U). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 27

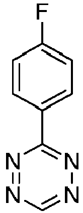

**3-(4-fluorophenyl)-1,2,4,5-tetrazine (RGV_55, (Y)):** The final compound was obtained from 4-fluorobenzonitrile (242 mg, 4 mmol) following *General Procedure C*. The crude was purified using flash chromatography (90/10 n-Heptane/EtOAc) to yield 0.12 g (34%) of a red solid. Rf = 0.33 (n-Heptane:10%EtOAC); ¹H NMR (400 MHz, Chloroform-*d*) δ 10.21 (s, 1H), 8.70-8.61 (m, 2H), 7.29 (t, *J* = 8.7 Hz, 2H); ¹³C NMR (101 MHz, Chloroform-*d*) δ 167.52 (d, *J* = 255.2 Hz), 164.98, 161.85 (d, *J* = 798.2 Hz), 130.85 (d, *J* = 9.2 Hz), 127.94 (d, *J* = 3.2 Hz), 116.81 (d, *J* = 22.1 Hz); HPLC-MS [M+H]⁺ m/z calc. for [C₈H₆FN₄]⁺: 177.06; Found: 177.34..

### Example 28

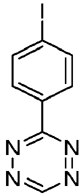

**3-(4-iodophenyl)-1,2,4,5-tetrazine (RGV_56):** The final compound was obtained from 4-iodobenzonitrile (458 mg, 4 mmol) following *General Procedure C*. The crude was purified using flash chromatography (90/10 n-Heptane/EtOAc) to yield 0.16 mg (27%) of a pink solid. *Rf* = 0.37 (n-Heptane:10%EtOAC); ¹H NMR (400 MHz, Chloroform-d) δ 10.24 (s, 1H), 8.39 - 8.32 (m, 2H), 8.02 - 7.95 (m, 2H).; ¹³C NMR (101 MHz, CDCl₃) δ 166.36, 158.10, 138.90, 131.24, 129.75, 101.22.

### Example 29

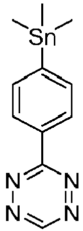

**3-(4-trimethyltin)-6-methyl-1,2,4,5-tetrazine (RGV_57).** The final compound was obtained from 50 mg (0.17 mmol) of the starting material, following the *General Procedure D.1.* The crude was purified using flash chromatography (90/10 n-Heptane/EtOAc) to yield 0.27 g (61%) of a pink solid. *Rf* = 0.43 (n-Heptane:20%EtOAc); ¹H NMR (400 MHz, Chloroform-d) δ 10.20 (s, 1H), 8.58-8.54 (m, 2H), 7.84-7.67 (m, 2H), 0.37 (s, 9H).; ¹³C NMR (101 MHz, Chloroform-d) δ 167.00, 157.96, 150.34, 136.90, 130.29, 127.35, -9.33.; HPLC-MS [M+H]⁺ m/z calc. for [C₁₁H₁₅SnN₄]⁺: 323.04; Found: 323.38.

### Example 30 - Radiolabeling of RGV_55 (Y):

The radiolabelling of RGV_55 was provided as described in Example 128, 129 and 130 and as shown in Table 1. In Table 1 ([¹⁸F]Y), the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 31

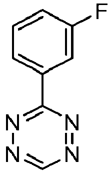

**3-(3-fluorophenyl)-1,2,4,5-tetrazine (RGV-52.(9)):** The final compound was obtained from 3-fluorobenzonitrile (242 mg, 4 mmol) following *General Procedure C*. The crude was purified using flash chromatography (90/10 n-Heptane/EtOAc) to yield 0.12 g (34%) of a red solid. *Rf* = 0.34 (n-Heptane:10%EtOAC); ¹H NMR (400 MHz, Chloroform-d) δ 10.25 (s, 1H), 8.44 (dt, *J* = 7.8, 1.3 Hz, 1H), 8.33 (ddd, *J* = 9.7, 2.7, 1.6 Hz, 1H), 7.60 (td, *J* = 8.1, 5.7 Hz, 1H), 7.36 (tdd, *J* = 8.3, 2.7, 1.0 Hz, 1H); ¹³C NMR (101 MHz, Chloroform-d) δ 165.87 (d, *J* = 3.3 Hz), 163.47 (d, *J* = 247.6 Hz), 158.17, 133.91 (d, *J* = 8.2 Hz), 131.23 (d, *J* = 8.0 Hz), 124.18 (d, *J* = 3.2 Hz), 120.35 (d, *J* = 21.3 Hz), 115.32 (d, *J* = 24.1 Hz); HPLC-MS [M+H]⁺ m/z calc. for [C₈H₆FN₄]⁺: 177.05; Found: 177.54.

### Example 32

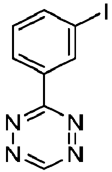

**3-(3-iodophenyl)-1,2,4,5-tetrazine** (RGV_53): The final compound was obtained from 3-iodobenzonitrile (458 mg, 4 mmol) following *General Procedure* C. The crude was purified using flash chromatography (90/10 n-Heptane/EtOAc) to yield 0.19 g (33%) of a pink solid. Rf = 0.36 (n-Heptane:10%EtOAC); ¹H NMR (400 MHz, Chloroform-d) δ 10.25 (s, 1H), 9.00 (t, *J* = 1.7 Hz, 1H), 8.60 (ddd, *J* = 7.9, 1.7, 1.1 Hz, 1H), 7.99 (ddd, *J* = 7.9, 1.8, 1.0 Hz, 1H), 7.35 (t, J= 7.9 Hz, 1H); ¹³C NMR (101 MHz, CDCl₃) δ 165.47, 158.17, 142.11, 137.21, 133.63, 131.08, 127.51, 95.02.

### Example 33

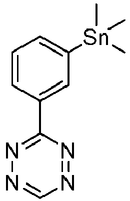

**3-(3-trimethyltin)-1,2,4,5-tetrazine (RGV_54).** The final compound was obtained from 50 mg (0.17 mmol) of the starting material, following the *General Procedure D.1.* The crude was purified using flash chromatography (90/10 n-Heptane/EtOAc) to yield 0.28 g (58%) of a pink solid. *Rf* = 0.30 (n-Heptane:10%EtOAc); ¹H NMR (400Hz, Chloroform-*d*) δ 10.14 (s,1H), 8.67 (s, 1H), 8.48 (s, J=7.9 Hz, 1H) 7.72 (d, J=7.5 Hz, 1H) 7.50 (t, J=7.9, 15.8 Hz, 1H) 0.30 (s, 9H); ¹³C NMR (101 MHz, Chloroform-d) δ 166.83, 157.75, 144.16, 140.60, 135.48, 130.96, 128.74 128.17, -9.38.; HPLC-MS [M+H]⁺ m/z calc. for [C₁₁H₁₅SnN₄]⁺: 323.04; Found: 323.38.

### Example 34 - Radiolabeling of RGV_52 (9):

The radiolabelling of RGV_49 was provided as described Example 128, 129 and 130 and as shown in Table 1 ([¹⁸F]9). In Table 1, the following letters in brackets defines the following: [a] Radiochemical conversion (RCC) and Radiochemical purity (RCP) were determined by radio-HPLC (n=3); [b] Radiochemical yield (RCY) was decay corrected to the starting amount of radioactivity received from the cyclotron and the isolated product without formulation step (n=3); [c] Second order rate constants estimated from stopped-flow measurements of the alkyne-Tz building block with TCO at 25°C in CAN; [d] No product formed or could not be isolated.

### Example 35

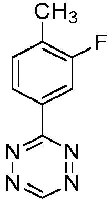

**3-(3-fluoro-4-methylphenyl)-1,2,4,5-tetrazine (UB-007).** The final compound was obtained from 3-fluoro-4-methylbenzonitrile (0.54 g, 4.00 mmol) following *General Procedure C*. The crude was purified using flash chromatography (95/5 n-Heptane/EtOAc) to yield after crystallization with n-Heptane 0.21 g (28%) of a red solid. *Rf* = 0.4 (n-Heptane:20%EtOAc); ¹H NMR (400 MHz, Chloroform-d) δ 10.21 (s, 1H), 8.33 (dd, *J* = 8.0, 1.7 Hz, 1H), 8.27 (dd, *J* = 10.5, 1.7 Hz, 1H), 7.43 (t, *J* = 7.8 Hz, 1H), 2.41 (s, 3H); ¹³C NMR (101 MHz, Chloroform-d) δ 165.80, 161.82 (d, *J* = 246.1 Hz), 157.83, 132.50 (d, *J* = 8.3 Hz), 131.11 (d, *J* = 8.3 Hz), 130.94 (d, *J* = 17.4 Hz), 123.78 (d, *J*= 3.5 Hz), 114.69 (d, *J*= 25.1 Hz), ; HPLC-MS [M+H]⁺ m/z calc. for [C₉H₈FN₄]⁺: 191.07; found 191.11 .

### Example 36 Radiolabeling of UB-007:

The radiolabelling of UB007 was provided as described in Example 128, 129 and 130 and as shown in Table 2 annexed to this description. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

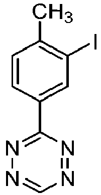

**3-(3-iodo-4-methylphenyl)-1,2,4,5-tetrazine (RGV_100).** The final compound was obtained from 3-iodo-4-methylbenzonitrile (972 mg, 4.00 mmol) following *General Procedure C*. The crude was purified using flash chromatography (95/5 n-Heptane/EtOAc) followed by recrystallization from n-Heptane afforded 0.21 g (18%) of a red solid. *Rf* = 0.42 (n-Heptane:10%EtOAc); ¹H NMR (600 MHz, Chloroform-*d*) δ 10.21 (s, 1H), 9.08 (s, 1H), 8.50 (d, *J* = 9.7 Hz, 1H), 7.46 (d, *J* = 8.0 Hz, 1H), 2.56 (s, 3H); ¹³C NMR (151 MHz, CDCl₃) δ 165.24, 157.88, 147.21, 138.55, 130.72, 130.45, 127.85, 101.69, 28.46.

### Example 37

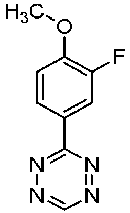

**3-(3-fluoro-4-methoxyphenyl)-1,2,4,5-tetrazine (UB-008).** The final compound was obtained from 3-Fluoro-4-methoxylbenzonitrile (0.60 g, 4.00 mmol) following *General Procedure C*. The crude was purified using flash chromatography (95/5 n-Heptane/EtOAc) followed by recrystallization from n-Heptane afforded 0.24 g (29%) of a red solid. *Rf* = 0.39 (n-Heptane:20%EtOAC); ¹H NMR (400 MHz, Chloroform-d) δ 10.18 (s, 1H), 8.45 (ddd, *J* = 8.6, 2.1, 1.3 Hz, 1H), 8.38 (dd, *J* = 12.1, 2.2 Hz, 1H), 7.18 (t, *J* = 8.5 Hz, 1H), 4.04 (s, 3H); ¹³C NMR (101 MHz, Chloroform-*d*) δ 165.53, 157.51, 152.72 (d, *J* = 247.7 Hz), 152.06 (d, *J* = 10.7 Hz), 125.29 (d, *J* = 3.6 Hz), 124.35 (d, *J* = 7.2 Hz), 115.80 (d, *J* = 20.8 Hz), 113.52 (d, *J* = 2.2 Hz), 56.36; HPLC-MS [M+H]⁺ m/z calc. for [C₉H₈FN₄O]⁺: 207.06; found 207.08

### Example 38

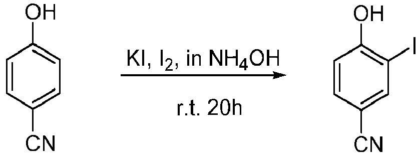

**4-hydroxy-3-iodobenzonitrile.** The preparation of this intermediates, was performed using a method described previously.^{[16]} To a solution of 4-hydroxybenzonitrile (4 g, 33.6 mmol) in 25% NH₄OH (180 mL) was added a mixture of KI (27.31 g, 167.9 mmol), I₂ (9.38 g, 36.9 mmol) in H₂O (40 mL). The reaction was allowed to stir at r.t. for 20 hours, in which time the mixture colour gradually turned from black to a white thick suspension. The precipitate formed was filtered off and the filtrate concentrated. The residue was then dissolved in CH₂Cl₂ and washed with H₂O, saturated aqueous Na₂S₂O₃ solution, and brine. The organic layer was dried over anhydrous MgSO₄, filtered and concentratedunder reduced pressure. The crude was purified using flash chromatography (90/10 n-Heptane/EtOAc) to yield the product 6.52 g (79%). ¹H NMR (400 Hz, Chloroform-d) δ 7.97 (d, *J* = 1.9 Hz, 1H), 7.55 (dd, *J* = 8.5, 1.95 Hz, 1H), 7.05 (d, *J* = 8.5 Hz, 1H), 5.87 (s, 1H); ¹³C NMR (600 Hz, CDCl₃) δ 158.72, 142.17, 134.28, 117.30, 116.31, 106.02, 85.48.

### Example 39

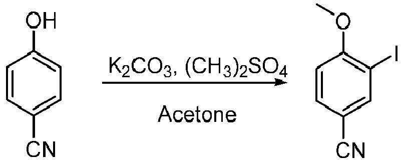

**3-iodo-4-methoxybenzonitrile.** The preparation of this intermediate, was performed using a method described previously.^{[17]} 4-Hydroxy-3-iodobenzonitrile (6.52 g, 26.6 mmol) and K₂CO₃ (11.03 g, 79.8 mmol) were suspended in acetone (130 mL) before (CH₃)₂SO₄ (5.03 g, 79.8 mmol) was added. Then the flask is fitted with a reflux condenser and the mixture was heated to 70°C for 90 minutes after which the mixture became a pale yellow thick suspension. After cooling to room temperature, the mixture was filtered, and the filter cake was washed with additional acetone. The solvent was removed on a rotary evaporator and the residue was suspended in water (250 mL) for 90 minutes. The precipitate was filtered off and dried overnight. The crude was purified using flash chromatography (90/10 n-Heptane/EtOAc) to yield the product 6.06 g (88%). ¹H NMR (400 Hz, Chloroform-d) δ 8.05 (d, *J* = 2.0 Hz, 1H), 7.64 (dd, *J* = 8.6, 2.0 Hz, 1H), 6.85 (d, *J* = 8.6 Hz,1H), 3.95 (s, 3H); ¹³C NMR (600 Hz, CDCl₃) δ 161.56, 142.81, 134.08, 117.55, 110.71, 105.94, 86.04, 56.72.

### Example 40

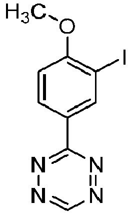

**3-(3-iodo-4-methoxyphenyl)-1,2,4,5-tetrazine (RGV_106):** The final compound was obtained from 3-iodo-4-methoxylbenzonitrile (1.03 g, 4.00 mmol) following *General Procedure C*. The crude was purified using flash chromatography (95/5 n-Heptane/EtOAc) to yield after recrystallization with n-Heptane 0.26 g (20%) as a red solid. *Rf* = 0.30 (n-Heptane:20%EtOAc); ¹H NMR (600 MHz, Chloroform-*d*) δ 10.16 (s, 1H), 9.07 (s, 1H), 8.62 (dd, *J* = 8.7, 2.1 Hz, 1H), 7.01 (d, *J* = 8.7 Hz, 1H), 4.01 (s, 3H); ¹³C NMR (151 MHz, CDCl₃) δ 165.20, 162.20, 157.70, 139.67, 130.28, 125.84, 111.17, 86.92, 56.85.

### Example 41

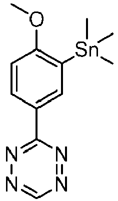

**3-(4-methoxy-3-(trimethylstannyl)phenyl)-1,2,4,5-tetrazine (RGV_109).** The final compound was obtained from 50 mg (0.17 mmol) of the starting material, following the *General Procedure D.1.* The crude was purified using flash chromatography (90/10 n-Heptane/EtOAc) to yield 0.20 g (36%) of a pink solid. Rf = 0.28 (n-Heptane:10%EtOAc); ¹H NMR (400Hz, Chloroform-*d*) δ 8.67-8.65 (m, 2H), δ 8.59 (d, J= 8.1 Hz, 2H), δ 7.76 (d, J= 8.1 Hz, 2H), δ 8.67-8.65 (m, 3H), δ 0.37 (s, 9H); ¹³C NMR (151 MHz, Chloroform-*d*) δ

### Example 42 of UB-008:

The radiolabelling of UB008 was provided as described in Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 43

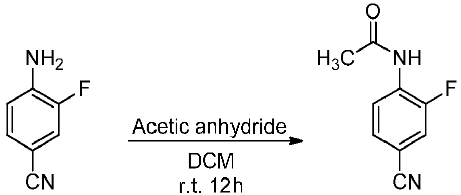

**N-(4-cyano-2-fluorophenyl)acetamide:** The preparation of this intermediate, was performed using a method described previously.^{[18]} To a solution of 4-amino-3-fluorobenzonitrile (0.82 g, 6.00 mmol) in CH₂Cl₂ (30.0 mL) was added acetic anhydride (0.80 mL, 8.40 mmol). The mixture was stirred at room temperature for 12 hours. The suspension was filtered, and the solvent removed under reduced pressure. Purification by flash chromatography (70/30 n-Heptane/EtOAc) afforded 0.90 g of N-(4-cyano-2-fluorophenyl)acetamide as a white solid. *Rf* = 0.27 (n-Heptane:40%EtOAC); ¹H NMR (400 MHz, DMSO-*d*₆) δ 10.12 (s, 1H), 8.28 (t, *J* = 8.2 Hz, 1H), 7.88 (dd, *J* = 11.1, 1.9 Hz, 1H), 7.65 (dt, *J* = 8.5, 1.3 Hz, 1H), 2.15 (s, 3H); ¹³C NMR (101 MHz, DMSO-*d*₆) δ 169.9, 152.05 (d, *J* = 247.2 Hz), 132.10 (d, *J* = 11.2 Hz), 129.82 (d, *J* = 3.6 Hz), 123.31 (d, *J* = 2.9 Hz), 119.77 (d, *J* = 23.4 Hz), 118.36 (d, *J* = 2.7 Hz), 106.18 (d, *J* = 9.4 Hz), 24.3.

### Example 44

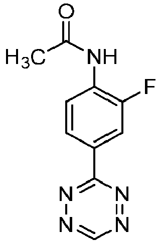

**N-(2-Fluoro-4-(1,2,4,5-tetrazin-3-yl)phenyl)acetamide (UB-148):** The final compound was obtained from N-(4-cyano-2-fluorophenyl)acetamide (0.71 g, 4.00 mmol) following *General Procedure C*. The crude was purified using flash chromatography (60/40 n-Heptane/EtOAc) to yield 0.37 g (40%) of UB-148 as a red solid. Rf= 0.25 (n-Heptane:40%EtOAC); ¹H NMR (400 MHz, DMSO-*d*₆) δ 10.58 (s, 1H), 10.09 (s, 1H), 8.45 - 8.21 (m, 3H), 2.18 (s, 3H); ¹³C NMR (101 MHz, DMSO-*d*₆) δ 169.79, 164.91 (d, *J* = 3.0 Hz), 158.44, 153.21 (d, *J* = 246.0 Hz), 131.38 (d, *J* = 11.3 Hz), 128.01 (d, *J* = 7.9 Hz), 124.72 (d, *J* = 3.3 Hz), 123.67, 114.75 (d, *J* = 22.1 Hz), 24.3; HPLC-MS [M+H]⁺ m/z calc. for [C₁₀H₉FN₅O]⁺: 234.08; found 234.10

### Example 45

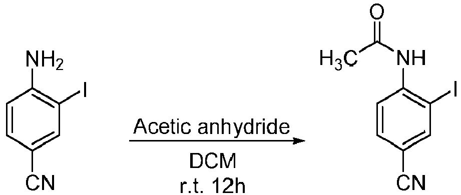

**N-(4-cyano-2-iodophenyl)acetamide (RGV_136):** The preparation of this intermediate, was performed using a method described previously.^{[18]} To a solution of the corresponding aniline (1.5 g, 6.00 mmol) in CH₂Cl₂ (30.0 mL) was added acetic anhydride (0.85 mL, 9 mmol). The mixture was stirred at room temperature for 12 hours. The suspension was filtered, and the solvent removed under vacuum. Purification by flash chromatography (70/30 n-Heptane/EtOAc) afforded 0.90 g (52%) of RGV_136 as a white solid. *Rf* = 0.5 (n-Heptane:40%EtOAc); ¹H NMR (600 MHz, DMSO-*d*₆) δ 9.50 (s, 1H), 8.36 (d, *J* = 1.9 Hz, 1H), 7.82 (dd, *J* = 8.4, 1.9 Hz, 1H), 7.73 (d, *J* = 8.4 Hz, 1H), 2.12 (s, 3H).; ¹³C NMR (151 MHz, DMSO) δ 168.72, 144.08, 142.44, 132.41, 125.76, 117.29, 108.88, 94.41, 23.51.

### Example 46

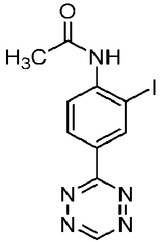

**N-(2-iodo-4-(1,2,4,5-tetrazin-3-yl)phenyl)acetamide (RGV_144):** The final compound was obtained from N-(4-cyano-2-iodophenyl)acetamide (1.14 g, 4.00 mmol) following *General Procedure C*. The crude was purified using flash chromatography (60/40 n-Heptane/EtOAc) to yield 0.29 g (21%) of RGV_144 as a pink solid. *Rf* = 0.29 (n- Heptane:40%EtOAc); ¹H NMR (600 MHz, Chloroform-d) δ 10.20 (s, 1H), 9.07 (s, 1H), 8.62 (d, *J* = 8.7 Hz, 1H), 8.58 (d, *J* = 8.6 Hz, 1H), 7.71 (s, 1H), 2.32 (s, 2H).; ¹³C NMR (151 MHz, CDCl₃) δ 168.57, 164.98, 157.88, 142.56, 138.78, 129.60, 128.51, 121.17, 25.27, 1.16.

### Example 47

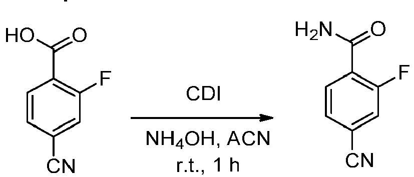

**4-Cyano-2-fluorobenzamide (UB-20).** To a solution of 4-cyano-2-fluorobenzoic acid (0.99 g, 6.0 mmol) in acetonitrile (20 ml) was added 1,1'-carbonyldiimidazole (1.46 g, 9.0 mmol). The mixture was stirred at room temperature for 45 minutes, before addition of aqueous ammonium hydroxide solution (35%, 20 ml). The reaction mixture was stirred for 45 minutes and ice-cold water (15 ml) was added. The precipitate was collected by filtration and dried to give the 0.78 g (79%) of UB-20 as a white solid. *Rf* = 0.25 (n-Heptane:60%EtOAC); ¹H NMR (400 MHz, DMSO-*d*₆) δ 8.02 - 7.91 (m, 2H), 7.86 (s, 1H), 7.80 - 7.74 (m, 2H); ¹³C NMR (101 MHz, DMSO-*d*₆) δ 164.56, 158.89 (d, *J* = 251.4 Hz), 131.56 (d, *J* = 4.0 Hz), 129.60 (d, *J* = 15.7 Hz), 129.16 (d, *J* = 4.0 Hz), 120.79 (d, *J* = 26.7 Hz), 117.68 (d, *J* = 2.8 Hz), 114.58 (d, *J* = 10.0 Hz).

### Example 48

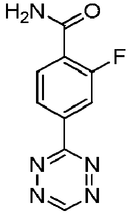

**2-Fluoro-4-(1,2,4,5-tetrazin-3-yl)benzamide (UB-22).** The final compound was obtained from 4-cyano-2-fluorobenzamide (0.78 g, 4.75 mmol) following *General Procedure C*. The crude was purified using flash chromatography (98/2 CH₂Cl₂/MeOH) to yield 0.21 g (20%) of UB22 as a red solid. *Rf* = 0.30 (n-Heptane:60%EtOAC); ¹H NMR (400 MHz, DMSO-*d*₆) δ 10.68 (s, 1H), 8.39 (dd, *J* = 8.0, 1.6 Hz, 1H), 8.30 (dd, *J* = 11.1, 1.6 Hz, 1H), 7.97 (s, 1H), 7.93 (t, *J* = 7.7 Hz, 1H), 7.83 (s, 1H); ¹³C NMR (101 MHz, DMSO-*d*₆) δ 165.07, 164.84 (d, *J* = 2.9 Hz), 159.91 (d, *J* = 250.1 Hz), 158.83, 136.15 (d, *J* = 8.5 Hz), 131.81 (d, *J* = 3.4 Hz), 128.25 (d, *J* = 15.2 Hz), 124.16 (d, *J* = 3.4 Hz), 115.57 (d, *J* = 25.4 Hz); HPLC-MS [M+H]⁺ m/z calc. for [C₉H₇FN₅O]⁺: 220.06; found 226.07.

### Example 49

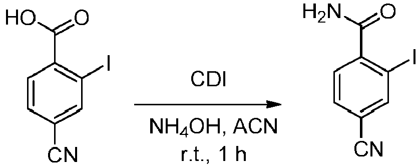

**4-Cyano-2-iodobenzamide (UB-282).** To a solution of 4-cyano-2-iodobenzoic acid (1.00 g, 3.66 mmol) in acetonitrile (20 ml) was added 1,1'-carbonyldiimidazole (0.89 g, 5.49 mmol). The mixture was stirred at room temperature for 45 minutes, before addition of aqueous ammonium hydroxide solution (35%, 20 ml). The reaction mixture was stirred for 45 minutes and ice-cold water (15 ml) was added. The precipitate was collected by filtration and dried to give the 0.89 g (89%) of UB-282 as a white solid. *Rf* = 0.23 (n-Heptane:60%EtOAC); ¹H NMR (400 MHz, DMSO-*d*₆) δ 8.37 (d, *J* = 1.5 Hz, 1H), 7.98 (s, 1H), 7.91 (dd, *J* = 7.9, 1.6 Hz, 1H), 7.72 (s, 1H), 7.48 (d, *J* = 7.8 Hz, 1H); ¹³C NMR (101 MHz, DMSO-*d*₆) δ 170.11, 148.12, 142.53, 132.32, 128.42, 117.40, 113.37, 94.10.

### Example 50

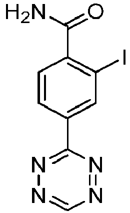

**2-lodo-4-(1,2,4,5-tetrazin-3-yl)benzamide (UB-283).** The final compound was obtained from 4-cyano-2-iodobenzamide (0.50 g, 1.83 mmol) following *General Procedure* C. The crude was purified using flash chromatography (98/2 CH₂Cl₂/MeOH) to yield 0.25 g (41%) of UB-283 as a red solid. *Rf* = 0.32 (n-Heptane:60%EtOAC); ¹H NMR (400 MHz, DMSO-*d*₆) δ 10.66 (s, 1H), 8.89 (d, *J* = 1.6 Hz, 1H), 8.52 (dd, *J* = 8.0, 1.7 Hz, 1H), 8.02 (s, 1H), 7.71 (s, 1H), 7.63 (d, *J* = 8.0 Hz, 1H); ¹³C NMR (101 MHz, DMSO-*d*₆) δ 170.57, 164.55, 158.77, 147.21, 138.45, 134.16, 128.99, 127.70, 94.43.

### Example 51

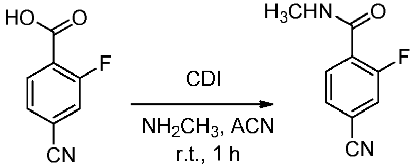

**4-Cyano-2-fluoro-N-methylbenzamide (UB-198).** To a solution of 4-cyano-2-fluorobenzoic acid (0.99 g, 6.0 mmol) in acetonitrile (20 ml) was added 1,1'-carbonyldiimidazole (1.46 g, 9.0 mmol). The mixture was stirred at room temperature for 45 minutes, before addition of aqueous methylamine solution (40%, 20 ml).The reaction mixture was stirred for 45 minutes and ice cold water (15 ml) was added. The precipitate was collected by filtration and dried to give the 0.86 g (80%) of UB-198 as a white solid. *Rf* = 0.29 (n-Heptane:60%EtOAC); ¹H NMR (400 MHz, DMSO-*d*₆) δ 8.50 (d, *J* = 6.3 Hz, 1H), 8.02 - 7.92 (m, 1H), 7.81 - 7.71 (m, 2H), 2.79 (d, *J* = 4.6 Hz, 3H); ¹³C NMR (101 MHz, DMSO-*d*₆) δ 163.25, 158.82 (d, *J* = 251.2 Hz), 131.56 (d, *J* = 3.8 Hz), 129.54 (d, *J* = 15.6 Hz), 129.23 (d, *J* = 3.9 Hz), 120.78 (d, *J* = 26.6 Hz), 117.67 (d, *J* = 2.9 Hz), 114.55 (d, *J* = 10.1 Hz), 26.69.

### Example 52

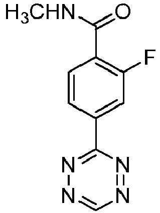

**2-Fluoro-N-methyl-4-(1,2,4,5-tetrazin-3-yl)benzamide (UB-204).** The final compound was obtained from 4-cyano-2-fluoro-N-methylbenzamide (0.77 g, 4.32 mmol) following *General Procedure C.* The crude was purified using flash chromatography (98/2 CH₂Cl₂/MeOH) to yield 0.36 g (36%) of UB-204 as a red solid. *Rf* = 0.35 (n-Heptane:60%EtOAC); ¹H NMR (400 MHz, DMSO-*d*₆) δ 10.68 (s, 1H), 8.50 (d, *J* = 5.3 Hz, 1H), 8.40 (dd, *J* = 8.1, 1.6 Hz, 1H), 8.30 (dd, *J* = 11.1, 1.6 Hz, 1H), 7.91 (t, *J* = 7.7 Hz, 1H), 2.83 (d, *J* = 4.6 Hz, 3H); ¹³C NMR (101 MHz, DMSO-*d*₆) δ 164.83 (d, *J* = 2.9 Hz), 163.79, 159.81 (d, *J* = 249.8 Hz), 158.82, 136.08 (d, *J* = 8.5 Hz), 131.76 (d, *J* = 3.5 Hz), 128.25 (d, *J* = 15.4 Hz), 124.23 (d, *J* = 3.3 Hz), 115.57 (d, *J* = 25.4 Hz), 26.75; HPLC-MS [M+H]⁺ m/z calc. for [C_{1O}H₉FN₅O]⁺: 234.08; found 234.10.

### Example 53

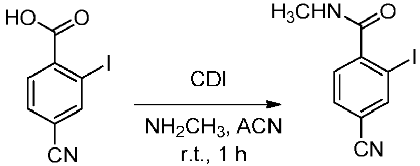

**4-Cyano-2-iodo-N-methylbenzamide (UB-306).** To a solution of 4-cyano-2-iodobenzoic acid (1.00 g, 3.66 mmol) in acetonitrile (20 ml) was added 1,1'-carbonyldiimidazole (0.89 g, 5.49 mmol). The mixture was stirred at room temperature for 45 minutes, before addition of aqueous methylamine solution (40%, 20 ml). The reaction mixture was stirred for 45 minutes and ice-cold water (15 ml) was added. The precipitate was collected by filtration and dried to give the 0.85 g (81%) of UB-306 as a white solid. *Rf* = 0.31 (n-Heptane:60%EtOAC); ¹H NMR (400 MHz, DMSO-*d*₆) δ 8.44 (d, *J* = 4.8 Hz, 1H), 8.38 (d, *J* = 1.5 Hz, 1H), 7.91 (dd, *J* = 7.8, 1.6 Hz, 1H), 7.47 (d, *J* = 7.8 Hz, 1H), 2.76 (d, *J* = 4.6 Hz, 3H); ¹³C NMR (101 MHz, DMSO-*d*₆) δ 168.77, 148.09, 142.44, 132.36, 128.67, 117.37, 113.50, 94.63, 26.41.

### Example 54

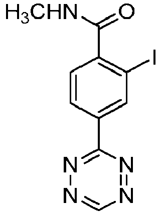

**2-lodo-N-methyl-4-(1,2,4,5-tetrazin-3-yl)benzamide (UB-318).** The final compound was obtained from 4-cyano-2-iodo-N-methylbenzamide (0.60 g, 2.09 mmol) following *General Procedure C*. The crude was purified using flash chromatography (98/2 CH₂Cl₂/MeOH) to yield 0.25 g (35%) of UB-318 as a red solid. *Rf* = 0.20 (n-Heptane:60%EtOAC); ¹H NMR (400 MHz, DMSO-*d*₆) δ 10.66 (s, 1H), 8.89 (d, *J* = 1.6 Hz, 1H), 8.57 - 8.44 (m, 2H), 7.60 (d, *J* = 8.0 Hz, 1H), 2.81 (d, *J* = 4.6 Hz, 3H); ¹³C NMR (101 MHz, DMSO-*d*₆) δ 169.20, 164.54, 158.77, 147.24, 138.33, 134.27, 129.20, 127.73, 94.92, 26.47.

### Example 55

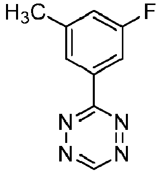

**3-(3-fluoro-5-methylphenyl)-1,2,4,5-tetrazine (UB-052).** The final compound was obtained from 3-Fluoro-5-methylbenzonitrile (0.54 g, 4.00 mmol) following *General Procedure C*. The crude was purified using flash chromatography (95/5 n-Heptane/EtOAc) to yield 0.26 g (34%) of a red oil. *Rf* = 0.39 (n-Heptane:20%EtOAc); ¹H NMR (400 MHz, Chloroform-d) δ 10.16 (s, 1H), 8.19 (d, *J* = 1.4 Hz, 1H), 8.05 (d, *J* = 9.4 Hz, 1H), 7.10 (d, *J* = 9.2 Hz, 1H), 2.43 (s, 3H); ¹³C NMR (101 MHz, Chloroform-*d*) δ 165.83 (d, *J* = 3.4 Hz), 163.30 (d, *J* = 246.8 Hz), 157.95, 141.81 (d, *J* = 7.7 Hz), 133.31 (d, *J* = 8.9 Hz), 124.67 (d, *J* = 2.7 Hz), 120.79 (d, *J* = 21.2 Hz), 112.29 (d, *J* = 24.3 Hz), 21.45 (d, *J* = 1.8 Hz); HPLC-MS [M+H]⁺ m/z calc. for [C₉H₈FN_{4]}⁺: 191.07; found 191.08.

### Example 56

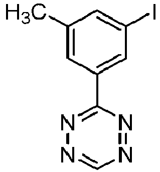

**3-(3-iodo-5-methylphenyl)-1,2,4,5-tetrazine (RGV_114).** The final compound was obtained from 3-iodo-5-methylbenzonitrile (0.97 g, 4.00 mmol) following *General Procedure C*. The crude was purified using flash chromatography (95/5 n-Heptane/EtOAc) to yield after recrystallization with n-Heptane 0.27 g (22%) as a red solid. *Rf* = 0.45 (n-Heptane:20%EtOAc ¹H NMR (400 MHz, Chloroform-*d*) δ 10.22 (s, 1H), 8.83 - 8.67 (m, 1H), 8.44 - 8.28 (m, 1H), 7.87 - 7.74 (m, 1H), 2.43 (s, 2H); ¹³C NMR (151 MHz, CDCl₃) δ 165.49, 158.09, 142.71, 141.41, 134.34, 133.30, 128.22, 94.99, 21.21.

### Example 57

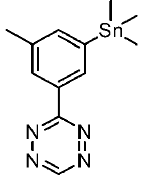

**3-(3-methyl-5-(trimethylstannyl)phenyl)-1,2,4,5-tetrazine (RGV_123).** The final compound was obtained from 50 mg (0.17 mmol) of the starting material, following the *General Procedure D.2.* The crude was purified using flash chromatography (90/10 n-Heptane/EtOAc) to yield 0.25 g (27%) of a pink solid. *Rf* = 0.34 (n-Heptane:20%EtOAc); ¹H NMR (400 MHz, Chloroform-*d*) δ 10.20 (s, 1H), 8.65 - 8.50 (m, 1H), 8.48 - 8.33 (m, 1H), 7.73 - 7.43 (m, 1H), 2.48 (s, 3H), 0.36 (s, 9H); ¹³C NMR (151 MHz, CDCl₃) δ 167.05, 157.86, 144.08, 141.58, 138.56, 132.79, 131.00, 128.91, 21.58, -9.25.; HPLC-MS [M+H]⁺ m/z calc. for [C₁₂H₁₆N₄Sn]⁺: 337.05; Found: 337.1.

### Example 58 of UB-052:

The radiolabelling of UB-052 was provided as described in Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 59

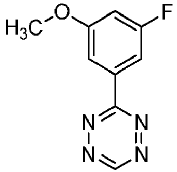

**3-(3-fluoro-5-methoxyphenyl)-1,2,4,5-tetrazine (UB-048).** The final compound was obtained from 3-fluoro-5-methoxylbenzonitrile (0.60 g, 4.00 mmol) following *General Procedure C*. The crude was purified using flash chromatography (85/15 n-Heptane/EtOAc) and recrystallized from n-Heptane to 0.21 g (26%) of UB-48 as a red solid. *Rf* = 0.41 (n-Heptane:20%EtOAc); ¹H NMR (400 MHz, Chloroform-d) δ 10.17 (s, 1H), 7.91 (d, *J* = 2.0 Hz, 1H), 7.87 (ddd, *J* = 9.1, 2.4, 1.4 Hz, 1H), 6.83 (dd, *J* = 10.1, 2.4 Hz, 1H), 3.86 (s, 3H); ¹³C NMR (101 MHz, Chloroform-d) δ 165.63 (d, *J* = 3.9 Hz), 164.07 (d, *J* = 246.5 Hz), 161.75 (d, *J* = 11.4 Hz), 158.02, 133.96 (d, *J* = 10.7 Hz), 108.96 (d, *J* = 2.8 Hz), 107.73 (d, *J* = 24.7 Hz), 106.98 (d, *J* = 24.9 Hz), 55.96; HPLC-MS [M+H]⁺ m/z calc. for [C₉H₈FN₄O]⁺: 207.07; found 207.05

### Example 60

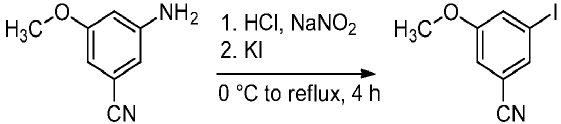

**3-lodo-5-methoxybenzonitrile (UB-224).** Concentrated. HCI (3 mL) was added to a solution of aniline (1.00 g, 6.75 mmol) in water (3 mL) at 0 °C. To this was added a chilled solution of sodium nitrite (0.84 g, 12.15 mmol) in water (4 mL), dropwise, with vigorous mechanical stirring. Stirring was continued at 0°C for 15 min. after the addition was complete, and then a solution of potassium iodide (2.24 g, 13.50 mmol) in water (4 mL) was added carefully. The cooling bath was removed, and the reaction heated to reflux. When the production of purple vapor ceased, the mixture was cooled to rt and extracted with DCM (3×20 mL). The combined organic extracts were dried (MgSO₄), filtered, and concentrated under reduced pressure. Purification by flash chromatography (90/10 Heptane/EtOAc) afforded 0.60 g (34%) of UB-224 as a white solid. *Rf* = 0.34 (n-Heptane:20%EtOAc); ¹H NMR (400 MHz, Chloroform-d) δ 7.48 (t, *J* = 1.4 Hz, 1H), 7.40 (dd, *J* = 2.4, 1.4 Hz, 1H), 7.04 (dd, *J* = 2.4, 1.4 Hz, 1H), 3.75 (s, 3H); ¹³C NMR (101 MHz, Chloroform-*d*) δ 159.92, 132.68, 128.19, 116.85, 114.60, 94.14, 55.83.

### Example 61

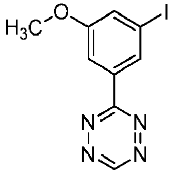

**3-(3-lodo-5-methoxyphenyl)-1,2,4,5-tetrazine (UB-225).** The final compound was obtained from 3-iodo-5-methoxylbenzonitrile (0.52 g, 2.00 mmol) following *General Procedure C*. The crude was purified using flash chromatography (85/15 n-Heptane/EtOAc) and recrystallized from n-Heptane 0.19 g (30%) of UB-225 as a red solid. *Rf* = 0.25 (n-Heptane:20%EtOAc); ¹H NMR (400 MHz, Chloroform-*d*) δ 10.17 (s, 1H), 8.49 (t, *J* = 1.4 Hz, 1H), 8.03 (dd, *J* = 2.4, 1.4 Hz, 1H), 7.44 (dd, *J* = 2.5, 1.5 Hz, 1H), 3.83 (s, 3H); ¹³C NMR (101 MHz, Chloroform-*d*) δ 165.13, 160.72, 158.02, 134.10, 129.53, 128.40, 112.47, 94.96, 55.80.

### Example 62

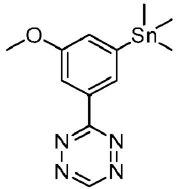

**3-(3-methoxy-5-(trimethylstannyl)phenyl)-1,2,4,5-tetrazine (UB-229).** The final compound was obtained from 55 mg (0.17 mmol) of the starting material, following the *General Procedure D.2.* The crude was purified using flash chromatography (90/10 n-Heptane/EtOAc) to yield 0.040 g (65%) of UB-229 as a purple solid. *Rf* = 0.41 (n-Heptane:20%EtOAc); ¹H NMR (400 MHz, Chloroform-*d*) δ 10.21 (s, 1H), 8.35 (d, *J* = 1.5 Hz, 1H), 8.07 (dd, *J* = 2.7, 1.6 Hz, 1H), 7.32 (d, *J* = 2.7 Hz, 1H), 3.94 (s, 3H), 0.37 (s, 9H); ¹³C NMR (101 MHz,

### Example 63 of UB-048:

The radiolabelling of UB-048 was provided as described in the Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 64

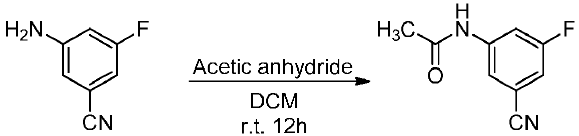

**N-(5-Cyano-3-fluorophenyl)acetamide (UB-149).** To a solution of 3-amino-5-fluorobenzonitrile (0.82 g, 6.00 mmol) in DCM (30.0 mL) was added acetic anhydride (0.80 mL, 8.40 mmol). The mixture was stirred at room temperature for 12 h. The suspension was filtered, and the solvent removed under vacuum. Purification by flash chromatography (70/30 n-Heptane/EtOAc) afforded 0.92 g of N-(5-cyano-3-fluorophenyl)acetamide as a white solid. *Rf* = 0.31 (n-Heptane:40%EtOAc); ¹H NMR (400 MHz, DMSO-*d*₆) δ 10.45 (s, 1H), 7.86 - 7.70 (m, 2H), 7.57 - 7.37 (m, 1H), 2.09 (s, 3H); ¹³C NMR (101 MHz, DMSO-*d*₆) δ 169.69, 162.24 (d, *J* = 244.3 Hz), 142.35 (d, *J* = 11.8 Hz), 118.65, 118.09 (d, *J* = 3.6 Hz), 113.70 (d, *J* = 25.5 Hz), 113.25 (d, *J* = 12.1 Hz), 110.95 (d, *J* = 26.2 Hz), 24.52.

### Example 65

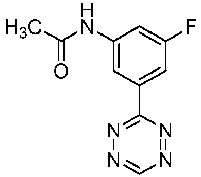

**N-(3-Fluoro-5-(1,2,4,5-tetrazin-3-yl)phenyl)acetamide (UB-150).** The final compound was obtained from N-(5-cyano-3-fluorophenyl)acetamide (0.58 g, 3.25 mmol) following *General Procedure C.* The crude was purified using flash chromatography (60/40 n-Heptane/EtOAc) to yield 0.19 g (25%) of a red solid. *Rf* = 0.25 (n-Heptane:40%EtOAc); ¹H NMR (400 MHz, DMSO-*d*₆) δ 10.64 (s, 1H), 10.48 (s, 1H), 8.52 (t, *J* = 1.7 Hz, 1H), 7.98 - 7.81 (m, 2H), 2.12 (s, 3H); ¹³C NMR (101 MHz, DMSO-*d*₆) δ 169.54, 165.03 (d, *J* = 3.8 Hz), 163.08 (d, *J* = 242.2 Hz), 158.83, 142.45 (d, *J* = 11.5 Hz), 134.61 (d, *J* = 10.1 Hz), 114.40 (d, *J* = 2.6 Hz), 109.87 (d, *J* = 26.6 Hz), 108.74 (d, *J* = 24.4 Hz), 24.58; HPLC-MS [M+H]⁺ m/z calc. for [C₁₀H₉FN₅O]⁺: 234.08; found 234.10.

### Example 66

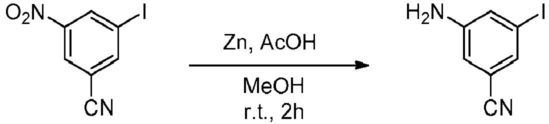

**3-Amino-5-iodobenzonitrile (UB-210).** To a solution of 3-iodo-5-nitrobenzonitrile (0.500 g, 1.82 mmol) and Zn (0.58 g, 8.87 mmol) in MeOH (20 mL) was added dropwise 1 mL of acetic acid. The reaction was stirred at room temperature for 2 h and then concentrated under reduced pressure. Purification by flash chromatography afforded 0.250 g (56%) of UB-210 as a white solid. *Rf* = 0.22 (n-Heptane:20%EtOAc); ¹H NMR (400 MHz, Chloroform-*d*) δ 7.24 (d, *J* = 1.4 Hz, 1H), 7.15 (t, *J* = 1.9 Hz, 1H), 6.81 - 6.73 (m, 1H), 3.81 (s, 2H); ¹³C NMR (101 MHz, Chloroform-*d*) δ 147.74, 129.88, 127.63, 117.39, 116.80, 114.45, 94.45.

### Example 67

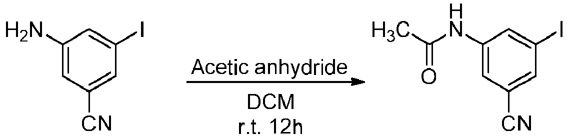

**N-(5-Cyano-3-iodophenyl)acetamide (UB-149).** To a solution of 3-amino-5-iodobenzonitrile (0.20 g, 0.81 mmol) in DCM (10.0 mL) was added acetic anhydride (0.1 mL, 1.15 mmol). The mixture was stirred at room temperature for 12 h. The suspension was filtered, and the solvent removed under vacuum. Purification by flash chromatography (70/30 n-Heptane/EtOAc) afforded 0.21 (90%) of UB-210 as a white solid. *Rf* = 0.29 (n-Heptane:40%EtOAc); ¹H NMR (400 MHz, Methanol-*d*₄) δ 8.13 (t, *J* = 1.8 Hz, 1H), 7.87 (t, *J* = 1.7 Hz, 1H), 7.66 (t, *J* = 1.5 Hz, 1H), 2.04 (s, 3H); ¹³C NMR (101 MHz, Methanol-*d*₄) δ 170.51, 140.51, 134.84, 132.16, 121.42, 116.54, 113.80, 93.18, 22.51.

### Example 68

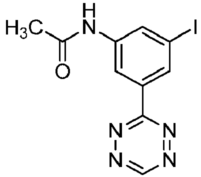

**N-(3-lodo-5-(1,2,4,5-tetrazin-3-yl)phenyl)acetamide (UB-216).** The final compound was obtained from N-(5-cyano-3-iodophenyl)acetamide (0.18 g, 0.63 mmol) following *General Procedure C*. The crude was purified using flash chromatography (60/40 n-Heptane/EtOAc) to yield 0.055 g (26%) of UB-216 as a red solid. *Rf* = 0.21 (n-Heptane:40%EtOAc); ¹H NMR (600 MHz, DMSO-*d*₆) δ 10.63 (s, 1H), 10.35 (s, 1H), 8.73 (t, *J* = 1.7 Hz, 1H), 8.44 (t, *J* = 1.5 Hz, 1H), 8.38 (t, *J* = 1.8 Hz, 1H), 2.11 (s, 3H); ¹³C NMR (151 MHz, DMSO-*d*₆) δ 169.44, 164.69, 158.81, 141.92, 134.71, 131.04, 130.64, 117.61, 95.93, 24.59.

### Example 69

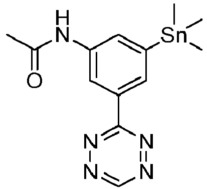

**N-(3-(1,2,4,5-tetrazin-3-yl)-5-(trimethylstannyl)phenyl)acetamide (UB-220).** The final compound was obtained from 55 mg (0.17 mmol) of the starting material, following the *General Procedure D.2.* The crude was purified using flash chromatography (70/30 n-Heptane/EtOAc) to yield 0.025 g (41%) of UB-220 as a purple oil. *Rf* = 0.35 (n-Heptane:50%EtOAc); ¹H NMR (400 MHz, Chloroform-d) δ 10.20 (s, 1H), 8.56 (t, *J* = 2.1 Hz, 1H), 8.44 (s, 1H), 8.03 (d, *J* = 2.2 Hz, 1H), 7.63 (s, 1H), 2.24 (s, 3H), 0.36 (s, 9H); ¹³C NMR (101 MHz, Chloroform-d) δ 168.68, 166.46, 157.80, 145.49, 138.40, 131.68, 131.52, 131.18, 119.43, 24.60, -9.30; HPLC-MS [M+H]⁺ m/z calc. for [C₁₃H₁₈N₅SnO]⁺: 380.05; Found: 380.09.

### Example 70 of UB-150:

The radiolabelling of UB150 was provided as described in the Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 71

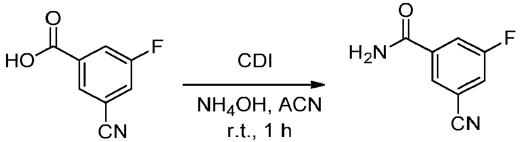

**3-Cyano-5-fluorobenzamide (UB-69).** To a solution of 5-cyano-3-fluorobenzoic acid (0.99 g, 6.0 mmol) in acetonitrile (20 ml) was added 1,1'-carbonyldiimidazole (1.46 g, 9.0 mmol). The mixture was stirred at room temperature for 45 min, before addition of aqueous ammonium hydroxide solution (35%, 20 ml). The reaction mixture was stirred for 45 min and ice-cold water (15 ml) was added. The precipitate was collected by filtration and dried to give the title compound 0.77 g (78%) of UB-69 as a white solid. *Rf* = 0.41 (n-Heptane:40%EtOAc); ¹H NMR (400 MHz, DMSO-*d*₆) δ 8.21 (s, 1H), 8.16 (d, *J* = 1.5 Hz, 1H), 8.05 (ddd, *J* = 8.4, 2.6, 1.3 Hz, 1H), 8.01 (ddd, *J* = 9.6, 2.5, 1.4 Hz, 1H), 7.78 (s, 1H); ¹³C NMR (101 MHz, DMSO-*d*₆) δ 165.12 (d, *J* = 2.4 Hz), 162.04 (d, *J* = 247.5 Hz), 138.39 (d, *J* = 7.3 Hz), 128.11 (d, *J* = 3.1 Hz), 122.37 (d, *J* = 25.7 Hz), 120.16 (d, *J* = 22.9 Hz), 117.69 (d, *J* = 3.1 Hz), 113.52 (d, *J* = 9.9 Hz).

### Example 72

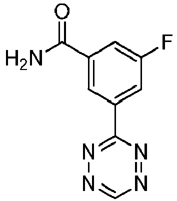

**3-Fluoro-5-(1,2,4,5-tetrazin-3-yl)benzamide (UB-70).** The final compound was obtained from 3-cyano-5-fluorobenzamide (0.75 g, 4.57 mmol) following *General Procedure C*. The crude was purified using flash chromatography (98/2 CH₂Cl₂/MeOH) to yield o yield 0.36 g (36%) of UB-70 as a pink solid. *Rf* = 0.31 (n-Heptane:60%EtOAC); ¹H NMR (400 MHz, DMSO-*d*₆) δ 10.69 (s, 1H), 8.88 (s, 1H), 8.48 - 8.20 (m, 2H), 8.16 - 7.92 (m, 1H), 7.71 (s, 1H); ¹³C NMR (101 MHz, DMSO-*d*₆) δ 166.08 (d, *J* = 2.3 Hz), 164.94 (d, *J* = 3.3 Hz), 162.85 (d, *J* = 245.6 Hz), 158.89, 138.32 (d, *J* = 6.9 Hz), 134.93 (d, *J* = 8.2 Hz), 123.56 (d, *J* = 2.9 Hz), 118.85 (d, *J* = 23.0 Hz), 117.31 (d, *J* = 24.1 Hz); HPLC-MS [M+H]⁺ m/z calc. for [C₉H₇FN₅O]⁺: 220.06; found 220.09.

### Example 73

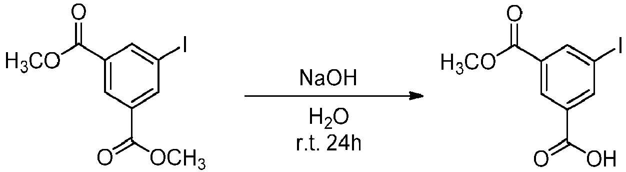

**3-lodo-5-(methoxycarbonyl)benzoic acid (UB-277).** The preparation of this intermediate, was performed using a method described previously.^{[19]} To a solution of dimethyl 5-iodoisophthalate (12.8 g, 40 mmol), methanol (80 mL), and CH₂Cl₂ (40 mL) was added NaOH (1.68 g, 42 mmol). The mixture was allowed to stir at room temperature for 24 hours. The solvents were removed under reduced pressure. Lots of white precipitate formed when water (9 mL), dichloromethane (10 mL), and ethyl acetate (10 mL) were added while stirring, which was collected by filtration, well washed with a mixture of dichloromethane (10 mL) and ethyl acetate (10 mL), and then with water (10 mL). After transferring the solid (mono sodium salt) to a separatory funnel, ethyl acetate (80 mL) and conc. HCl (3 mL) diluted with water (20 mL) were successively added. The mixture was vigorously shaken until the solid was disappeared. Then the organic layer was separated, and the aqueous layer was extracted by ethyl acetate (25 mL). The organic layers were combined and washed by brine (20 mL), dried over MgSO4, filtered, and concentrated. The solid obtained was washed recrystallized from MeOH to give 10.3 g (84%) of UB-277 as a white solid. *Rf* = 0.33 (CH₂Cl₂:5%MeOH:0.1%AcOH); ¹H NMR (400 MHz, DMSO-*d*₆) δ 13.57 (s, 1H), 8.49 - 8.27 (m, 3H), 3.89 (s, 3H); ¹³C NMR (101 MHz, DMSO-*d*₆) δ 165.55, 164.68, 142.24, 141.62, 133.67, 132.31, 129.36, 95.42, 53.15.

### Example 74

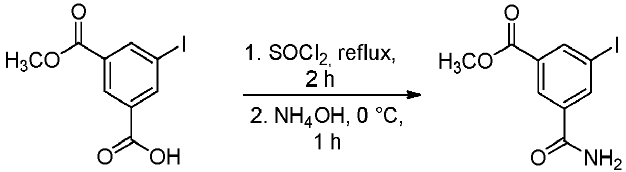

**Methyl 3-carbamoyl-5-iodobenzoate (UB-280).** A solution of 3-methoxycarbony5-iodobenzoic acid (10.3 g, 33.65 mmol) in thionyl chloride (30.0 mL) was heated for 2 hours at 60°C The reaction mixture was cooled and concentrated under reduced pressure. The intermediate acid chloride was then diluted with tetrahydrofuran (40 mL) and cooled to 0 °C The mixture was then treated with a solution of 2M ammonia (60 mL, 120 mmol, methanol) and the reaction stirred for 1 hour at 0°C. The mixture was then filtered, and the solvent removed under reduced pressure. Recrystallization from methanol afforded 7.70 g (75%) of UB-280 as a white solid. *Rf* = 0.25 (n-Heptane:40%EtOAc); ¹H NMR (400 MHz, DMSO-*d*₆) δ 8.47 (t, *J* = 1.6 Hz, 1H), 8.44 (d, *J* = 1.6 Hz, 1H), 8.35 (t, *J* = 1.6 Hz, 1H), 8.24 (s, 1H), 7.61 (s, 1H), 3.89 (s, 3H); ¹³C NMR (101 MHz, DMSO-*d*₆) δ 165.86, 164.96, 140.72, 140.21, 136.99, 132.10, 128.09, 95.18, 53.06.

### Example 75

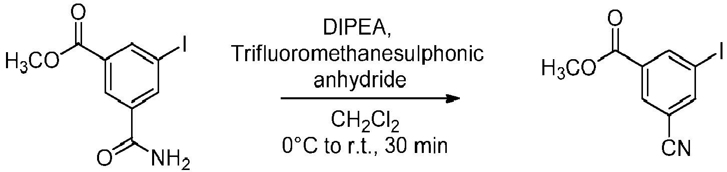

**Methyl 3-cyano-5-iodobenzoate (UB-284).** At a temperature of about 0°C, a solution of 2.8 ml (16.5 mmol) of trifluoromethanesulphonic anhydride in 50 ml of dichloromethane was added dropwise to a solution of 2.80 g (9.18 mmol) of methyl 3-carbamoyl-5-iodobenzoate and 8 ml (45.9 mmol) of N,N-diisopropylethylamine in 150 ml of dichloromethane. After a reaction time of 30 min at 0°C, 50 ml of saturated aqueous sodium bicarbonate solution were added, and the mixture was stirred vigorously at room temperature for 10 minutes. The organic phase was separated off, dried over anhydrous MgSO₄, filtered and freed from the solvent on a rotary evaporator. Purification by flash chromatography (80/20 Heptane/EtOAc) afforded 2.4 g (91%) of UB-284 as a white solid. *Rf* = 0.5 (n-Heptane:20%EtOAc); ¹H NMR (400 MHz, Chloroform-*d*) δ 8.58 (s, 1H), 8.27 (s, 1H), 8.14 (s, 1H), 3.96 (s, 3H); ¹³C NMR (101 MHz, Chloroform-d) δ 163.72, 144.07, 142.59, 132.70, 132.25, 116.25, 114.55, 93.68, 52.99.

### Example 76

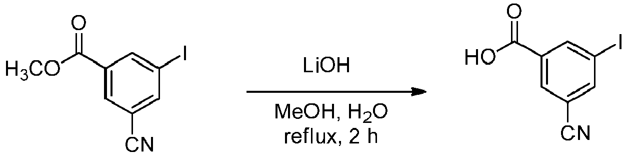

**3-Cyano-5-iodobenzoic acid (UB-285).** A solution of methy3-cyano-5-iodobenzoate (2.36 g, 8.22 mmol) in THF (25 mL) was treated with 0.5M LiOH (20 mL, 9.86 mmol) and methanol. The reaction mixture was heated at reflux for 1 hour. The solvent was concentrated in vacuo and the mixture treated with 1N HCl. The resulting white precipitate was filtered, and the filtrate was extracted with dichloromethane. The residue and the extracted filtrate were combined and concentrated in vacuo to afford 2.1 g (94%) UB-285 as a white solid. *Rf* = 0.33 (n-Heptane:40%EtOAc); ¹H NMR (400 MHz, Chloroform-*d*) δ 10.73 (s, 1H), 8.65 (s, 1H), 8.35 (s, 1H), 8.22 (s, 1H); ¹³C NMR (101 MHz, Chloroform-*d*) δ 168.43, 145.01, 143.10, 132.80, 131.74, 116.04, 114.77, 93.84.

### Example 77

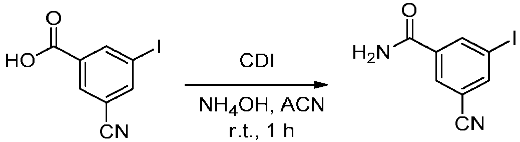

**3-Cyano-5-iodobenzamide (UB-263).** To a solution of 5-cyano-3-iodobenzoic acid (0.40 g, 1.46 mmol) in acetonitrile (10 ml) was added 1,1'-carbonyldiimidazole (0.36 g, 2.20 mmol). The mixture was stirred at room temperature for 45 min, before addition of ammonium hydroxide solution (80%, 5 ml). The reaction mixture was stirred for 45 min and ice-cold water (15 ml) was added. The precipitate was collected by filtration and dried to give the title compound 0.35 g (88%) of UB-263 as a white solid. *Rf* = 0.44 (n-Heptane:40%EtOAc); ¹H NMR (400 MHz, Methanol-*d*₄) δ 8.41 (s, 1H), 8.18 (s, 1H), 8.11 (s, 1H); ¹³C NMR (101 MHz, Methanol-*d*₄) δ 166.85, 142.88, 140.78, 136.39, 130.23, 116.17, 113.99, 93.36.

### Example 78

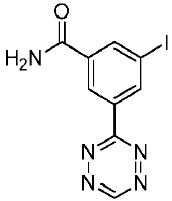

**3-lodo-5-(1,2,4,5-tetrazin-3-yl)benzamide (UB-265).** The final compound was obtained from 3-cyano-5-iodobenzamide (0.21 g, 0.77 mmol) following *General Procedure C*. The crude was purified using flash chromatography (98/2 CH₂Cl₂/MeOH) to yield 0.07 g (28%) of UB-265 as a pink solid. *Rf* = 0.41 (n-Heptane:60%EtOAC); ¹H NMR (400 MHz, DMSO-*d*₆) δ 10.68 (s, 1H), 8.99 (d, *J* = 1.7 Hz, 1H), 8.89 (d, *J* = 1.7 Hz, 1H), 8.55 (d, *J* = 1.7 Hz, 1H), 8.34 (s, 1H), 7.66 (s, 1H); ¹³C NMR (101 MHz, DMSO-*d*₆) δ 165.96, 164.64, 158.84, 140.13, 138.75, 137.55, 134.60, 126.77, 95.96.

### Example 79

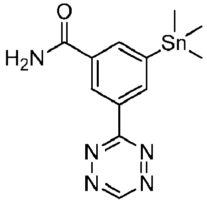

**3-(1,2,4,5-tetrazin-3-yl)-5-(trimethylstannyl)benzamide (UB-272).** The final compound was obtained from 55 mg (0.17 mmol) of the starting material, following the *General Procedure D.2.* The crude was purified using flash chromatography (40/60 n-Heptane/EtOAc) to yield 0.025 g (41%) of UB-220 as a purple oil. *Rf* = 0.42 (n-Heptane:60%EtOAc); ¹H NMR (600 MHz, Methanol-*d*₄) δ 10.28 (s, 1H), 8.76 (dd, *J* = 1.8, 0.9 Hz, 1H), 8.20 (dd, *J* = 1.9, 0.8 Hz, 1H), 0.31 (s, 9H); ¹³C NMR (151 MHz, Methanol-*d*₄) δ 170.47, 166.26, 158.03, 144.81, 138.52, 137.69, 134.02, 131.68, 126.83, -11.10; HPLC-MS [M+H]⁺ m/z calc. for [C₁₂H₁₆N₅SnO]⁺: 366.04; Found: 366.08.

### Example 80 of UB-70:

The radiolabelling of UB-70 was provided as described in Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 81

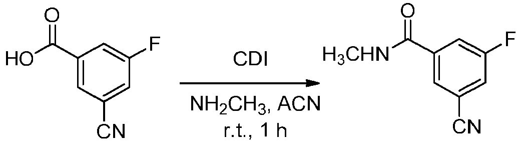

**5-Cyano-3-fluoro-N-methylbenzamide (UB-199).** To a solution of 5-cyano-3-fluorobenzoic acid (0.99 g, 6.0 mmol) in acetonitrile (20 ml) was added 1,1'-carbonyldiimidazole (1.46 g, 9.0 mmol). The mixture was stirred at room temperature for 45 min, before addition of aqueous methylamine solution (40%, 20 ml). The reaction mixture was stirred for 45 min and ice-cold water (15 ml) was added. The precipitate was collected by filtration and dried to give the title compound 0.81 g (76%) of UB199 as a white solid. *Rf* = 0.32 (n-Heptane:60%EtOAc); ¹H NMR (400 MHz, DMSO-*d*₆) δ 8.72 (d, *J* = 5.8 Hz, 1H), 8.11 (t, *J* = 1.5 Hz, 1H), 8.05 (ddd, *J* = 8.1, 2.7, 1.4 Hz, 1H), 7.97 (ddd, *J* = 9.5, 2.7, 1.5 Hz, 1H), 2.81 (d, *J* = 4.6 Hz, 3H); ¹³C NMR (101 MHz, DMSO-*d*₆) δ 163.96 (d, *J* = 2.5 Hz), 162.02 (d, *J* = 247.5 Hz), 138.49 (d, *J* = 7.4 Hz), 127.76 (d, *J* = 3.3 Hz), 122.17 (d, *J* = 25.6 Hz), 119.85 (d, *J* = 23.1 Hz), 117.66 (d, *J* = 3.4 Hz), 113.55 (d, *J* = 10.0 Hz), 26.83.

### Example 82

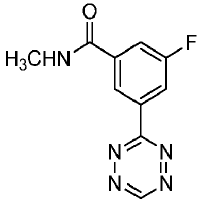

**3-Fluoro-N-methyl-5-(1,2,4,5-tetrazin-3-yl)benzamide (UB-200).** The final compound was obtained from 5-cyano-3-fluoro-N-methylbenzamide (0.62 g, 3.48 mmol) following *General Procedure C*. The crude was purified using flash chromatography (98/2 CH₂Cl₂/MeOH) to afford 0.28 g (34%) of UB-200 as a pink solid. *Rf* = 0.31 (n-Heptane:60%EtOAC); ¹H NMR (400 MHz, DMSO-*d*₆) δ 10.70 (s, 1H), 8.87 (s, 2H), 8.39 (dd, *J* = 9.3, 1.9 Hz, 1H), 7.99 (dt, *J* = 9.5, 2.0 Hz, 1H), 2.85 (d, *J* = 4.5 Hz, 3H); ¹³C NMR (101 MHz, DMSO-*d*₆) δ 164.88 (d, *J* = 11.1 Hz), 162.89 (d, *J* = 245.8 Hz), 158.92, 138.41 (d, *J* = 7.5 Hz), 135.00 (d, *J* = 8.2 Hz), 133.13, 123.08, 118.56 (d, *J* = 22.8 Hz), 117.16 (d, *J* = 24.0 Hz), 26.89; HPLC-MS [M+H]⁺ m/z calc. for [C₁₀H₉FN₅O]⁺: 234.08; found 234.06.

### Example 83

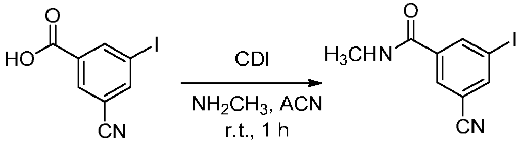

**5-Cyano-3-iodo-N-methylbenzamide (UB-262).** To a solution of 5-cyano-3-iodobenzoic acid (0.40 g, 1.46 mmol) in acetonitrile (10 ml) was added 1,1'-carbonyldiimidazole (0.36 g, 2.20 mmol). The mixture was stirred at room temperature for 45 min, before addition of aqueous Methylamine solution (80%, 5 ml). The reaction mixture was stirred for 45 min and ice-cold water (15 ml) was added. The precipitate was collected by filtration and dried to give the title compound 0.41 g (98%) of UB-262 as a white solid. *Rf* = 0.55 (n-Heptane:40%EtOAc); ¹H NMR (400 MHz, Methanol-*d*₄) δ 8.45 (d, *J* = 1.7 Hz, 1H), 8.27 (d, *J* = 1.9 Hz, 1H), 8.15 (s, 1H), 2.93 (s, 3H); ¹³C NMR (101 MHz, Methanol*-d*₄) δ 165.38, 142.63, 140.38, 136.78, 129.83, 116.20, 113.97, 93.44, 25.67.

### Example 84

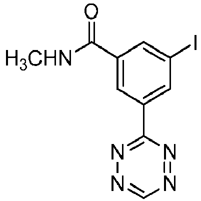

**3-lodo-N-methyl-5-(1,2,4,5-tetrazin-3-yl)benzamide (UB-264).** The final compound was obtained from 5-cyano-3-iodo-N-methylbenzamide (0.38 g, 1.32 mmol) following *General Procedure C*. The crude was purified using flash chromatography (98/2 CH₂Cl₂/MeOH) to yield 0.11 g (24%) of UB254 as a pink solid. *Rf* = 0.45 (n-Heptane:60%EtOAC); ¹H NMR (600 MHz, DMSO-*d*₆) δ 10.68 (s, 1H), 8.97 (s, 1H), 8.89 (s, 1H), 8.84 (q, *J* = 4.5 Hz, 1H), 8.51 (s, 1H), 2.83 (d, *J* = 4.5 Hz, 3H); ¹³C NMR (151 MHz, DMSO-*d*₆) δ 164.67, 164.62 158.89, 139.78, 138.57, 137.63, 134.64, 126.31, 96.04, 26.89.

### Example 85

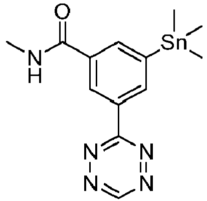

**N-methyl-3-(1,2,4,5-tetrazin-3-yl)-5-(trimethylstannyl)benzamide (UB-269).** The final compound was obtained from 50 mg (0.14 mmol) of the starting material, following the *General Procedure D.2.* The crude was purified using flash chromatography (60/40 n-Heptane/EtOAc) to yield 0.035 g (63%) of UB269 as a purple oil. *Rf* = 0.46 (n-Heptane:60%EtOAc); ¹H NMR (400 MHz, Chloroform-d) δ 10.25 (s, 1H), 8.89 - 8.80 (m, 2H), 8.26 (dd, *J* = 1.9, 0.9 Hz, 1H), 6.37 (s, 1H), 3.08 (d, *J* = 4.8 Hz, 3H), 0.39 (s, 9H); ¹³C NMR (101 MHz, Chloroform-d) δ 167.68, 166.22, 157.96, 145.55, 139.44, 138.02, 134.89, 130.88, 125.60, 26.97, -9.23; HPLC-MS [M+H]⁺ m/z calc. for [C₁₃H₁₈N₅SnO]⁺: 380.05; Found: 380.02.

### Example 86 of UB-200:

The radiolabelling of UB-007 was provided as described in the Example 128, 129 and 130 and as shown in Table 2. In Table 2, the following letters in brackets defines the following:
[a] Stannate precursor could not be synthesized.
[b] No tetrazine formation detected.
[c] Decomposition during Cu-mediated ¹⁸F-fluorination reaction.
[d] RCCs were determined by radio-HPLC (n = 3).

### Example 87

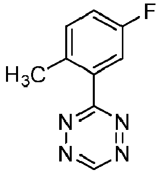

**3-(3-fluoro-6-methylphenyl)-1,2,4,5-tetrazine (RGV-117).** The final compound was obtained from 5-fluoro-2-methylbenzonitrile (0.54 g, 4.00 mmol) following *General Procedure C*. The crude was purified using flash chromatography (95/5 n-Heptane/EtOAc) to yield after crystallization with n-Heptane 0.12 g (16%) of a red solid. *Rf* = 0.37 (n-Heptane:10%EtOAc); ¹H NMR (600 MHz, Chloroform-d) δ 10.23 (s, 1H), 7.72 (dd, *J* = 8.5, 3.2 Hz, 1H), 7.32 - 7.27 (m, 1H), 7.08 (dd, *J* = 9.1, 4.2 Hz, 1H), 3.90 (s, 3H); ¹³C NMR (151 MHz, Chloroform-d) δ 167.77 (d, *J* = 2.2 Hz), 157.08 (d, *J* = 240.5 Hz), 156.99, 154.97 (d, *J* = 2.1 Hz), 122.91 (d, *J* = 7.8 Hz), 120.08 (d, *J* = 22.9 Hz), 118.48 (d, *J* = 25.3 Hz), 113.94 (d, *J* = 7.9 Hz), 56.96.; HPLC-MS [M+H]⁺ m/z calc. for [C₉H₇FN₄]⁺: 191.07; Found 191.09.

### Example 88

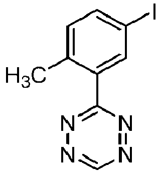

**3-(5-iodo-2-methylphenyl)-1,2,4,5-tetrazine (RGV_125).** The final compound was obtained from 3-iodo-5-methylbenzonitrile (0.97 g, 4.00 mmol) following *General Procedure C*. The crude was purified using flash chromatography (90/10 n-Heptane/EtOAc) to yield after crystallization with n-Heptane 0.21 g (17%) of a red solid. *Rf* = 0.42 (n n-Heptane:10%EtOAc).; ¹H NMR (600 MHz, Chloroform-d) δ 10.26 (s, 1H), 8.43 (s, 1H), 7.81 (d, J = 8.1 Hz, 1H), 7.15 (d, J = 8.1 Hz, 1H), 2.59 (s, 3H); ¹³C NMR (151 MHz, Chloroform-d) δ 168.60, 157.06, 140.71, 139.61, 138.56, 133.95, 133.52, 90.88, 21.28.

### Example 89

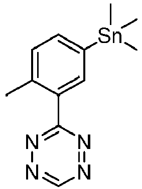

**3-(2-methyl-5-(trimethylstannyl)phenyl)-1,2,4,5-tetrazine (RGV_139).** The final compound was obtained from 50 mg (0.17 mmol) of the starting material, following the *General Procedure D.2.* The crude was purified using flash chromatography (90/10 n-Heptane/EtOAc) to yield 0.10 g (18%) of a pink solid. *Rf* = 0.32 (n-Heptane:20%EtOAc); ¹H NMR (600 MHz, Chloroform-d) δ 10.23 (s, 1H), 8.15 (s, 1H), 7.63 (d, *J* = 7.4 Hz, 1H), 7.38 (d, *J* = 7.4 Hz, 1H), 2.62 (s, 3H), 0.33 (s, 9H); ¹³C NMR (151 MHz, Chloroform-*d*) δ 170.16, 156.91, 140.46, 139.45, 138.73, 138.49, 131.61, 131.48, 21.39, -9.27.; HPLC-MS [M+H]⁺ m/z calc. for [C₁₂H₁₆N₄Sn]⁺: 337.05; Found: 337.1.
Chloroform-*d*) δ 166.64, 157.77, 145.64, 132.10, 127.94, 127.56, 111.71, 55.45, -9.36; HPLC-MS [M+H]⁺ m/z calc. for [C₁₂H₁₇N₄SnO]⁺: 353.04; Found: 353.07.

### Example 90

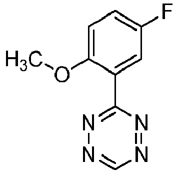

**3-(3-fluoro-6-methoxyphenyl)-1,2,4,5-tetrazine (RGV-116).** The final compound was obtained from 5-fluoro-2-methoxylbenzonitrile (0.60 g, 4.00 mmol) following *General Procedure C*. The crude was purified using flash chromatography (95/5 n-Heptane/EtOAc) to yield after crystallization with n-Heptane 0.13 g (16%) as a red solid. *Rf* = 0.18 (n-Heptane:10%EtOAc); ¹H NMR (600 MHz, Chloroform-*d*) δ 10.23 (s, 1H), 8.26 (s, 1H), 8.12 (d, *J* = 9.4 Hz, 1H), 7.17 (d, *J* = 9.1 Hz, 1H), 2.50 (s, 3H); ¹³C NMR (151 MHz, Chloroform-*d*) δ 165.99 (d, *J* = 3.4 Hz), 163.45 (d, *J* = 246.8 Hz), 158.11, 141.97 (d, *J* = 7.8 Hz), 133.45 (d, *J* = 8.9 Hz), 124.81, 120.95 (d, *J* = 21.2 Hz), 112.44 (d, *J* = 24.2 Hz), 21.60; HPLC-MS [M+H]⁺ m/z calc. for [C₉H₇FN₄O]⁺: 207.07; Found: 207.9.

### Example 91

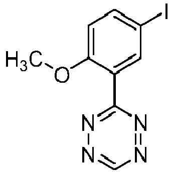

**3-(5-iodo-2-methoxyphenyl)-1,2,4,5-tetrazine (RGV_105).** The final compound was obtained from 3-iodo-5-methoxybenzonitrile (1.03 g, 4.00 mmol) following *General Procedure C*. The crude was purified using flash chromatography (95/5 n-Heptane/EtOAc) to yield after crystallization with n-Heptane 0.19 g (15%) as a red solid. *Rf* = 0.21 (n-Heptane:20%EtOAc); ¹H NMR (600 MHz, Chloroform-d) δ 10.21 (s, 1H), 8.21 (s, 1H), 7.82 (dd, *J* = 8.8, 2.3 Hz, 1H), 6.88 (d, *J* = 8.8 Hz, 1H), 3.87 (s, 3H); ¹³C NMR (151 MHz, CDCl₃) δ 167.31, 158.39, 156.94, 142.10, 140.14, 124.08, 114.62, 82.71, 56.35.

### Example 92

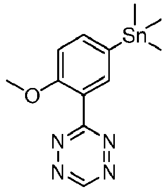

**3-(2-methoxy-5-(trimethylstannyl)phenyl)-1,2,4,5-tetrazine (RGV_110).** The final compound was obtained from 50 mg (0.17 mmol) of the starting material, following the *General Procedure D.1.* The crude was purified using flash chromatography (90/10 n-Heptane/EtOAc) to yield 0.23 g (40%) of a pink solid. *Rf* = 0.16 (n-Heptane:10%EtOAc); ¹H NMR (600 MHz, Chloroform-*d*) δ 10.21 (s, 1H), 8.11 - 7.92 (m, 1H), 7.75 - 7.58 (m, 1H), 7.19 - 7.05 (m, 1H), 3.90 (s, 3H), 0.32 (s, 9H); ¹³C NMR (151 MHz, CDCl₃) δ 168.91, 158.89, 156.86, 141.13, 139.19, 133.70, 122.09, 112.20, 56.11, -9.19; HPLC-MS [M+H]⁺ m/z calc. for [C₁₂H₁₆N₄OSn]⁺: 353.04; Found: 353.1.

### Example 93

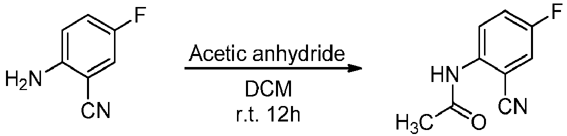

**N-(2-cyano-4-fluorophenyl)acetamide (UB-154).** To a solution of the 2-amino-5-fluorobenzonitrile (0.82 g, 6.00 mmol) in DCM (30.0 mL) was added acetic anhydride (0.80 mL, 8.40 mmol). The mixture was stirred at room temperature for 12 hours. The suspension was filtered, and the solvent removed under vacuum. Purification by flash chromatography (70/30 Heptane/EtOAc) afforded 0.81 g (76%) of UB-154 as a white solid. *Rf* = 0.41 (n-Heptane:40%EtOAc); ¹H NMR (400 MHz, DMSO-*d*₆) δ 10.16 (s, 1H), 7.81 (dd, *J*= 8.4, 1.8 Hz, 1H), 7.59 (d, *J* = 1.7 Hz, 1H), 7.57 (d, *J* = 1.7 Hz, 1H), 2.09 (s, 3H); ¹³C NMR (101 MHz, DMSO-*d*₆) δ 169.22, 158.86 (d, *J* = 244.3 Hz), 137.55 (d, *J* = 3.0 Hz), 128.41 (d, *J* = 8.7 Hz), 121.74 (d, *J* = 22.4 Hz), 120.01 (d, *J* = 26.0 Hz), 116.21 (d, *J* = 2.7 Hz), 109.34, 23.49.
164.47, 164.13, 149.66, 136.88, 132.80, 132.00, 131.62, 129.46, 128.11, 127.08, -9.32.; HPLC-MS [M+H]⁺ m/z calc. for [C₁₂H₁₆N₄OSn]⁺: 353.04; Found: 353.1.

### Example 94

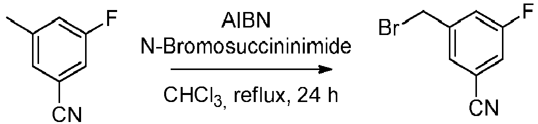

**3-(Bromomethyl)-5-fluorobenzonitrile (UB-98).** To a solution of 3-fluoro-5-methylbenzonitrile (2.61 g, 19.24 mmol) and N-bromosuccinmide (5.13 g, 28.86 mmol) in CHCl₃ was added AIBN (1.26 g, 7.69 mmol). The reaction was refluxed for 24 h. The solvent was removed under vacuum and the crude purified by flash chromatography (heptane/EtOAc 95/5) to give 2.10 g (51%) of UMB-98 as a colorless oil. *Rf* = 0.32 (n-Heptane:5%EtOAc); ¹H NMR (400 MHz, Chloroform-d) δ 7.50 (t, *J* = 1.5 Hz, 1H), 7.38 (dt, *J* = 8.9, 2.0 Hz, 1H), 7.30 (ddd, *J* = 7.9, 2.5, 1.3 Hz, 1H), 4.46 (s, 2H); ¹³C NMR (101 MHz, Chloroform-d) δ 162.12 (d, *J* = 251.3 Hz), 141.93 (d, *J* = 8.0 Hz), 128.52 (d, *J* = 3.4 Hz), 121.06 (d, *J* = 22.0 Hz), 118.97 (d, *J* = 24.7 Hz), 117.07 (d, *J* = 3.3 Hz), 114.26 (d, *J* = 9.9 Hz), 30.32 (d, *J* = 1.9 Hz).

### Example 95

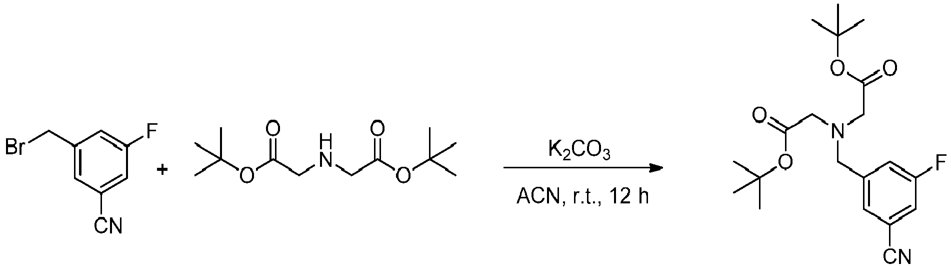

**Di-tert-butyl 2,2'-((3-cyano-5-fluorobenzyl)azanediyl)diacetate (UB-100).** To a solution of 3-fluoro-5-bromomethylbenzonitrile (1.09 g, 5.10 mmol) in CH₃CN (30 mL) was added K₂CO₃ (1.06 g, 7.65 mmol) and di-tert-butyl iminodiacetate (1.50 g, 6.12 mmol). The reaction mixture was stirred at room temperature overnight. The solvent was removed in vacuo, and the resulting mixture was diluted with water (20 mL), extracted with EtOAc (2 x 25 mL), washed with brine (30 mL), dried over MgSO₄, filtered and concentrated under reduced pressure. Purification by flash chromatography (90/10 Heptane/EtOAc) afforded 1.72 g (89%) of UB-100 as a white solid. *Rf* = 0.24 (n-Heptane:10%EtOAc); ¹H NMR (400 MHz, Chloroform-*d*) δ 7.53 (s, 1H), 7.51 - 7.42 (m, 1H), 7.23 (ddd, *J* = 7.8, 2.5, 1.4 Hz, 1H), 3.93 (s, 2H), 3.39 (s, 4H), 1.46 (s, 18H);

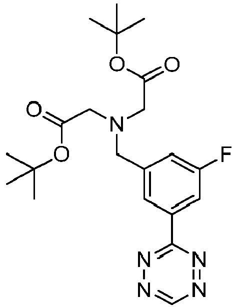

### Example 96

**Di-tert-butyl 2,2'-((3-fluoro-5-(1,2,4,5-tetrazin-3-yl)benzyl)azanediyl)diacetate (UB-104).** The compound was obtained from di-tert-butyl 2,2'-((3-cyano-5-fluorobenzyl)azanediyl)diacetate (1.70 g, 4.49 mmol) following *General Procedure C*. The crude was purified using flash chromatography (heptane/EtOAc 95/5) to yield 0.11 g (24%) of UB-104 as a red solid. *Rf* = 0.39 (n-Heptane:20%EtOAC); ¹H NMR (400 MHz, Chloroform-*d*) δ 10.17 (s, 1H), 8.34 (d, *J* = 1.4 Hz, 1H), 8.14 (ddd, *J* = 9.2, 2.5, 1.5 Hz, 1H), 7.55 - 7.45 (m, 1H), 3.98 (s, 2H), 3.40 (s, 4H), 1.41 (s, 18H); ¹³C NMR (101 MHz, Chloroform-d) δ 170.25, 165.78 (d, *J* = 3.2 Hz), 163.58 (d, *J* = 247.5 Hz), 157.97, 143.30 (d, *J* = 7.1 Hz), 133.46 (d, *J* = 8.7 Hz), 124.18 (d, *J* = 2.7 Hz), 120.64 (d, *J* = 21.8 Hz), 114.15 (d, *J* = 24.5 Hz), 81.34, 56.96 (d, *J* = 1.8 Hz), 55.35, 28.17.

### Example 97

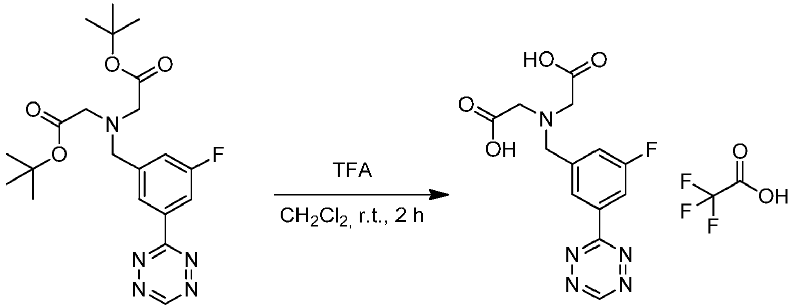

**1-Carboxy-N-(carboxymethyl)-N-(3-fluoro-5-(1,2,4,5-tetrazin-3-yl)benzyl)methanaminium 2,2,2-trifluoroacetate (UB-108).** To a solution of di-tert-butyl 2,2'-((3-fluoro-5-(1,2,4,5-tetrazin-3-yl)benzyl)azanediyl)diacetate (0.15 g, 0.36 mmol) in DCM (5 mL) was added trifluoroacetic acid (5 mL). The reaction was stirred at room temperature for 2 hours. The solvent was then removed under reduced pressure to obtain a pik solid. NMR of the crude shows full conversion. Purification by preparative HPLC afforded 0.08 g (51%) of UMB-108 as a pink solid. ¹H NMR (400 MHz, Methanol-*d*₄) δ 10.42 (s, 1H), 8.60 (d, *J* = 1.4 Hz, 1H), 8.41 - 8.32 (m, 1H), 7.73 - 7.64 (m, 1H), 5.11 (s, 7H), 4.59 (s, 2H), 4.11 (s, 4H); ¹³C NMR (101 MHz, Methanol-*d*₄) δ 168.68, 165.12 (d, *J* = 3.2 Hz), 163.27 (d, *J* = 247.6 Hz), 158.24, 135.40 (d, *J* = 7.7 Hz), 135.10 (d, *J* = 8.6 Hz), 126.05 (d, *J* = 3.0 Hz), 121.71 (d, *J* = 22.7 Hz), 115.46 (d, *J* = 24.4 Hz), 57.85, 53.62; HPLC-MS [M+H]⁺ m/z calc. for [C₁₃H₁₃FN₅O₄]⁺: 322.09; Found: 323.11.

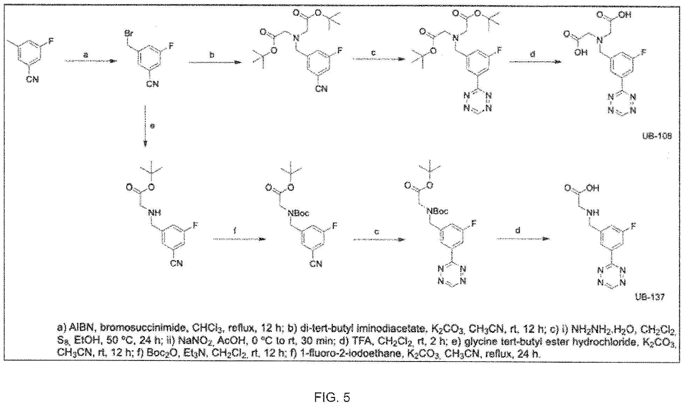

Figure 5 displays the whole synthesis scheme. Figure 7 displays the NMR chromatogram.

### Example 98

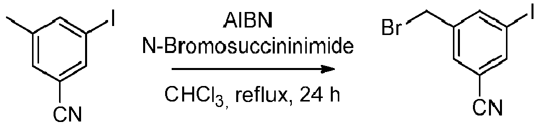

**3-(Bromomethyl)-5-iodobenzonitrile (UB-142).** To a solution of 3-iodo-5-methylbenzonitrile (2.50 g, 10.28 mmol) and N-bromosuccinimide (2.28 g, 12.86 mmol) in CHCl₃ (40 mL) was added AIBN (0.67 g, 4.11 mmol). The reaction was refluxed for 24 h. The solvent was removed under vacuum and the crude purified by flash chromatography (heptane/EtOAc 95/5) to give 1.61 g (49%) of UMB-142 as a white solid. *Rf* = 0.28 (n-Heptane:5%EtOAc); ¹H NMR (400 MHz, Chloroform-d) δ 7.96 (d, *J* = 1.6 Hz, 1H), 7.89 (d, *J* = 1.6 Hz, 1H), 7.64 (t, *J* = 1.6 Hz, 1H), 4.38 (s, 2H) ¹³C NMR (101 MHz, Chloroform-d) δ 142.20, 140.90, 140.12, 131.67, 116.55, 114.56, 94.05, 29.90.

### Example 99

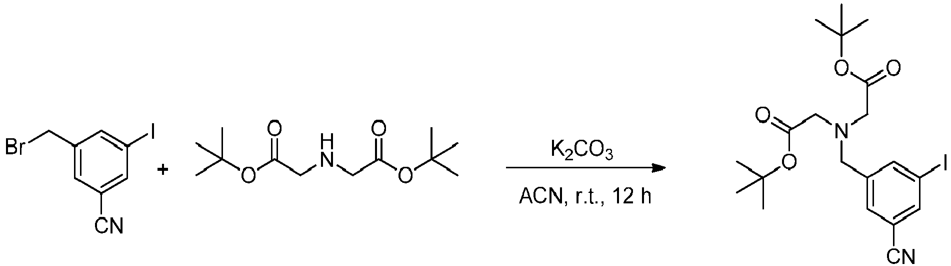

**Di-tert-butyl 2,2'-((3-cyano-5-iodobenzyl)azanediyl)diacetate (UB-152).** To a solution of 3-(bromomethyl)-5-iodobenzonitrile (1.00 g, 3.10 mmol) in CH₃CN (30 mL) was added K₂CO₃ (0.64 g, 7.65 mmol) and the corresponding amine (0.91 g, 3.72 mmol). The reaction mixture was stirred at room temperature overnight. The solvent was removed in vacuo, and the resulting mixture was diluted with water (20 mL), extracted with EtOAc (2 x 25 mL), washed with brine (30 mL), dried over MgSO₄, filtered and concentrated in vacuo. Purification by flash chromatography (90/10 Heptane/EtOAc) afforded 1.35 g (89%) of UB-152 as a colorless oil. *Rf* = 0.31 (n-Heptane:5%EtOAc); ¹H NMR (400 MHz, Chloroform-*d*) δ 7.96 (s, 1H), 7.80 (s, 1H), 7.65 (s, 1H), 3.83 (s, 2H), 3.33 (s, 4H), 1.40 (s, 18H); ¹³C NMR (101 MHz, Chloroform-d) δ 169.95, 142.47, 142.14, 139.29, 131.57, 117.13, 114.11, 93.93, 81.52, 56.16, 55.23, 28.17.

### Example 100

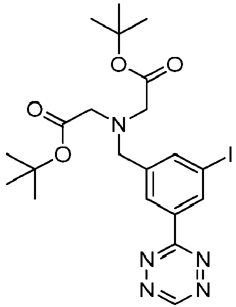

**Di-tert-butyl 2,2'-((3-iodo-5-(1,2,4,5-tetrazin-3-yl)benzyl)azanediyl)diacetate (UB-156).** The compound was obtained from di-tert-butyl 2,2'-((3-cyano-5-iodobenzyl)azanediyl)diacetate (1.30 g, 2.67 mmol) following *General Procedure C*. The crude was purified using flash chromatography (heptane/EtOAc 95/5) to 0.37 g (26%) of UB-156 as red oil. *Rf* = 0.39 (n-Heptane:20%EtOAC); ¹H NMR (600 MHz, Chloroform-*d*) δ 10.22 (s, 1H), 8.84 (s, 1H), 8.55 (s, 1H), 8.10 (s, 1H), 3.99 (s, 2H), 3.44 (s, 4H), 1.46 (s, 18H); ¹³C NMR (151 MHz, Chloroform-d) δ 170.11, 165.31, 157.97, 142.35, 136.05, 133.37, 127.89, 95.12, 81.35, 56.66, 55.28, 28.19.

### Example 101

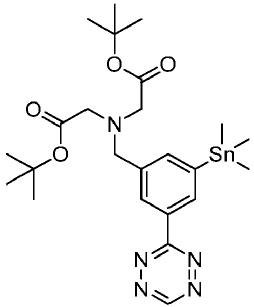

**Di-tert-butyl 2,2'-((3-(1,2,4,5-tetrazin-3-yl)-5-(trimethylstannyl)benzyl)azanediyl)diacetate (UB-161).** The final compound was obtained from 50 mg (0.09 mmol) of the starting material, following the *General Procedure D.2.* The crude was purified using flash chromatography (95/5 n-Heptane/EtOAc) to yield 0.025 g (47%) of UB-161 as a purple oil. *Rf* = 0.37 (n-Heptane:20%EtOAc); ¹H NMR (400 MHz, Chloroform-d) δ 10.20 (s, 1H), 8.63 (d, *J* = 1.8 Hz, 1H), 8.55 (s, 1H), 7.97 - 7.73 (m, 1H), 4.08 (s, 2H), 3.50 (s, 4H), 1.47 (s, 18H), 0.37 (s, 9H); ¹³C NMR (101 MHz, Chloroform-d) δ 170.12, 166.84, 157.74, 144.34, 141.42, 134.63, 131.05, 129.00, 81.28, 57.37, 55.09, 28.19, -9.27; HPLC-MS [M+H]+ m/z calc. for [C₂₄H₃₈N₅SnO₄]⁺: 580.19; Found: 580.22.
The synthesis of UB-108 and UB-137 using UB-161 is shown in Figure 6. Figure 7a displays the NMR chromatogram.

### Example 102 of UB-108:

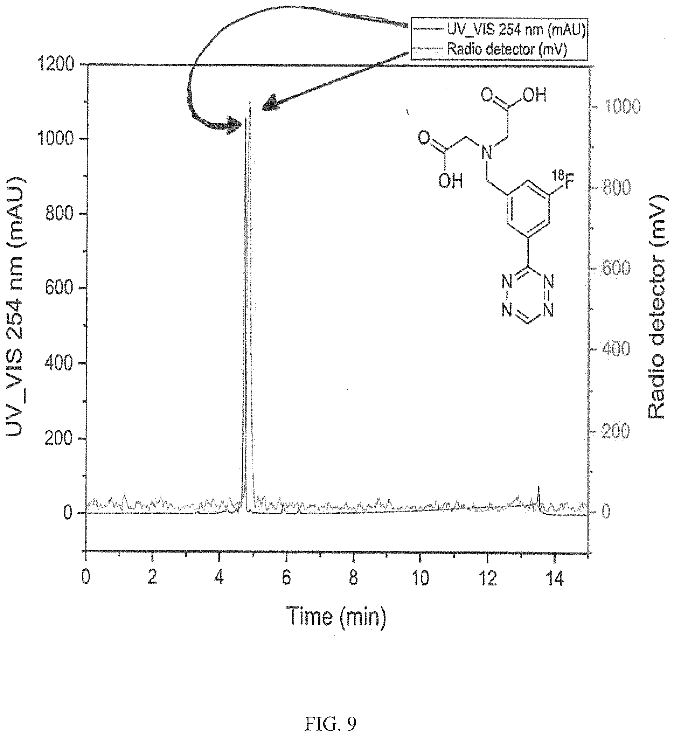

The radiolabelling of UB-108 was provided as described Example 128, 129, 130, 131 and 132 and as shown in Figure 1 and the final structure is also shown in Figure 4. The HPLC test of UB108 was performed as described in Example 135, and the HPLC chromatogram is shown in Figure 9.

### Example 103

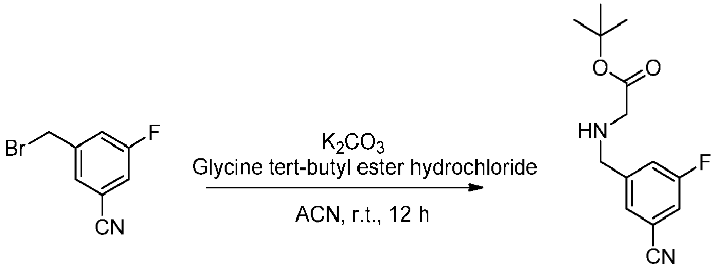

**Tert-butyl 2-((3-cyano-5-fluorobenzyl)amino)acetate (UB-127).** To a solution of 3-(bromomethyl)-5-fluorobenzonitrile (3.34 g, 15.60 mmol) in CH₃CN (40 mL) was added K₂CO₃ (10.78 g, 78.02 mmol) and glycine tert-butyl ester hydrochloride (7.85 g, 46.81 mmol). The reaction mixture was stirred at room temperature overnight. The solvent was removed in vacuo, and the resulting mixture was diluted with water (20 mL), extracted with EtOAc (2 x 25 mL), washed with brine (30 mL), dried over MgSO₄, filtered and concentrated under reduced pressure. Purification by flash chromatography afforded 3.52 g (85%) of UB-127 as a colorless oil. *Rf* = 0.23 (n-Heptane:20%EtOAc); ¹H NMR (400 MHz, Chloroform-d) δ 7.46 (d, *J* = 1.5 Hz, 1H), 7.35 (dt, *J* = 9.3, 1.8 Hz, 1H), 7.25 - 7.18 (m, 1H), 3.83 (s, 2H), 3.28 (s, 2H), 1.92 (s, 1H), 1.47 (s, 9H); ¹³C NMR (101 MHz, Chloroform-d) δ 171.35, 162.36 (d, *J* = 250.1 Hz), 144.68 (d, *J* = 7.4 Hz), 127.53 (d, *J*= 3.2 Hz), 119.97 (d, *J* = 21.3 Hz), 117.66 (d, *J* = 24.8 Hz), 117.63 (d, *J* = 3.3 Hz), 113.66 (d, *J* = 9.7 Hz), 81.60, 51.96 (d, *J* = 1.8 Hz), 50.82, 28.12.

### Example 104

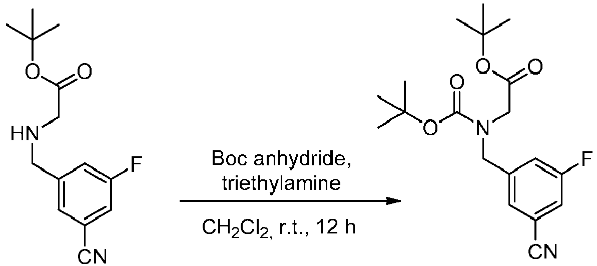

**Tert-butyl** 2-((tert-butoxycarbonyl)(3-cyano-5-fluorobenzyl)amino)acetate (UB-128). To a solution of tert-butyl 2-((3-cyano-5-fluorobenzyl)amino)acetate (1.5 g, 5.67 mmol) and EtsN (1.90 mL, 13.62 mmol) in DCM (40 mL) was added BoC₂O (1.48 g, 6.81 mmol). The reaction was stirred at room temperature for 12 h. The solution was then washed with water (50 mL) and K₂CO₃ saturated solution (50 mL), dried over anhydrous Na₂SO₄ filtered and concentrated under reduced pressure to afford 2.1 g of the crude. Purification by flash chromatography (90/10 Heptane/EtOAc) afforded 1.84 g (89%) of UB-128 as a colorless oil (60/40 unassigned rotamers mixture). *Rf* = 0.48 (n-Heptane:20%EtOAc); ¹H NMR (400 MHz, Chloroform-*d*) δ 7.42 - 7.34 (m, 1H), 7.33 - 7.25 (m, 2H), 4.54 (s, 1.2H), 4.49 (s, 0.8H), 3.89 (s, 0.8H), 3.74 (s, 1.2H), 1.67 - 1.35 (m, 18H); ¹³C NMR (101 MHz, Chloroform-d) δ 168.51, 168.43, 162.39 (d, *J* = 250.6 Hz), 155.62, 155.21, 143.03 (d, *J* = 7.3 Hz), 142.68 (d, *J* = 7.3 Hz), 127.11 (d, *J* = 3.2 Hz), 126.85 - 126.63 (m), 119.78 (d, *J* = 21.6 Hz), 119.25 (d, *J* = 21.5 Hz), 117.99 (d, *J* = 24.7 Hz), 117.92 (d, *J* = *24.8* Hz), 117.52 - 117.42 (m), 113.90 (d, *J* = 9.8 Hz), 81.63 (d, *J* = 78.3 Hz), 81.51 (d, *J* = 81.3 Hz), 51.17, 50.90, 49.88, 49.52, 31.87, 29.00, 28.21, 28.03, 22.67, 14.09.

### Example 105

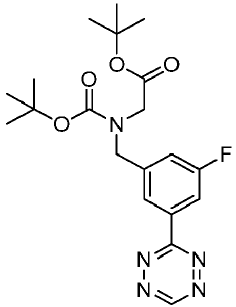

**Tert-butyl 2-((tert-butoxycarbonyl)(3-fluoro-5-(1,2,4,5-tetrazin-3-yl)benzyl)amino)acetate (UB-133).** The compound was obtained from tert-butyl 2-((tert-butoxycarbonyl)(3-cyano-5-fluorobenzyl)amino)acetate (1.56 g, 4.28 mmo) following *General Procedure C*. The crude was purified using flash chromatography (heptane/EtOAc 95/5) to yield 0.45 g (25%) of UB-133 as red oil (60/40 unassigned rotamers mixture). *Rf* = 0.41 (n-Heptane:20%EtOAC); ¹H NMR (400 MHz, Chloroform-d) δ 10.27 (s, 1H), 8.37 - 8.30 (m, 1H), 8.27 - 8.20 (m, 1H), 7.40 - 7.28 (m, 1H), 4.67 (s, 1.2H), 4.60 (s, 0.8H), 3.93 (s, 0.8H), 3.79 (s, 1.2H), 1.53 - 1.41 (m, 18H); ¹³C NMR (101 MHz, Chloroform-d) δ 168.64, 165.56, 163.56 (d, *J* = 248.0 Hz), 163.49 (d, *J* = 248.1 Hz), 158.02, 155.73, 155.42, 142.44 (d, *J* = 6.3 Hz), 142.19 (d, *J* = 6.9 Hz), 133.73 (d, *J* = 8.9 Hz), 123.05, 122.88, 119.57 (d, *J* = 22.0 Hz), 119.08 (d, *J* = 21.7 Hz), 81.43 (d, *J* = 82.1 Hz), 81.30 (d, *J* = 91.4 Hz), 51.41, 51.06, 49.60, 49.28, 28.30, 28.26, 28.04.

### Example 106

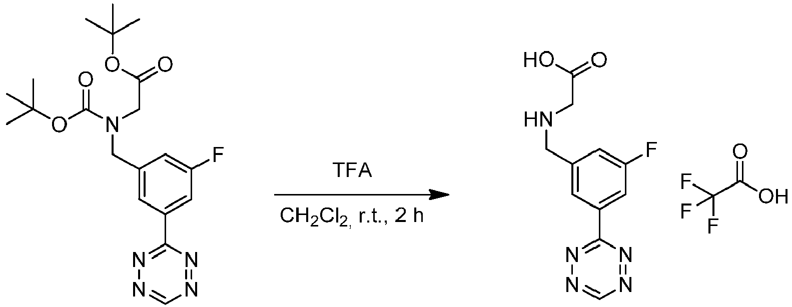

**1-Carboxy-N-(3-fluoro-5-(1,2,4,5-tetrazin-3-yl)benzyl)methanaminium 2,2,2-trifluoroacetate (UB-137).** To a solution of tert-butyl 2-((tert-butoxycarbonyl)(3-fluoro-5-(1,2,4,5-tetrazin-3-yl)benzyl)amino)acetate (0.30 g, 0.71 mmol) in DCM (5 mL) was added trifluoroacetic acid (5 mL). The reaction was stirred at room temperature for 2 hours. The solvent was then removed under reduced pressure to obtain a red solid. NMR of the crude shows full conversion. Purification by preparative HPLC afforded 0.11 g (41%) of UMB-137 as a pink oil. ¹H NMR (400 MHz, Methanol-*d*₄) δ 10.32 (s, 1H), 8.52 (s, 1H), 8.30 (ddd, *J* = 9.4, 2.5, 1.5 Hz, 1H), 7.85 - 7.25 (m, 1H), 4.36 (s, 2H), 3.93 (s, 2H); ¹³C NMR (101 MHz, Methanol-*d*₄) δ 167.31, 165.08 (d, *J* = 3.3 Hz), 163.31 (d, *J* = 247.8 Hz), 158.31, 135.47 (d, *J* = 8.8 Hz), 134.73 (d, *J* = 7.8 Hz), 125.19 (d, *J* = 3.1 Hz), 120.87 (d, *J* = 22.9 Hz), 115.54 (d, *J* = 24.3 Hz), 49.76 (d, *J* = 1.9 Hz), 46.57; HPLC-MS [M+H]⁺ m/z calc. for [C₁₁H₁₁FN₅O₂]⁺: 264.09; Found: 264.07.

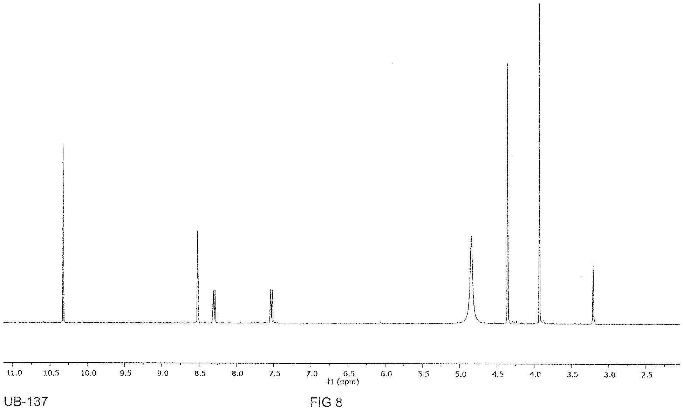

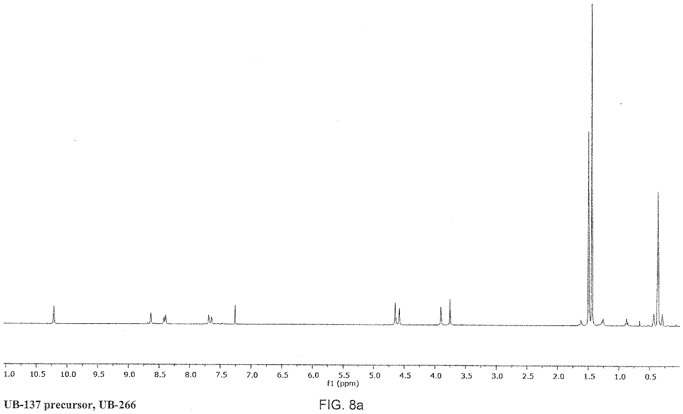

Figure 5 displays the whole synthesis scheme. Figure 8 displays the NMR chromatogram.

### Example 107

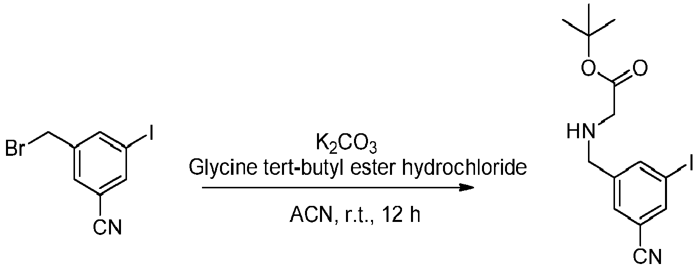

**Tert-butyl 2-((3-cyano-5-iodobenzyl)amino)acetate (UB-231).** To a solution of 3-(bromomethyl)-5-iodobenzonitrile (2.00 g, 6.21 mmol) in CH₃CN (40 mL) was added K₂CO₃ (4.29 g, 31.07 mmol) and glycine tert-butyl ester hydrochloride (3.12 g, 18.63 mmol). The reaction mixture was stirred at RT overnight. The solvent was removed in vacuo, and the resulting mixture was diluted with water (20 mL), extracted with EtOAc (2 x 25 mL), washed with brine (30 mL), dried over MgSO₄, filtered and concentrated in vacuo. Purification by flash chromatography afforded 1.91 g (83%) of UB-231 as a colorless oil. *Rf* = 0.23 (n-Heptane:20%EtOAC); ¹H NMR (400 MHz, Chloroform-d) δ 7.89 (s, 1H), 7.79 (s, 1H), 7.56 (s, 1H), 3.72 (s, 2H), 3.21 (s, 2H), 1.91 (s, 1H), 1.41 (s, 9H); ¹³C NMR (101 MHz, Chloroform-d) δ 171.24, 143.30, 141.56, 139.06, 130.89, 117.13, 114.11, 93.95, 81.68, 51.74, 50.76, 28.13.

### Example 108

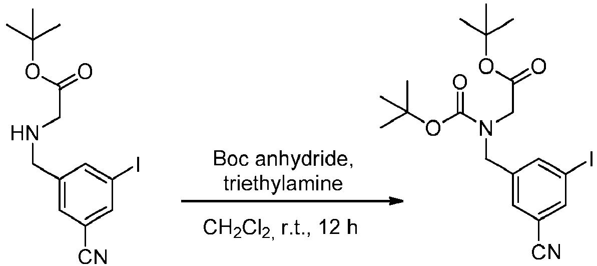

**Tert-butyl 2-((tert-butoxycarbonyl)(3-cyano-5-iodobenzyl)amino)acetate (UB-232).** To a solution of tert-butyl 2-((3-cyano-5-iodobenzyl)amino)acetate (1.9 g, 5.10 mmol) and EtsN (1.71 mL, 12.25 mmol) in DCM (40 mL) was added BoC₂O (1.41 g, 6.12 mmol). The reaction was stirred at room temperature for 12 hours. The solution was then washed with water (50 mL) and K₂CO₃ saturated solution (50 mL), dried over anhydrous Na₂SO₄, filtered and concentrated under reduced pressure to afford 2.5 g of the crude. Purification by flash chromatography (80/20 Heptane/EtOAc) afforded 2.2 g (91%) of UB-232 as a colorless oil (60/40 unassigned rotamers mixture). *Rf* = 0.38 (n-Heptane:20%EtOAc); ¹H NMR (400 MHz, Chloroform-*d*) δ 7.99 - 7.80 (m, 2H), 7.71 - 7.41 (m, 1H), 4.48 (s, 1.2H), 4.42 (s, 0.8H), 3.86 (s, 0.8H), 3.70 (s, 1.2H), 1.51 - 1.42 (m, 18H); ¹³C NMR (101 MHz, Chloroform-*d*) δ 168.43, 155.62, 142.14, 141.49, 141.13, 140.79, 139.30, 131.33, 130.50, 130.04, 117.00, 114.29, 94.03, 82.03, 81.93, 81.30, 81.13, 50.85, 50.56, 49.78, 49.53, 28.22, 28.05.

### Example 109

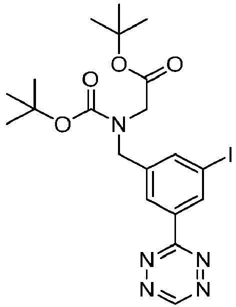

**Tert-butyl 2-((tert-butoxycarbonyl)(3-iodo-5-(1,2,4,5-tetrazin-3-yl)benzyl)amino)acetate (UB-235).** The compound was obtained from tert-butyl 2-((tert-butoxycarbonyl)(3-cyano-5-iodobenzyl)amino)acetate (2.10 g, 4.44 mmol) following *General Procedure C*. The crude was purified using flash chromatography (heptane/EtOAc 95/5) to yield 0.64 g (27%) of UB-235 as red oil (60/40 unassigned rotamers mixture). *Rf* = 0.31 (n-Heptane:20%EtOAC); ¹H NMR (400 MHz, Chloroform-*d*) δ 10.18 (s, 1H), 8.84 - 8.71 (m, 1H), 8.45 - 8.36 (m, 1H), 7.87 - 7.74 (m, 1H), 4.54 (s, 1.2H), 4.47 (s, 0.8H), 3.85 (s, 0.8H), 3.69 (s, 1.2H), 1.44 - 1.36 (m, 9H), 1.38 (s, 9H); ¹³C NMR (101 MHz, Chloroform-d) δ 168.63, 165.14, 158.04, 155.71, 155.39, 141.70, 141.46, 141.26, 140.93, 136.06, 135.93, 133.58, 133.52, 126.70, 126.48, 95.21, 81.83, 81.74, 81.07, 80.85, 51.11, 50.71, 49.47, 49.33, 28.31, 28.26, 28.07.

### Example 110

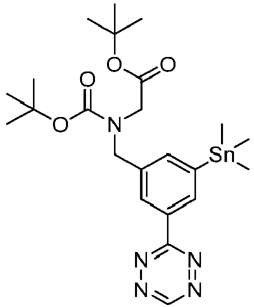

**tert-butyl 2-((3-(1,2,4,5-tetrazin-3-yl)-5-(trimethylstannyl)benzyl)(tert-butoxycarbonyl)amino)acetate (UB-266).** The final compound was obtained from 77 mg (0.15 mmol) of the starting material, following the *General Procedure D.2.* The crude was purified using flash chromatography (95/5 n-Heptane/EtOAc) to yield 0.055 g (66%) of UB-266 as a purple oil (60/40 unassigned rotamers mixture). *Rf* = 0.38 (n-Heptane:20%EtOAc); ¹H NMR (400 MHz, Chloroform-d) δ 10.21 (s, 1H), 8.74 - 8.60 (m, 1H), 8.45 - 8.37 (m, 1 H), 7.70 - 7.61 (m, 2H), 4.65 (s, 1.2H), 4.58 (s, 0.8H), 3.90 (s, 0.8H), 3.75 (s, 1.2H), 1.49 (s, 9H), 1.44 (s, 9H), 0.36 (s, 9H); ¹³C NMR (101 MHz, Chloroform-d) δ 168.85, 166.70, 157.76, 155.81, 155.62, 144.69, 140.25, 139.75, 138.58, 138.33, 134.55, 131.09, 127.41, 81.62, 81.51, 80.69, 80.51, 51.60, 51.17, 49.25, 48.95, 28.37, 28.29, 28.07, -9.34; HPLC-MS [M+H]⁺ m/z calc. for [C₂₃H₃₆N₅SnO₄]⁺: 566.18; Found: 566.19.
The synthesis of UB-108 and UB-137 using UB-266 is shown in Figure 6. Figure 8a displays the NMR chromatogram.

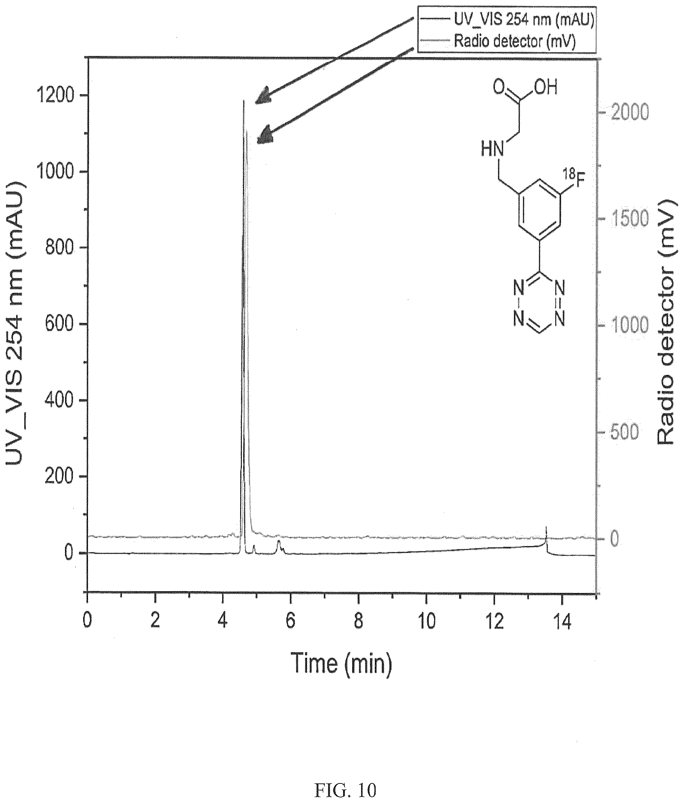

**Example 111 of UB-137:** See radiochemistry section and Figure 1, 6 and 12
The radiolabelling of UB-134 was provided as described in the Example 128, 129, 130, 131 and 132 and as shown in Figure 1 and the final structure is also shown in Figure 4. The HPLC test of UB-137 was performed as described in Example 135, and the HPLC chromatogram is shown in Figure 10.

### Example 112

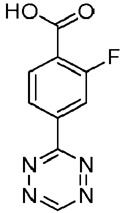

**2-Fluoro-4-(1,2,4,5-tetrazin-3-yl)benzoic acid (UB-12)** The compound was obtained from 4-cyano-2-fluorobenzoic acid (0.66 g, 4.00 mmol) following the following *General Procedure C.* Purification by flash chromatography (98/2 CH₂Cl₂/MeOH) afforded 0.32 g (36%) of UB-12 as a red solid. Rf = 0.31 (Heptane/EtOAc 30/70); ¹H NMR (600 MHz, Methanol-*d*₄) δ 10.44 (s, 1H), 8.50 (dd, *J* = 8.1, 1.6 Hz, 1H), 8.40 (dd, *J* = 11.5, 1.7 Hz, 1H), 8.20 (t, *J* = 7.7 Hz, 1H); ¹³C NMR (151 MHz, Methanol-*d*₄) δ 165.11 (d, *J* = 3.3 Hz), 165.04 (d, *J* = 2.7 Hz), 162.10 (d, *J* = 259.0 Hz), 158.24 (d, *J* = 10.2 Hz), 137.97 (d, *J* = 8.8 Hz), 132.85 (d, *J* = 1.2 Hz), 123.10 (d, *J* = 4.0 Hz), 122.84 (d, *J* = 10.7 Hz), 115.83 (d, *J* = 25.7 Hz); MS (ESI) m/z [M - H]⁻: 219.04

### Example 113

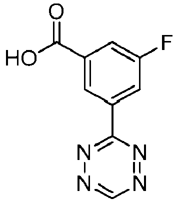

**3-Fluoro-5-(1,2,4,5-tetrazin-3-yl)benzoic acid (UB-65).** The final compound was obtained from 3-Fluoro-5-cyanobenzoic acid (0.66 g, 4.00 mmol) following *General Procedure C*. The crude was purified using flash chromatography (98/2 CH₂Cl₂/MeOH) to yield 0.21 g (24%) of UB-65 as a red solid. *Rf* = 0.31 (n-Heptane:60%EtOAC); ¹H NMR (400 MHz, Methanol-*d*₄) δ 10.32 (s, 1H), 8.95 (t, *J* = 1.5 Hz, 1H), 8.42 (ddd, *J* = 9.2, 2.6, 1.5 Hz, 1H), 7.90 (ddd, *J* = 8.8, 2.7, 1.4 Hz, 1H); ¹³C NMR (101 MHz, Methanol-*d*₄) δ 166.04, 165.07, 163.08 (d, *J* = 247.1 Hz), 158.29, 134.91 (d, *J* = 8.2 Hz), 134.49 (d, *J* = 7.3 Hz), 124.63 (d, *J* = 3.1 Hz), 119.87 (d, *J* = 23.2 Hz), 118.29 (d, *J* = 24.7 Hz); HPLC-MS [M-H]⁻ m/z calc. for [C₉H₄FN₄O₂]⁺: 219.03; Found: 219.07.

### Example 114

**Di-tert-butyl 2,2'-((4-cyano-2-fluorobenzyl)azanediyl)diacetate (UB-41)** The compound was obtained from 4-(bromomethyl)-3-fluorobenzonitrile (0.85 g, 3.97 mmol) following the procedure employed for example 95. Purification by flash chromatography (n-Heptane/EtOAc 90/10) afforded 1.5 g (90%) of the desired compound as a colorless oil. Rf = 0.34 (n-Heptane/EtOAc 80/20); ¹H NMR (400 MHz, Chloroform-*d*) δ 7.83 (t, *J* = 7.5 Hz, 1H), 7.47 (dd, *J* = 7.9, 1.6 Hz, 1H) , 7.33 (dd, *J* = 9.2, 1.7 Hz, 1H) , 4.03 (s, 2H), 3.45 (s, 4H), 1.48 (s, 18H); ¹³C NMR (101 MHz, Chloroform-d) δ 170.21, 160.53 (d, *J* = 250.0 Hz), 132.26 (d, *J* = 14.0 Hz), 132.19 (d, *J* = 5.1 Hz), 128.20 (d, *J* = 3.8 Hz), 118.79 (d, *J* = 25.5 Hz), 117.73 (d, *J* = 2.8 Hz), 112.16 (d, *J* = 9.5 Hz), 81.34, 55.61, 50.06, 28.13.

### Example 115

### Di-tert-butyl 2,2'-((2-fluoro-4-(1,2,4,5-tetrazin-3-yl)benzyl)azanediyl)diacetate (UB-42)

The compound was obtained from Di-*tert*-butyl 2,2'-((4-cyano-2-fluorobenzyl)azanediyl)diacetate (1.5 g, 3.96 mmol) following following *General Procedure C.* Purification by flash chromatography (n-Heptane/EtOAc 85/15) afforded 0.25 g (15%) of the desired compound as a red oil. Rf = 0.39 (n-Heptane/EtOAc 80/20); ¹H NMR (400 MHz, Chloroform-d) δ 10.16 (s, 1H), 8.34 (dd, *J* = 8.1, 1.6 Hz, 1H), 8.21 (dd, *J* = 10.6, 1.7 Hz, 1H), 7.80 (t, *J* = 7.7 Hz, 1H), 4.01 (s, 2H), 3.42 (s, 4H), 1.40 (s, 18H); ¹³C NMR (101 MHz, Chloroform-d) δ 170.33, 165.69 (d, *J* = 3.0 Hz), 161.70 (d, *J* = 247.8 Hz), 157.89, 132.38, 132.31 (d, *J* = 4.4 Hz), 131.48 (d, *J* = 14.5 Hz), 124.02 (d, *J* = 3.4 Hz), 114.88 (d, *J* = 25.1 Hz), 81.21, 55.58, 50.18 (d, *J* = 2.9 Hz), 28.15.

### Example 116

### 1-Carboxy-N-(carboxymethyl)-N-(2-fluoro-4-(1,2,4,5-tetrazin-3-yl)benzyl)methanaminium 2,2,2-trifluoroacetate (UB-44)

The compound was obtained from di-*tert*-butyl 2,2'-((2-fluoro-4-(1,2,4,5-tetrazin-3-yl)benzyl)azanediyl)diacetate (0.24 g, 0.55 mmol) following the procedure employed in example 97. Purification by preparative HPLC afforded 0.02 g (8%) of UB-44 as a red solid. ¹H NMR (400 MHz, Methanol-*d*₄) δ 10.41 (s, 1H), 8.50 (dd, *J* = 8.0, 1.6 Hz, 1H), 8.38 (dd, *J* = 10.9, 1.6 Hz, 1H), 7.90 (t, *J* = 7.7 Hz, 1H), 4.49 (s, 2H), 3.96 (s, 4H); ¹³C NMR (101 MHz, Methanol-*d*₄) δ 170.22, 165.29, 162.05 (d, *J* = 248.2 Hz), 158.17, 135.18 (d, *J* = 8.2 Hz), 133.50 (d, *J* = 3.6 Hz), 125.50 (d, *J* = 13.7 Hz), 123.84 (d, *J* = 3.5 Hz), 114.61 (d, *J* = 25.2 Hz), 53.85, 51.04; HPLC-MS [M+H]⁺ m/z calc. for [C₁₃H₁₃FN₅O₄]⁺: 322.09; Found: 322.12

### Example 117

### Tert-butyl 2-((4-cyano-2-fluorobenzyl)amino)acetate (UB-33)

The compound was obtained from 4-(bromomethyl)-3-fluorobenzonitrile (1.00 g, 4.67 mmol) following the procedure example 103. Purification by flash chromatography (n-Heptane/EtOAc 80/20) afforded 0.52 g (42%) of the desired compound as a colorless oil. Rf = 0.31 (n-Heptane/EtOAc 60/40); ¹H NMR (400 MHz, Chloroform-d) δ 7.48 (t, *J* = 7.5 Hz, 1H), 7.32 (dd, *J* = 7.9, 1.5 Hz, 1H), 7.21 (dd, *J* = 9.4, 1.6 Hz, 1H), 3.79 (s, 2H), 3.19 (s, 2H), 1.91 (s, 1H), 1.34 (s, 9H); ¹³C NMR (101 MHz, Chloroform-*d*) δ 171.14, 160.28 (d, *J* = 249.5 Hz), 133.12 (d, *J* = 14.8 Hz), 130.95 (d, *J* = 5.2 Hz), 128.13 (d, *J* = 3.8 Hz), 118.69 (d, *J* = 25.5 Hz), 117.51, 112.03 (d, *J* = 9.6 Hz), 81.23, 50.85, 45.89 (d, *J* = 3.1 Hz).

### Example 118

### Tert-butyl 2-((2-fluoro-4-(1,2,4,5-tetrazin-3-yl)benzyl)amino)acetate (UB-35)

Tert-butyl 2-((4-cyano-2-fluorobenzyl)amino)acetate (0.5 g, 1.89 mmol), CH₂Cl₂ (0.121 mL, 1.89 mmol), sulfur (0.12 g, 0.47 mmol) and ethanol (4.0 mL) were mixed together in a 20 ml microwave reaction tube. Hydrazine monohydrate (0.74 mL, 15.13 mmol) was added slowly with stirring afterwards. The vessel was sealed and the reaction mixture was heated to 50 °C for 24 hours. Then 3 ml of CH₂Cl₂ and sodium nitrite (1.30 g, 18.92 mmol) in 10 ml of H₂O were added to the mixture. Excess acetic acid (7 mL) was then added slowly during which the solution turned bright red in color. The reaction mixture was extracted with dichloromethane. The organic phase was dried over anhydrous magnesium sulfate (MgSO₄), filtered and concentrated under reduced pressure. The resulting residue was purified using flash chromatography (70/30 heptane/EtoAc) to yield 0.120 g (20%) of red oil. Rf = 0.32 (n-Heptane/EtOAc 60/40); ¹H NMR (400 MHz, Chloroform-d) δ 10.44 (s, 1H), 8.51 (dd, *J* = 8.1, 1.7 Hz, 1H) , 8.43 (dd, *J* = 10.8, 1.7 Hz, 1H) , 7.88 (t, *J* = 7.8 Hz, 1H) , 3.79 (s, 2H), 3.19 (s, 2H), 1.91 (s, 1H) , 1.34 (s, 9H).

### Example 119

### 1-Carboxy-N-(2-fluoro-4-(1,2,4,5-tetrazin-3-yl)benzyl)methanaminium 2,2,2-trifluoroacetate (UB-39)

To a solution of *tert*-butyl 2-((*tert*-butoxycarbonyl)(2-fluoro-4-(1,2,4,5-tetrazin-3-yl)benzyl)amino)acetate (0.10 g, 0.33 mmol) in DCM (5 mL) was added trifluoroacetic acid (5 mL). The reaction was stirred at room temperature for 2 h. The solvent was then removed under reduced pressure to obtain a pink solid. NMR of the crude shows full conversion. Purification by preparative HPLC afforded 0.05 g (40%) of UB-39 as a red solid. ¹H NMR (400 MHz, Methanol-*d*₄) δ 10.44 (s, 1H), 8.54 (dd, *J* = 8.1, 1.6 Hz, 1H), 8.45 (dd, *J* = 10.8, 1.6 Hz, 1H) , 7.86 (t, *J* = 7.7 Hz, 1H) , 4.52 (s, 2H), 4.07 (s, 2H); ¹³C NMR (101 MHz, Methanol-*d*₄) δ 167.23, 165.09 (d, *J* = 2.9 Hz), 161.74 (d, *J* = 249.3 Hz), 158.27, 136.24 (d, *J* = 8.7 Hz), 133.05 (d, *J* = 3.2 Hz), 124.10, 122.61 (d, *J* = 15.4 Hz), 114.86 (d, *J* = 24.6 Hz), 43.79 (d, *J* = 3.7 Hz); HPLC-MS [M+H]⁺ m/z calc. for [C₁₁H₁₁FN₅O₂]⁺: 264.09; Found: 264.08.

### Example 120

### 2-(4-Cyanophenyl)-N-(2-fluoroethyl)acetamide (UB-160)

To a solution of the 2-(4-cyanophenyl)acetic acid (0.50 g, 3.10 mmol) in DCM (40 mL) was added CDI (0.65 g, 4.03 mmol) and the resulting mixture was stirred for 1 h at room temperature. Subsequently the amine (0.62 g, 6.20 mmol) was added and the reaction stirred overnight. The organic layer was washed with NaHCO₃ saturated solution (2 x 20 mL), 1 M HCI solution (2 x 20 mL) and brine (2 x 20 mL); dried over Na₂SO₄ filtered and conventrated under reduced pressure to give 0.46 g (72%) of UB-160 as a white solid. R.f.: 0.35 (Heptane/EtOAc 30/70); m.p.: 139-141 °C; ¹H-NMR (CDCl₃, 400 MHz): 3.43-3.49 (m, 1H, CH), 3.50-3.59 (m, 3H, Ch + CH₂), 4.35 (t, J = 4.8 Hz, 1H, CH), 4.47 (t, J = 4.8 Hz, 1H, CH), 5.88 (br s, 1H, NH), 7.34 (d, J = 8.2 Hz, 1H, CH), 7.57 (d, J = 8.2 Hz, 1H, CH); ¹³C-NMR (CDCl₃, 100 MHz): 40.2 (J_{C-F} = 19.5 Hz), 43.4, 82.5 (J_{C-F} = 166.7 HZ), 111.3, 118.6, 130.1, 132.6, 140.0, 169.6.

### Example 120a

### 2-(4-(1,2,4,5-Tetrazin-3-yl)phenyl)-N-(2-fluoroethyl)acetamide (UB-163)

The compound was obtained from tert-butyl 2-(4-Cyanophenyl)-N-(2-fluoroethyl)acetamide (0.40 g, 1.94 mmol),) following *General Procedure C*. The resulting residue was purified using flash chromatography (30/70 Heptane/EtOAc) to yield 0.15 g of a red solid. The powder was triturated in DCM and fileterd to afford 0.21 g (41%) of UB-163 as a pink solid. R.f. = 0.22 (Heptane/EtOAc 40/60); ¹H-NMR (DMSO-d₆, 400 MHz): 3.36 (q, J = 5.2 Hz, 1H, CH), 3.43 (q, J = 5.2 Hz, 1H, CH), 3.62 (s, 2H, CH₂), 4.39 (t, J = 5.0 Hz, 1H, CH), 5.02 (t, J = 5.0 Hz, 1H, CH), 7.57 (d, J = 8.2 HZ, 2H, Ar-H), 8.39-8.49 (m, 3H, NH + Ar-H), 10.58 (s, 1H, Ar-H); ¹³C-NMR (DMSO-*d*₆, 100 MHz): 40.6 (J_{C-F} = 21.2 Hz), 42.6, 82.9 (J_{C-F} = 165.0 Hz), 128.1, 130.5, 130.6, 142.0, 158.5, 165.9, 170.3.

### Example 120b

### 5-((4-cyano-2-fluorophenyl)amino)-5-oxopentanoic acid (UB-34)

To a solution of 3-fluoro-4aminobenzonitrile (1.0 g, 7.34 mmol) in anhydrous THF (20.0 mL) was added under nitrogen glutaric anhydride (0.84 g, 7.34 mmol). The reaction was refluxed 24 h. The solvent was removed under reduced pressure and the crude purified by flash chromatography (95/5 DCM/MeOH) to give 1.81 g of a yellow solid. Recrystallization from EtOAc afforeded 1.65 g (90%) of UB-34 as a white solid. m.p.: 140-142 °C; ¹H-NMR (DMSO-d₆, 400 MHz): 1.81 (quint, J = 7.4 Hz, 2H, CH₂), 2.28 (t, J = 7.4 Hz, 2H, CH₂), 2.49 (t, J = 7.4 Hz, 2H, CH₂), 7.65 (dd, J = 1.2, 8.6 Hz, 1H, Ar-H), 7.88 (d, J = 1.9, 11.0, Hz, 1H, Ar-H), 8.27 (pseudo t, J = 8.2 Hz, 1H, Ar-H), 10.09 (br s, 1H, NH), 12.07 (br s, 1H, COOH); ¹³C-NMR (DMSO-d₆, 100 MHz): 20.7, 33.3, 35.5, 106.3 (J_{C-F} = 9.4 Hz), 118.3, 119.8 (J_{C-F} = 23.4 Hz), 123.6 (J_{C-F} = 2.9 Hz), 129.8 (J_{C-F} = 3.5 Hz), 132.0 (J_{C-F} =11.3 Hz), 152.2 (J_{C-F} = 247.5 Hz), 172.4, 174.5.

### Example 120c

### 5-((2-fluoro-4-(1,2,4,5-tetrazin-3-yl)phenyl)amino)-5-oxopentanoic acid (UB-37)

5-((4-Cyano-2-fluorophenyl)amino)-5-oxopentanoic acid (0.80 g, 3.20 mmol), CH₂Cl₂ (3.19 mmol, 0.204 mL), sulfur (0.204 g, 0.80 mmol) and ethanol (5.0 mL) were mixed together in a 20 ml microwave reaction tube. Hydrazine monohydrate (1.24 mL, 25.57 mmol) was added slowly with stirring afterwards. The vessel was sealed and the reaction mixture was heated to 50 °C for 24 hours. Then 3 ml of CH₂Cl₂ and sodium nitrite (2.20 g, 31.97 mmol) in 40 ml of H₂O were added to the mixture. Excess acetic acid (14 mL) was then added slowly during which the solution turned bright red in color. The reaction mixture was extracted with dichloromethane. The organic phase was dried over anhydrous magnesium sulfate (MgSO₄), filtered and concentrated under reduced pressure. The resulting residue was purified using flash chromatography (95/5 DCM/MeoH) to yield 0.25 g of a red solid. The NMR shows some impurity so the compound was crystallized from EtOH to 0.210 (21%) of UB-37 as a red solid. R.f. = 0.27 (DCM/MeoH 95/5); mp = 205-207 °C; ¹H-NMR (DMSO-d₆, 400 MHz): 1,83 (quint, J = 7.3 Hz, 2H, CH₂), 2.31 (t, J = 7.4 Hz, 2H, CH₂), 2.53 (t, J = 7.4 Hz, 2H, CH₂), 8.24-8.39 (m, 3H, Ar-H), 10.07 (br s, 1H, NH), 10.58 (s, 1H, Ar-H) 12.08 (br s, 1H, COOH); ¹³C-NMR (DMSO-d₆, 100 MHz): 20.8, 33,4, 35.5, 114.8 (J_{C-F} = 22.2 Hz), 123.9 (J_{C-F} = 2.4 Hz), 124.7 (J_{C-F} = 3.2 Hz), 128.1 (J_{C-F} = 7.7 Hz), 131.3 (J_{C-F} = 11.3 Hz), 153.4 (J_{C-F} = 245.8 Hz), 158.4, 164.9 (J_{C-F} = 3.0 Hz), 174.5

### Example 120d

### Tert-butyl 4-cyano-2-fluorobenzylcarbamate (UB-32)

4-(Aminomethyl)-3-fluorobenzonitrile hydrochloride (1.5 g, 8.04 mmol) and triethylamine (2.35 mL, 16.87 mmol) were dissolved in anhydrous DCM (40 mL) at 0 ° C. To this stirred solution was added di-tert-butyl dicarbonate (2.10 g, 9.64 mmol), and the reaction allowed to warm to room temperature and stirred for 16 hours. The reaction mixture was evaporated in vacuo, and the residue was re-dissolved in diethyl ether (50 mL), which was washed successively with 0.5 M aq. HCI (2 x 25 mL), saturated NaHCO₃ (2 x 25 mL) and brine (25 mL). The organic layer was dried with MgSO₄, filtered and evaporated in vacuo to give an off-white solid. The residue was purified by flash column chromatography (Heptane/EtOAc = 85/15) to afford 1.51 g (75%) od UB-32 as an orange solid. m.p.: 51-53 °C; ¹H-NMR (CDCl₃, 400 MHz): 1.47 (s, 9H, C(CH₃)₃), 4.42 (d, J = 4.4 Hz, 2H, CH₂), 4.99 (br s, 1H, NH), 7.36 (dd, J = 1.4, 9.4 Hz, 1H, Ar-H), 7.42-7.56 (m, 2H, Ar-H); ¹³C-NMR (CDCl₃, 100 MHz): 28.2, 30.0, 79.8, 112.1 (J_{C-F} = 9.5 Hz), 117.4 (J_{C-F} = 2.9 Hz), 118.7 (J_{C-F} = 25.0 Hz), 128.2 (J_{C-F} = 3.9 Hz), 130.1 (J_{C-F} = 5.0 Hz), 132.6 (J_{C-F} = 15.5 Hz), 156.0, 159.9 (J_{C-F} = 249.8 Hz).

### Example 120e

### Tert-butyl 2-fluoro-4-(1,2,4,5-tetrazin-3-yl)benzylcarbamate (UB-35)

Tert-butyl 4-cyano-2-fluorobenzylcarbamate (1.5 g, 5.99 mmol), CH₂Cl₂ (5.99 mmol, 0.38 mL), sulfur (0.38 g, 1.49 mmol) and ethanol (7.0 mL) were mixed together in a 20 ml microwave reaction tube. Hydrazine monohydrate (2.33 mL, 47.94 mmol) was added slowly with stirring afterwards. The vessel was sealed and the reaction mixture was heated to 50 °C for 24 hours. Then 12 ml of CH₂Cl₂ and sodium nitrite (4.13 g, 59.93 mmol) in 30 ml of H₂O were added to the mixture. Excess acetic acid (25 mL) was then added slowly during which the solution turned bright red in color. The reaction mixture was extracted with dichloromethane. The organic phase was dried over anhydrous magnesium sulfate (MgSO₄), filtered and concentrated under reduced pressure. The resulting residue was purified using flash chromatography (90/10 Heptane/EtOAc) and recrystallized from Heptane to yield 0.39 g (21%) of UB-35 as a red solid. R.f = 0.38 (Heptane/EtOAc 80/20); ¹H-NMR (CDCl₃, 400 MHz): 1.48 (s, 9H, C(CH₃)₃), 4.49 (d, J = 6.3 Hz, 2H, CH₂), 5.11 (br s, 1H, NH), 7.60 (pseudo t, J = 7.7 Hz, 1H, Ar-H), 8.30 (dd, J = 1.7, 10.8 Hz, 1H, Ar-H), 8.41 (dd, J = 1.6, 8.0 Hz, 1H, Ar-H), 10.25 (s, 1H, Ar-H); ¹³C-NMR (CDCl₃, 100 MHz): 28.4, 38.6, 80.0, 115.0 (J_{C-F} = 24.5 Hz), 124.1, 130.5, 131.6 (J_{C-F} = 15.0 Hz), 132.6 (J_{C-F} = 8.5 Hz), 155.8, 157.9, 161.2 (J_{C-F} = 247.6 Hz), 165.5.

### Example 120f

### (2-Fluoro-4-(1,2,4,5-tetrazin-3-yl)phenyl)methanamine (UB-38)

To a solution of tert-butyl 2-fluoro-4-(1,2,4,5-tetrazin-3-yl)benzylcarbamate (0.300 g, 0.98 mmol) in DCM (20 mL) was added a solution of HCI in diethyl ether (1.0 M, 20.0 mL). The mixture was stirred at room temperature for 72 h. The reaction was then concentrated under reduced pressure to give 0.23 g (97%) of UB-38 as a pink solid. ¹H-NMR (MeOD, 400 MHz): 2.17 (s, 2H, CH₂), 7.74 (pseudo t, J = 7.8 Hz, 1H, Ar-H), 8.33 (dd, J = 1.6, 10.9 Hz, 1H, Ar-H), 8.43 (dd, J = 1.6, 8.0 Hz, 1H, Ar-H), 10.34 (s, 1H, Ar-H); ¹³C-NMR (MeOD, 100 MHz): 36.4 (J_{C-F} = 4.2 Hz), 114.7 (J_{C-F} = 24.5 Hz), 124.1 (J_{C-F} = 3.7 Hz), 124.8 (J_{C-F} = 15.3 Hz), 132.0 (J_{C-F} = 3.5 Hz), 135.6 (J_{C-F} = 8.4 Hz), 158.3, 161.4 (J_{C-F} = 248.3 Hz), 165.1.

### Example 120g

### 5-((2-Fluoro-4-(1,2,4,5-tetrazin-3-yl)benzyl)amino)-5-oxopentanoic acid (UB-40)

To a solution of UB-38 (0.20 g, 0.83 mmol), and EtsN (0.34 mL, 2.48 mmol) in THF (20 mL) was added glutaric anhydride (0.14 g, 1.24 mmol). The reaction was stirred at 50 °C for 2 h. The solvent was then removed under reduced pressure and the residue solubilized in water (20 mL). The pH was adjusted to 2 with 1 M HCI and the reaction mixture was extracted with EtOAc (3 x 20 mL). The organic phase was dried over anhydrous magnesium sulfate (MgSO₄), filtered and concentrated under reduced pressure to give 0.300 g of crude material (NMR confirmed full conversion). The resulting residue was purified using flash chromatography (95/5 DCM/MeOH) and crystallized from EtOH to give 0.21 g of UB-40 as a red solid. R.f. = 0.21 (DCM/MeoH 95/5); m.p.= 147-149 °C ; ¹H-NMR (CD₃OD, 400 MHz): 1.95 (quint, J = 7.4 Hz, 2H, CH₂), 2.37 (t, J = 7.4 Hz, 4H, 2 x CH₂), 4,52 (s, 2H, CH₂), 7.63 (pseudo t, J = 7.8 Hz, 1H, Ar-H), 8.29 (dd, J = 1.7, 11.0 Hz, 1H, Ar-H), 8.41 (dd, J = 1.7, 8.1 Hz, 1H, Ar-H), 10.37 (s, 1H, Ar-H); ¹³C-NMR (CD₃OD, 100 MHz): 20.8, 32,7, 34.5, 36.4 (J_{C-F} = 4.5 Hz), 114.1 (J_{C-F} = 24.6 Hz), 123.6 (J_{C-F} = 3.4 Hz), 130.3 (J_{C-F} = 4.5 Hz), 130.7 (J_{C-F} = 15.1 Hz), 158.0, 161.1 (J_{C-F} = 246.6 Hz), 165.4, 174.1, 175.4.

### Example 121

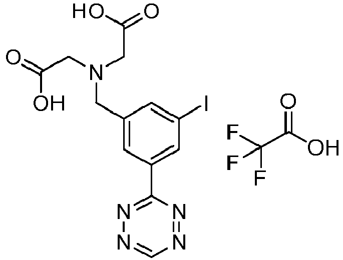

### 1-carboxy-N-(carboxymethyl)-N-(3-iodo-5-(1,2,4,5-tetrazin-3-yl)benzyl)methanaminium 2,2,2-trifluoroacetate (UB-164)

To a solution of Di-tert-butyl 2,2'-((3-iodo-5-(1,2,4,5-tetrazin-3 yl)benzyl)azanediyl)diacetate (UB-156) (0.05 g, 0.15 mmol) in DCM (5 mL) was added trifluoroacetic acid (5 mL). The reaction was stirred at room temeperature for 2 h. The solvent was then removed under reduced pressure to obtain a pik solid. NMR of the crude shows full conversion. Purification by preparative HPLC afforded 0.05 g (62%) of UB-164 as a pink solid. ¹H NMR (400 MHz, Methanol-*d*₄) δ 10.30 (s, 1H), 8.87 (s, 1H), 8.64 (s, 1H), 8.14 (s, 1H), 4.45 (s, 2H), 4.01 (s, 4H); ¹³C NMR (101 MHz, Methanol-*d*₄) δ 168.19, 164.71, 158.23, 143.71, 137.65, 134.59, 134.01, 129.55, 94.51, 57.76, 53.56.

### Example 122

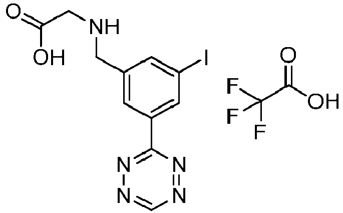

### 1-carboxy-N-(3-iodo-5-(1,2,4,5-tetrazin-3-yl)benzyl)methanaminium 2,2,2-trifluoroacetate (UB-240)

To a solution of tert-butyl 2-((tert-butoxycarbonyl)(3-iodo-5-(1,2,4,5-tetrazin-3-yl)benzyl)amino)acetate (UB-235) (0.07 g, 0.13 mmol) in DCM (6 mL) was added trifluoroacetic acid (6 mL). The reaction was stirred at room temperature for 2 h. The solvent was then removed under reduced pressure to obtain a pink solid. NMR of the crude shows full conversion. Purification by preparative HPLC afforded 0.04 g (65%) of UMB-240 as a pink oil. ¹H NMR (400 MHz, Chloroform-d) δ 10.42 (d, *J* = 1.3 Hz, 1H), 9.01 (d, *J* = 1.7 Hz, 1H), 8.76 (d, *J* = 1.7 Hz, 1H), 8.22 (d, *J* = 1.7 Hz, 1H), 4.40 (s, 2H), 4.02 (s, 2H); ¹³C NMR (101 MHz, Chloroform-d) δ 171.21, 168.65, 162.21, 146.63, 141.53, 138.77, 138.15, 132.44, 98.49, 53.50, 50.50.

### Example 123

### Di-tert-butyl 2,2'-((4-cyano-2-iodobenzyl)azanediyl)diacetate (UB-45)

To a solution of 4-cyano-2-iodobenzylbromide (2.0 g, 6.21 mmol) in CH₃CN (50 mL) was added K₂CO₃ (1.28 g, 9.32 mmol) and the corresponding amine (1.83 g, 7.45 mmol). The reaction mixture was stirred at RT overnight. The solvent was removed in vacuo, and the resulting mixture was diluted with water (20 mL), extracted with EtOAc (2 x 50 mL), washed with brine (30 mL), dried over MgSO₄, filtered and concentrated in vacuo to give 3.01 g (99%) of UB-45 as a yellow oil. R.f. = 0.32 (80/20 Heptane/EtOAc); ¹H NMR (400 MHz, Chloroform-*d*) δ 7.99 (d, *J* = 1.6 Hz, 1H), 7.79 (d, *J* = 8.0 Hz, 1H), 7.56 (dd, *J* = 8.0, 1.7 Hz, 1H), 3.90 (s, 2H), 3.38 (s, 4H), 1.39 (s, 18H); ¹³C NMR (101 MHz, Chloroform-*d*) δ 170.28, 147.15, 142.08, 131.71, 130.39, 117.24, 112.49, 98.69, 62.50, 55.72, 28.18.

### Example 123a

### Di-tert-butyl 2,2'-((2-iodo-4-(1,2,4,5-tetrazin-3-yl)benzyl)azanediyl)diacetate (UB-46)

Di-tert-butyl 2,2'-((4-cyano-2-iodobenzyl)azanediyl)diacetate (3.0 g, 6.17 mmol), CH₂Cl₂ (0.39 mL, 6.17 mmol), sulfur (0.39 g, 1.54 mmol) and ethanol (6.0 mL) were mixed together in a 20 ml microwave reaction tube. Hydrazine monohydrate (2.40 mL, 49.34 mmol) was added slowly with stirring afterwards. The vessel was sealed, and the reaction mixture was heated to 50 °C for 24 hours. Then 12 ml of CH₂Cl₂ and sodium nitrite (4.25 g, 61.68 mmol) in 60 ml of H₂O were added to the mixture. Excess acetic acid (30 mL) was then added slowly during which the solution turned bright red in color. The reaction mixture was basified with NaHCO₃ saturated solution and extracted with dichloromethane. The organic phase was dried over anhydrous magnesium sulfate (MgSO₄), filtered and concentrated under reduced pressure. The resulting residue was purified using flash chromatography (90/10 heptane/EtoAc) and recrustallized from heptane to yield 0.71 g (21%) of UB-46 as a red solid. R.f. = 0.33 (80/20 Heptane/EtOAc); ¹H NMR (400 MHz, Chloroform-*d*) δ 10.16 (s, 1H), 9.01 (d, *J* = 1.8 Hz, 1H), 8.53 (dd, *J* = 8.1, 1.8 Hz, 1H), 7.88 (d, *J* = 8.1 Hz, 1H), 4.01 (s, 2H), 3.45 (s, 4H), 1.41 (s, 18H); ¹³C NMR (101 MHz, Chloroform-*d*) δ 170.21, 165.29, 157.90, 146.48, 138.84, 132.03, 130.94, 127.96, 99.98, 81.40, 62.44, 55.58, 28.21.

### Example 123b

### 1-Carboxy-N-(carboxymethyl)-N-(2-iodo-4-(1,2,4,5-tetrazin-3-yl)benzyl)methanaminium 2,2,2-trifluoroacetate (UB-50)

To a solution of di-tert-butyl 2,2'-((2-iodo-4-(1,2,4,5-tetrazin-3-yl)benzyl)azanediyl)diacetate (0.14 g, 0.26 mmol) in DCM (5 mL) was added trifluoroacetic acid (4 mL). The reaction was stirred at room temeperature for 2 h. The solvent was then removed under reduced pressure to obtain a pik solid. NMR of the crude shows full conversion. Purification by preparative HPLC afforded 0.11 g (78%) of UMB-50 as a pink solid. ¹H NMR (400 MHz, Methanol-*d*₄) δ 10.39 (s, 1H), 9.09 (d, *J* = 1.7 Hz, 1H), 8.64 (dd, *J* = 8.2, 1.8 Hz, 1H), 7.96 (d, *J* = 8.1 Hz, 1H), 4.43 (s, 2H), 3.92 (s, 4H); ¹³C NMR (101 MHz, Methanol-*d*₄) δ 170.76, 164.89, 158.11, 142.21, 138.85, 133.82, 131.71, 127.60, 100.50, 62.38, 53.99

### Example 123c

### Tert-butyl 2-((4-cyano-2-iodobenzyl)amino)acetate (UB-120)

To a solution of 2-iodo-4-cyano-benzylbromide (1.50 g, 4.66 mmol) in CH₃CN (40 mL) was added K₂CO₃ (3.22 g, 23.29 mmol) and tert-butylglycinate HCI (2.34 g, 13.98 mmol). The reaction mixture was stirred at RT overnight. The solvent was removed in vacuo, and the resulting mixture was diluted with water (20 mL), extracted with EtOAc (2 x 25 mL), washed with brine (30 mL), dried over MgSO₄, filtered and concentrated in vacuo. Purification by flash chromatography (80/20 Heptane/EtOAc) afforded 1.45 g (84%) of UB-120 as a colorless oil. R.f = 0.24 (heptane/EtOAc 80/20); ¹H NMR (400 MHz, Chloroform-d) δ 8.08 (d, *J* = 1.6 Hz, 1H), 7.67 - 7.53 (m, 2H), 3.85 (s, 2H), 3.33 (s, 2H), 2.02 (s, 1H), 1.48 (s, 9H); ¹³C NMR (101 MHz, Chloroform-d) δ 171.29, 147.65, 142.16, 131.76, 129.25, 117.10, 112.45, 98.90, 81.53, 57.47, 51.03, 28.13.

### Example 123d

### Tert-butyl 2-((tert-butoxycarbonyl)(4-cyano-2-iodobenzyl)amino)acetate (UB-123)

To a solution of UB-120 (1.4 g, 3.76 mmol) and Et₃N (1.26 mL, 9.03 mmol) in DCM (40 mL) was added Boc₂O (0.91 g, 4.51 mmol). The reaction was stirred at room temerature for 12 h. The solution was then washed with water (50 mL) and K₂CO₃ saturated solution (50 mL), dried over anhydrous Na₂SO₄, filtered and contratated under reduced pressure to afford 2.1 g of the crude. Purification by flash chromatography (90/10 Heptane/EtOAc) afforded 1.42 g (80%) of UB-123 as a white solid. R.f. = 0.25 (Heptane/EtOAc 80/20); ¹H NMR (400 MHz, Chloroform-*d*) δ 8.13 - 8.06 (m, 1H), 7.72 - 7.61 (m, 1H), 7.52 - 7.42 (m, 1H), 4.58 (s, 1.2H), 4.50 (s, 0.8H), 3.92 (s, 0.8H), 3.79 (s, 1.2H), 1.52 - 1.43 (m, 11H), 1.41 (s, 4H); ¹³C NMR (101 MHz, Chloroform-d) δ 168.53, 168.47, 155.60, 155.37, 145.86, 145.55, 142.35, 142.30, 131.88, 131.74, 128.58, 127.98, 117.02, 112.71, 97.71, 97.22, 81.90, 81.59 (d, *J* = 83.6 Hz), 81.13, 57.31, 56.91, 50.37, 49.81, 28.21, 28.09.

### Example 123e

### Tert-butyl 2-((tert-butoxycarbonyl)(2-iodo-4-(1,2,4,5-tetrazin-3-yl)benzyl)amino)acetate (UB-126)

Tert-butyl 2-((tert-butoxycarbonyl)(4-cyano-2-iodobenzyl)amino)acetate (1.30 g, 2.75 mmol), CH₂Cl₂ (0.176 mL, 2.75 mmol), sulfur (0.176 g, 2.75 mmol) and ethanol (3.0 mL) were mixed together in a 20 ml microwave reaction tube. Hydrazine monohydrate (1.10 mL, 22.02 mmol) was added slowly with stirring afterwards. The vessel was sealed and the reaction mixture was heated to 50 °C for 24 hours. Then 3 ml of CH₂Cl₂ and sodium nitrite (1.89 g, 27.52 mmol) in 30 ml of H₂O were added to the mixture. Excess acetic acid (13 mL) was then added slowly during which the solution turned bright red in color. The reaction mixture was basified with NaHCO₃ saturated solution and extracted with dichloromethane. The organic phase was dried over anhydrous magnesium sulfate (MgSO₄), filtered and concentrated under reduced pressure. The resulting residue was purified using flash chromatography (85/15 heptane/EtoAc) to yield 0.22 g (15%) of UB-126 as red solid. R.f. = 0.24 (Heptane/EtoAc 80/20); ¹H NMR (400 MHz, Chloroform-d) δ 10.25 (s, 1H), 9.13 - 9.03 (m, 1H), 8.70 - 8.57 (m, 1H), 7.60 - 7.51 (m, 1H), 4.67 (s, 1.2H), 4.59 (s, 0.8H), 3.97 (s, 0.8H), 3.83 (s, 1.2H), 1.57 - 1.37 (m, 18H); ¹³C NMR (101 MHz, Chloroform-d) δ 168.67, 168.62, 165.12, 157.95, 155.67, 155.56, 145.45, 145.21, 132.09, 129.07, 128.46, 128.05, 127.92, 98.89, 98.39, 81.87, 81.77, 80.98, 80.90, 57.05, 56.57, 50.08, 49.60, 28.26, 28.11.

### Example 123f

### 1-Carboxy-N-(2-iodo-4-(1,2,4,5-tetrazin-3-yl)benzyl)methanaminium 2,2,2-trifluoroacetate (UB-138)

To a solution of *tert*-butyl 2-((tert-butoxycarbonyl)(2-iodo-4-(1,2,4,5-tetrazin-3-yl)benzyl)amino)acetate (0.10 g, 0.19 mmol) in DCM (5 mL) was added trifluoroacetic acid (5 mL). The reaction was stirred at room temeperature for 2 h. The solvent was then removed under reduced pressure to obtain a pik solid. NMR of the crude shows full conversion. Purification by preparative HPLC afforded 0.04 g (43%) of UMB-138 as a pink solid. ¹H NMR (400 MHz, Methanol-*d*₄) δ 10.32 (s, 1H), 9.06 (d, *J* = 1.7 Hz, 1H), 8.60 (dd, *J* = 8.1, 1.8 Hz, 1H), 7.73 (d, *J* = 8.1 Hz, 1H), 4.49 (s, 2H), 4.00 (s, 2H); ¹³C NMR (101 MHz, Methanol-*d*₄) δ 167.23, 164.65, 158.25, 139.13, 138.52, 134.84, 130.89, 128.04, 100.35, 54.74, 46.93.

### Example 124

### Di-tert-butyl 2,2'-((4-cyanobenzyl)azanediyl)diacetate (UB-14)

To a suspension of 4-cyanobenzylamine hydrochloride (0.67 g, 4.00 mmol) in DCM (20 mL) was added triethylamine (2.78 mL, 20.00 mmol) and tert-butylbromoacetate (2.93 mL, 20.00 mL). The reaction was stirred at room temperature for 12 h. The solution was then washed with water (2 x 20 mL), dried over Na₂SO₄, fileterd and concetrated under reduced pressure. The crude was purified by flash chromatography (24 g column, 100% DCM to 98/2 DCM/MeOH) to give 0.85 g (59%) of UB-14 as a colorless oil. ¹H-NMR (CDCl₃, 400 MHz): 1.39 (br s, 18H, 2 x C(CH)₃), 3.14 (s, 4H, 2 x CH₂), 3.90 (s, 2H, CH₂), 7.49 (d, J = 8.2 Hz, 2H, Ar-H), 7.54 (d, J = 8.2 Hz, 2H, Ar-H); ¹³C-NMR (CDCl₃, 100 MHz): 28.2, 55.2, 57.2, 81.3, 111.1, 119.0, 129.5, 132.2, 144.7, 170.2.

### Example 124a

### Di-tert-butyl 2,2'-((4-(1,2,4,5-tetrazin-3-yl)benzyl)azanediyl)diacetate (UB-16)

Di-tert-butyl 2,2'-((4-cyanobenzyl)azanediyl)diacetate (0.85 g, 2.36 mmol), CH₂Cl₂ (0.151 mL, 2.36 mmol), sulfur (0.151 g, 0.58 mmol) and ethanol (4.0 mL) were mixed together in a 20 ml microwave reaction tube. Hydrazine monohydrate (0.92 mL, 18.86 mmol) was added slowly with stirring afterwards. The vessel was sealed and the reaction mixture was heated to 50 °C for 24 hours. Then 3 ml of CH₂Cl₂ and sodium nitrite (1.6 3g, 23.58 mmol) in 40 ml of H₂O were added to the mixture. Excess acetic acid (14 mL) was then added slowly during which the solution turned bright red in color. The reaction mixture was basified with NaHCO₃ saturated solution and extracted with dichloromethane. The organic phase was dried over anhydrous magnesium sulfate (MgSO₄), filtered and concentrated under reduced pressure. The resulting residue was purified using flash chromatography (85/15 heptane/EtoAc) to yield 0.14 g (14%) of UB-16 as a red oil. ¹H-NMR (CDCl₃, 400 MHz): 1.40 (s, 18H, 3 x C(CH₃)), 3.39 (s, 4H, 2 x CH₂), 3.96 (s, 2H, CH₂), 7.59 (d, J = 8.4 Hz, 2H, Ar-H), 8.50 (d, J = 8.4 Hz, 1H, Ar-H), 10.13 (s, 1H, Ar-H); ¹³C-NMR (CDCl₃, 100 MHz): 28.2, 55.3, 57.3, 81.1, 128.3, 129.8, 130.5, 157.7, 166.4, 170.4.

### Example 124b

### N-(4-(1,2,4,5-tetrazin-3-yl)benzyl)-1-carboxy-N-(carboxymethyl)methanaminium 2,2,2-trifluoroacetate (UB-19)

To a solution of di-tert-butyl 2,2'-((4-(1,2,4,5-tetrazin-3-yl)benzyl)azanediyl)diacetate (0.13 g, 0.31 mmol) in DCM (5 mL) was added TFA (3 mL). The reaction was stirred at room temperature for 1 h. Th-e solvent was then evaporated under reduced pressure to give 0.14 g of crude. NMR confirmed full conversion. Purification by preparative HPLC to give 0.035 g (26%) of UB-19 as a red solid. m.p.: 100-102 °C; 1H-NMR (MeOD, 400 MHz): 3.89 (s, 4H, 2 x CH2), 4.36 (s, 2H, CH2), 7.68 (d, J = 8.4 Hz, 2H, Ar-H), 8.55 (d, J = 8.4 Hz, 2H, Ar-H), 10.27 (s, 1H, Ar-H); 13C-NMR (MeOD, 100 MHz): 53.6, 58.0, 128.2, 131.2, 132.9, 137.5, 158.0, 166.0, 169.5.

### Example 124c

### Tert-butyl 2-((4-cyanobenzyl)amino)acetate (UB-15)

To a suspension of 4-cyanobenzylamine hydrochloride (0.67 g, 4.00 mmol) in DCM (20 mL) was added triethylamine (1.22 mL, 8.80 mmol) and tert-butylbromoacetate (0.64 mL, 20.00 mL). The reaction was stirred at room temperature for 12 h. The solution was then washed with water (2 x 20 mL), dried over Na₂SO₄, fileterd and concetrated under reduced pressure. The crude was purified by flash chromatography (24 g column, 100% DCM to 98/2 DCM/MeOH) to give 0.42 g (43%) of UB-15 as a colorless oil. ¹H-NMR (CDCl₃, 400 MHz): 1.49 (br s, 9H, C(CH)₃), 1.95 (br s, 1H, NH), 3.34 (s, 2H, CH₂), 3.90 (s, 2H, CH₂), 7.48 (d, J = 8.0 Hz, 2H, Ar-H), 7.64 (d, J = 8.0 Hz, 2H, Ar-H); ¹³C-NMR (CDCl₃, 100 MHz): 28.1, 50.4, 52.5, 81.8, 111.2, 118.8, 129.0, 132.3, 144.5, 170.0.

### Example 124d

### N-(4-(1,2,4,5-tetrazin-3-yl)benzyl)-1-carboxymethanaminium 2,2,2-trifluoroacetate (UB-28)

Tert-butyl 2-((4-cyanobenzyl)amino)acetate (0.73 g, 2.96 mmol), CH₂Cl₂ (0.190 mL, 2.96 mmol), sulfur (0.19 g, 0.74 mmol) and ethanol (4.0 mL) were mixed together in a 20 ml microwave reaction tube. Hydrazine monohydrate (1.15 mL, 23.71 mmol) was added slowly with stirring afterwards. The vessel was sealed and the reaction mixture was heated to 50 °C for 24 hours. Then 3 ml of CH₂Cl₂ and sodium nitrite (2.05 3g, 29.64 mmol) in 40 ml of H₂O were added to the mixture. Excess acetic acid (14 mL) was then added slowly during which the solution turned bright red in color. The reaction mixture was extracted with dichloromethane. The organic phase was dried over anhydrous magnesium sulfate (MgSO₄), filtered and concentrated under reduced pressure. The resulting residue was purified using flash chromatography (80/20 heptane/EtoAc) to yield 0.170 g of tert-butyl 2-((4-(1,2,4,5-tetrazin-3-yl)benzyl)amino)acetate as a red oil. The compound (0.10 g, 0.33 mmol) was solubilized in DCM (5 mL) and to this mixture was added TFA (3 mL). The reaction was stirred at room temperature for 1 h. The solvent was then evaporated under reduced pressure to give 0.14 g of crude. NMR confirmed full conversion. The compound was crystallized from MeoH to give 0.030 g (37%) of UMB-28 as a pink solid. m.p.: 186-188 °C; ¹H-NMR (MeOD, 400 MHz): 4.13 (s, 1.25H, CH₂), 4.89 (s, 0.75H, CH₂), 4.92 (s, 0.75H, CH₂), 5.49 (s, 1.25H, CH₂), 7.33 (d, J = 8.4 Hz, 0.75H, Ar-H), 7.55 (d, J = 8.4 Hz, 1.25H, Ar-H), 8.43 (d, J = 8.4 Hz, 0.75H, Ar-H), 8.52 (d, J = 8.4 Hz, 1.25H, Ar-H), 10.22 (s, 0.33H, Ar-H), 10.25 (s, 0.67H, Ar-H); ¹³C-NMR (MeOD, 100 MHz): 45.0, 52.1, 55.7, 128.0, 128.2, 128.9, 129.1, 131.5, 132.3, 139.3, 139.8, 157.89, 157.94, 166.0, 167.3, 169.9.

### Example 124e

### Di-tert-butyl 2,2'-((3-cyanobenzyl)azanediyl)diacetate (UB-74)

To a solution of 3-bromomethylbenzonitrile (1.00 g, 5.10 mmol) in CH₃CN (30 mL) was added K₂CO₃ (1.06 g, 7.65 mmol) and the corresponding amine (1.50 g, 6.12 mmol). The reaction mixture was stirred at RT overnight. The solvent was removed in vacuo, and the resulting mixture was diluted with water (20 mL), extracted with EtOAc (2 x 25 mL), washed with brine (30 mL), dried over MgSO₄, filtered and concentrated in vacuo. Purification by flash chromatography (90/10 Heptane/EtOAc) afforded 1.62 g (88%) of UB-74 as a colorless oil. ¹H-NMR (CDCl₃, 400 MHz): 1.46 (s, 18H, 2 x C(CH₃)₃), 3.39 (s, 4H, 2 x CH₂), 3.92 (s, 2H, CH₂), 7.42 (t, J = 7.7 Hz, 1H, Ar-H), 7.51-7.58 (m, 1H, Ar-H), 7.64-7.70 (m, 1H, Ar-H), 7.74 (s, 1H, Ar-H); ¹³C-NMR (CDCl₃, 100 MHz): 28.1, 55.2, 56.7, 81.2, 112.4, 118.8, 129.1, 131.0, 132.3, 133.3, 140.6, 170.2.

### Example 124f

### Di-tert-butyl 2,2'-((3-(1,2,4,5-tetrazin-3-yl)benzyl)azanediyl)diacetate (UB-77)

Di-tert-butyl 2,2'-((3-cyanobenzyl)azanediyl)diacetate (1.60 g, 4.43 mmol), CH₂Cl₂ (0.284 mL, 4.43 mmol), sulfur (0.284 g, 1.11 mmol) and ethanol (4.0 mL) were mixed together in a 20 ml microwave reaction tube. Hydrazine monohydrate (1.73 mL, 35.51 mmol) was added slowly with stirring afterwards. The vessel was sealed and the reaction mixture was heated to 50 °C for 24 hours. Then 3 ml of CH₂Cl₂ and sodium nitrite (3.06 g, 44.39 mmol) in 40 ml of H2O were added to the mixture. Excess acetic acid (16 mL) was then added slowly during which the solution turned bright red in color. The reaction mixture was basified with NaHCO₃ saturated solution and extracted with dichloromethane. The organic phase was dried over anhydrous magnesium sulfate (MgSO₄), filtered and concentrated under reduced pressure. The resulting residue was purified using flash chromatography (85/15 heptane/EtoAc) to yield 0.37 g (20%) of UB-77 as a red oil. R.f. = 0.41 (Heptane/EtOAc 80/20); ¹H-NMR (CDCl₃, 400 MHz): 1.46 (s, 9H, 2 x (C(CH3)3), 3.45 (s, 2H, 2 x CH2), 4.02 (s, 2H, CH2), 7.56 (t, J = 7.7 Hz, 1H, Ar-H), 7.77 (d, J = 7.7 Hz, 1H, Ar-H), 7.85 (d, J = 7.7 Hz, 1H, Ar-H), 8.59 (s, 1H, Ar-H); 10.20 (s, 1H, Ar-H); ¹³C-NMR (CDCl₃, 100 MHz): 28.2, 55.3, 57.2, 81.1, 127.3, 128.7, 129.4, 131.6, 133.9, 140.3, 157.8, 166.5, 170.4.

### Example 124g

### N-(3-(1,2,4,5-tetrazin-3-yl)benzyl)-1-carboxy-N-(carboxymethyl)methanaminium 2,2,2-trifluoroacetate (UB-80)

To a solution of di-tert-butyl 2,2'-((3-(1,2,4,5-tetrazin-3-yl)benzyl)azanediyl)diacetate (0.15 g, 0.36 mmol) in DCM (5 mL) was added trifluoroacetic acid (5 mL). The reaction was stirred at room temeperature for 2 h. The solvent was then removed under reduced pressure to obtain a pik solid. NMR of the crude shows full conversion. Purification by preparative HPLC afforded 0.08 g (53%) of UMB-80 as a pink solid. ¹H-NMR (MeOD, 400 MHz): 4.21 (s, 4H, 2 x CH₂), 4.71 (s, 2H, CH₂), 7.77 (t, J = 7.4 Hz, 1H, Ar-H), 7.88 (d, J = 7.4 Hz, 1H, Ar-H), 8.60-8.73 (m, 1H, Ar-H), 8.8 (s, 1H, Ar-H), 10.40 (s, 1H, Ar-H); ¹³C-NMR (MeOD, 100 MHz): 53.3, 58.5, 129.3, 130.0, 130.6, 131.1, 133.1, 135.4, 158.1, 165.8, 167.6.

### Example 124h

### Tert-butyl 2-((3-cyanobenzyl)amino)acetate (UB-81)

To a solution of 3-cyano-benzylbromide (1.80 g, 9.18 mmol) in CH₃CN (40 mL) was added K₂CO₃ (6.34 g, 45.91 mmol) and tert-butylglycinate HCI (4.62 g, 27.54 mmol). The reaction mixture was stirred at RT overnight. The solvent was removed in vacuo, and the resulting mixture was diluted with water (20 mL), extracted with EtOAc (2 x 25 mL), washed with brine (30 mL), dried over MgSO₄, filtered and concentrated in vacuo. Purification by flash chromatography afforded 1.32 g (58%) of UB-81 as a colorless oil. ¹H-NMR (CDCl₃, 400 MHz): 1.47 (s, 9H, C(CH₃)₃), 3.29 (s, 2H, CH₂), 3.83 (s, 2H, CH₂), 7.43 (t, J = 7.7 Hz, 1H, Ar-H), 7.54 (d, J = 7.7 Hz, 1H, Ar-H), 7.59 (d, J = 7.7 Hz, 1H, Ar-H), 7.66 (s, 1H, Ar-H); ¹³C-NMR (CDCl₃, 100 MHz): 28.1, 50.8, 52.4, 81.4, 112.5, 118.8, 129.2, 130.8, 131.7, 132.6, 141.4, 171.4.

### Example 124i

### Tert-butyl 2-((tert-butoxycarbonyl)(3-cyanobenzyl)amino)acetate (UB-85)

To a solution of tert-butyl 2-((3-cyanobenzyl)amino)acetate (1.16 g, 4.70 mmol) and Et₃N (1.57 mL, 11.30 mmol) in DCM (50 mL) was added Boc₂O (1.23 g, 5.65 mmol). The reaction was stirred at room temerature for 12 h. The solution was then washed with water (50 mL) and K₂CO₃ saturated solution (50 mL), dried over anhydrous Na₂SO₄, filtered and contratated under reduced pressure to afford 1.60 g (98%) of UB-85 as a yellow oil. ¹H-NMR (CDCl₃, 400 MHz): 1.37-1.49 (m, 18H, 2 x C(CH₃)₃), 3.70 (s, 1.25H, CH₂), 3.85 (s, 0.75H, CH₂), 4.48 (s, 0.75H, CH₂), 4.53 (s, 1.25H, CH₂), 7.37-7.60 (m, 4H, Ar-H); ¹³C-NMR (CDCl₃, 100 MHz): 28.0, 28.2, 49.3, 49.5, 50.9, 51.3, 80.8, 80.9, 81.7, 81.8, 112.6, 118.7, 129.3, 130.8, 131.0, 131.2, 131.8, 132.4, 155.3, 155.6, 168.6.

### Example 124I

### Tert-butyl 2-((3-(1,2,4,5-tetrazin-3-yl)benzyl)(tert-butoxycarbonyl)amino)acetate (UB-88)

Tert-butyl 2-((tert-butoxycarbonyl)(3-cyanobenzyl)amino)acetate (1.45 g, 4.18 mmol), CH₂Cl₂ (0.268 mL, 4.18 mmol), sulfur (0.268 g, 1.04 mmol) and ethanol (4.0 mL) were mixed together in a 20 ml microwave reaction tube. Hydrazine monohydrate (1.63 mL, 33.48 mmol) was added slowly with stirring afterwards. The vessel was sealed and the reaction mixture was heated to 50 °C for 24 hours. Then 3 ml of CH₂Cl₂ and sodium nitrite (2.88 g, 41.85 mmol) in 40 ml of H₂O were added to the mixture. Excess acetic acid (16 mL) was then added slowly during which the solution turned bright red in color. The reaction mixture was basified with NaHCO₃ saturated solution and extracted with dichloromethane. The organic phase was dried over anhydrous magnesium sulfate (MgSO₄), filtered and concentrated under reduced pressure. The resulting residue was purified using flash chromatography (85/15 heptane/EtoAc) to yield 0.33 g (20%) of UB-88 as red oil (55/45 rotamers mixture). R.f. = 0.42 (Heptane/EtOAc 80/20); ¹H-NMR (CDCl₃, 400 MHz): 1.37 (s, 9H, C(CH₃)₃), 1.40-1.45 (m, 9H, C(CH₃)₃), 3.69 (s, 1.1H, CH₂), 3.83 (s, 0.9H, CH₂), 4.53 (s, 0.9H, CH₂), 4.59 (s, 1.1H, CH₂), 7.40-7.59 (m, 2H, Ar-H), 8.36-8.53 (m, 2H, Ar-H), 10.15 (s, 1H, Ar-H); ¹³C-NMR (CDCl₃, 100 MHz): 28.0, 28.2, 28.3, 49.0, 49.3, 51.1, 51.6, 80.6, 80.7, 81.6, 81.7, 127.29, 127.36, 127.39, 127.5, 129.6, 129.7, 131.8, 131.9, 132.2, 132.9, 155.6, 155.8, 157.8, 157.9, 165.3, 168.81, 168.85.

### Example 124m

### N-(3-(1,2,4,5-tetrazin-3-yl)benzyl)-1-carboxymethanaminium 2,2,2-trifluoroacetate (UB-92)

To a solution of tert-butyl 2-((3-(1,2,4,5-tetrazin-3-yl)benzyl)(tert-butoxycarbonyl)amino)acetate (0.14 g, 0.36 mmol) in DCM (5 mL) was added trifluoroacetic acid (5 mL). The reaction was stirred at room temeperature for 2 h. The solvent was then removed under reduced pressure to obtain a pik solid. NMR of the crude shows full conversion. Purification by preparative HPLC afforded 0.08 g (62%) of UMB-92 as a pink solid. ¹H-NMR (MeOD, 400 MHz): 4.03 (s, 2H, CH₂), 4.46 (s, 2H, CH₂), 7.79 (t, J = 7.8 Hz, 1H, Ar-H), 7.85 (d, J = 7.8 Hz, 1H, Ar-H), 8.72 (d, J = 7.8 Hz, 1H, Ar-H), 8.79 (s, 1H, Ar-H), 10.41 (s, 1H, Ar-H); ¹³C-NMR (MeOD, 100 MHz): 46.4, 50.3, 128.9, 129.3, 130.0, 132.2, 133.3, 134.0, 158.1, 165.8, 167.3.

### Example 125 Blocking assay

### Establishing tumor xenografts in mice (same procedure for the pretargeted PET imaging)

All animal studies were approved by the Danish Animal Welfare Council, ministry of Justice. Five weeks old female nude BALB/c mice (Charles River, Sulzfeld, Germany) were allowed to acclimatize for one week with access to water and chow ad libitum. Human colon cancer cell line (LS174T; obtained from ATCC) was cultured in minimum essential medium (MEM) supplemented with 10% fetal bovine serum, 1% L-Glutamine, 1% Sodium pyruvate, 1% non-essential amino acids, and 1% penicillin-streptomycin at 37 °C and 5% CO₂. Cells were harvested by trypsination at a confluence of 70-90% and subcutaneous tumors were established in the left flank of the 6 weeks old animals by inoculation of ∼ 5 x 10⁶ LS174T cells resuspended in sterile PBS (100 µL) and allowed to grow for 7-10 days. Tumors were measured using a caliper and the volume calculated using the formula volume = ½ (length x width²).

### Example 126 Blocking experiments

These blocking experiments were made using the method described in Example 125. Tumor-bearing animals were matched in groups based on their tumor volume (tumor volumes of ∼ 100-300 mm³, *n* = 3 in each group) and were administered 100 µg/100 µL of CC49-TCO (100 µg/100 µL, ∼7 TCO/mAb) per mouse. After 3 days, animals were injected with non-radioactive Tz (10-20 nmol). 2 hours later, they were administered with [¹¹¹In]Q (∼13 MBq/100 µL, 1-2 nmol) via the tail vein. Tz [¹¹¹In]**Q** was radiolabeled as described in literature⁹ and chapter 7 in the thesis. The mice were euthanized after 22 h and tumor, blood, heart, lung, liver, spleen, kidney, and muscle were resected. All tissues were weighted and the radioactivity measured in a gamma counter (Wizard2, Perkin Elmer) and data was corrected for decay, tissue weight and injected amount of radioactivity. Figure 3 displays the results. Compounds RGV_52, RGV_55, UB-12, UB-19, UB-28, UB-37, UB-44, UB-39, UB-40, UB-50, UB-138, UB-80, UB-92, UB-108, UB-137 and UB-163 were tested. Results indicate that provide a H-tetrazine compound (with a rate constant of approximately 50.000-90.000 M⁻¹ s⁻¹ (determined with standard TCO at 37 °C in PBS) and a lipophilicity of clogD_{7.4} < -0.5 result in a normalized blocking effect > 70.

### Example 127 Pretargeted Imaging with TCO-modified mAb (CC49-TCO)

Tumor-bearing animals were matched into 4 groups based on their tumor volume (tumor volumes of ∼ 60-180 mm³, *n* = 3 in each group) and were administered with CC49-TCO (100 µg/100 µL, 6.7 nmol) per mouse. After 72 h, the animals were injected via the tail vein with ¹⁸F-labeled Tz (UB-108 and UB-137, respectively) (5-10 MBq/100 µL) was injected via the tail vein. The animals were PET/CT scanned 1 h p.i. of the ¹⁸F-labeled Tz and then euthanized. Tumor and blood was collected, weighted and the radioactivity measured in a gamma counter. Data were corrected for decay, tissue weight and injected amount of radioactivity. Static PET images were acquired with an energy window of 350-650 KeV and a time resolution of 6 ns. CT scans were acquired using 360 projections, 65 kV, 500 µA and 400 ms. PET images were reconstructed using a 3-dimensional maximum a posteriori algorithm with CT-based attenuation correction. PET and CT images were co-registered and analyzed using Inveon software (Siemens). The mean percentage of injected dose per grams of tissue (%ID/g) in the tissue volume was extracted by manually drawing regions of interest (ROI) on the entire tissue. The PET image from a mouse being injected with UB108 is shown in Figure 12, whereas the PET image from a mouse being injected with UB137 is shown in Figure 13.

### Radiochemistry Examples

**Example 128 Production of [¹⁸F]fluoride.** [¹⁸F]fluoride was produced via a (p,n)-reaction on a CTI Siemens cyclotron (Rigshospitalet, Denmark) by irradiating [¹⁸O]H₂O with 11 MeV protons. An anion exchange resin (Sep-Pak Light Waters Accell Plus QMA cartridge) was washed with EtOH (10 mL), 90 mg/mL KOTf (aq) (10 mL) and water (10 mL) and dried with air. Then the aqueous [¹⁸F]fluoride solution was passed through this exchange resin and the resin eluted with a mixture of KOTf (10 mg) and 50 ug K₂CO₃ in 550 µL water. The resulting mixture was then gently concentrated to dryness at 100 °C by azeotropic drying with 2 x ACN (0.6 mL), under a nitrogen stream for 20 min, to give no-carrier-added K[¹⁸F]F complex as a white semi-solid residue. Samples were analyzed by analytical HPLC as previously described, and radioactivity was analyzed using a flow-through GM tube based radiodetector (Scansys). Radiochemical conversion (RCC) of all radiolabeled compounds were determined by analytical HPLC by comparison of the ¹⁹F reference compounds synthesised previously. The radiochemical yield (RCY) was determined using the activity of [¹⁸F]fluoride received from the cyclotron at the beginning of the synthesis and that of the purified product and the decomposition was corrected and have been decay corrected.

**Example 129 General Tetrazine radiolabeling (manual synthesis).** The preparation of the final compound, was performed using a method described previously, with minor modifications according to the optimization results described below.^{[10]} The organotin precursor (0.01 mmol) was dissolved in 0.8 mL DMA and added 0.1 mL of stock solutions of Cu(OTf)₂ (7.2 mg, 0.02 mmol in 0.1 mL DMA) and pyridine (12 µL, 0.15 mmol in 0.1 mL DMA). This mixture was added to the dried [¹⁸F]FK and heated to 100 °C for 5 min. The mixture was cooled down before quenched with 1 mL of water. Samples were analysed via Radio-HPLC to determine the radiochemical conversion of [¹⁸F]-Tz (decay corrected).

**Example 130 General Tetrazine radiolabeling (automated synthesis).** Automated synthesis was performed on a Scansys Laboratorieteknik synthesis module. The same procedure was used as in the optimization with minor differences. A solution of the organotin precursors (0.01 mmol), Cu(OTf)₂ (7.2 mg, 0.02mmol), and pyridine (12 µL, 0.15 mmol) in 1 mL DMA was added to a reaction vial containing the dried fluoride and the reaction allowed to proceed at 100 °C for 5 minutes. The solution was then cooled to 40 °C with compressed air before quenched with 2 mL of water. The crude reaction was then purified via semi-preparative HPLC (Thermo Fisher UltiMate 3000) with a C-18 column (Luna 5 µm C18(2) 100 Å, 250 mm x 10 mm) used an isocratic method (with different H₂O:ACN solvent mixtures for each product, flowrate 4 mL/min). Identification and purity were determined by analytical HPLC.

### Example 131 Labeling of UB108 and UB137

**Production of [¹⁸F]fluoride.** [¹⁸F]fluoride was produced via a (p,n)-reaction on a CTI Siemens cyclotron (Rigshospitalet, Denmark) by irradiating [¹⁸O]H₂O with 11 MeV protons. An anion exchange resin (Sep-Pak Light Waters Accell Plus QMA cartridge) was washed with EtOH (10 mL), 90 mg/mL KOTf (aq) (10 mL) and water (10 mL) and dried with air. Then the aqueous [¹⁸F]fluoride solution was passed through this exchange resin and the resin eluted with a mixture of KOTf (10 mg) and 50 ug K₂CO₃ in 550 µL water. The resulting mixture was then gently concentrated to dryness at 100 °C by azeotropic drying with 2 x ACN (0.6 mL), under a nitrogen stream for 20 min, to give no-carrier-added K[¹⁸F]F complex as a white semi-solid residue. Samples were analyzed by analytical HPLC as previously described, and radioactivity was analyzed using a flow-through GM tube based radiodetector (Scansys).

**Example 132 Labeling of UB108 and UB137 (manual synthesis).** The preparation of the final compound was performed using a method described previously, with minor modifications according to the optimization results described below.^{[10]} The organotin precursor (0.01 mmol) was dissolved in 0.8 mL DMA and added 0.1 mL of stock solutions of Cu(OTf)₂ (7.2 mg, 0.02 mmol in 0.1 mL DMA) and pyridine (12 µL, 0.15 mmol in 0.1 mL DMA). This mixture was added to the dried [¹⁸F]FK and heated to 100 °C for 5 min. The mixture was cooled down before quenched with 1 mL of water. The reaction mixture was diluted with 10 mL of H₂O and then put through a Sep-Pak Plus 18 cartridge (SPE) preconditioned by flushing with 10 mL of EtOH followed by 10 mL of H₂O. The SPE was eluted with 3 mL of ACN into a vial containing 1 mL TFA. The mixture containing the protected product was heated during 15 minutes at 80 °C for fully deprotection. The mixture was cooled down and the sample was analysed via Radio-HPLC to determine the final radiochemical conversion of [¹⁸F]-Tz.

**Example 133 Labeling of UB-108 and UB-137 (automated synthesis for animal experiments).** Automated synthesis was performed on a Scansys Laboratorieteknik synthesis module. The same procedure was used as in manual synthesis (Example 132) with minor differences. A solution of the organotin precursors (0.01 mmol), Cu(OTf)₂ (7.2 mg, 0.02 mmol), and pyridine (12 µL, 0.15 mmol) in 1 mL DMA was added to a reaction vial containing the dried fluoride and the reaction allowed to proceed at 100 °C for 5 minutes. The solution was then cooled to 40 °C with compressed air before quenched with 3 mL of water. The reaction mixture was diluted then put through a Sep-Pak Plus 18 cartridge (SPE) preconditioned by flushing with 10 mL of EtOH followed by 10 mL of H₂O. The SPE was wash with 10 mL water and dry with air, then was eluted with 3 mL of ACN into a vial containing 1 mL TFA. The mixture containing the protected product was heated during 15 minutes at 80 °C for fully deprotection. The crude was heated to 100 °C to evaporate the ACN/TFA mixture. The solution was then cooled to 40 °C with compressed air before the addition of 3 mL of water. The crude reaction was then purified via semi-preparative HPLC (Thermo Fisher UltiMate 3000) with a C-18 column (Luna 5 µm C18(2) 100 Å, 250 mm x 10 mm) using an isocratic method (15% EtOH in water 0.1% TFA, flowrate 4 mL/min). The collected fraction from HPLC was diluted with 0.1 M phosphate buffer to pH 7.4, and the concentration of radioactivity and ethanol was adjusted by diluting with saline solution to a final concentration of 100-50 MBq/mL and ≥ 5%vol. EtOH respectively. The automated synthesis including [¹⁸F]fluoride collection, azeotropic drying, labeling, HPLC separation and formulation was carried out within 90 minutes.

### Example 134 TCO click ability.

To a solution of TCO 500 µL (1 mg/mL) PBS was added 100 uL of the formulated ¹⁸F-tetrazine (UB108/UB137). The solution was injected in the analytical HPLC to determine the fraction of tetrazines reacted to the TCO. All the tetrazines reacted immediately with the TCO (Figure 11).

### Example 135 HPLC tests

Radio-HPLC tracer of the purified compounds, following the general procedure for the automated synthesis of the tetrazines describe in Section S3, with authentic UV references overlaid are shown below. The solid red line indicates the radio-HPLC trace and the solid black line indicates the UV trace for the cold reference material. All samples were run using analytical HPLC method: Thermo Fisher UltiMate 3000 with a C-18 column (Luna 5 µm C18(2) 100 Å, 150 mm x 4.6 mm). Eluents: A, H₂O with 0.1% TFA; B, MeCN with 0.1% TFA. Gradient from 100% A to 100% B over 12 minutes, back to 100% A over 3 min, flow rate 2 mL/min. Detection by UV absorption at λ = 254 nm on a UVD 170U detector.

### Example 136 Calculation of cLogD_{7.4}

Distribution coefficient at physiological pH (7.4) were calculated in Chemicalize 2019, Chemaxaon. Values are displayed in Figure 3.

### Example 137 Synthesis of Q

Q was prepared as previously described in Rossin, R.; Verkerk, P.R.; van den Bosch, S.M.; Vulders, R.C.; Verel, I.; Lub, J.; Robillard, M.S. In vivo chemistry for pretargeted tumor imaging in live mice. Angew. Chem. Int. Ed. 2010, 49, 3375-3378

### Example 138 Rate Constant determinations

Reaction kinetics of the Tz-derivatives were determined by pseudo-first order measurements in dioxane at 25.0 ± 0.1°C or PBS at 27.0 ± 0.1°C in a SX20 stopped flow photometer (Applied Photophysics). The pseudo first order rate constant was determined by linearization of the decay curve followed by linear fitting. The second order rate constant was calculated from the pseudo-first order rate constant. Figure 3 displays the results.

### Tables

**Table 1. Product scope regarding the tetrazine scaffold, relative to the optimized radiolabeling condition of the Cu-mediated ¹⁸F-fluorination reaction.**

| | | | | | | | | |
|---|---|---|---|---|---|---|---|---|
| 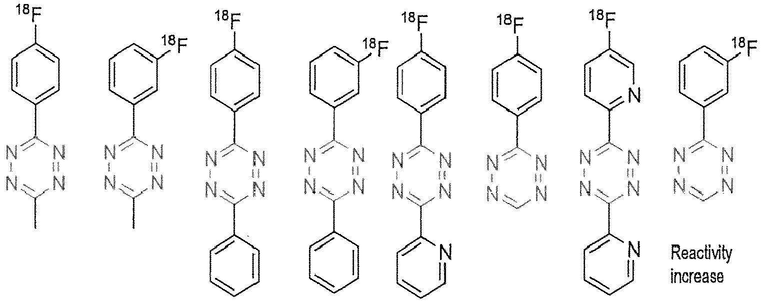 | | | | | | | | |

| | **[¹⁸F]Tz1** | **[¹⁸F]W** | **[¹⁸F]E** | **[¹⁸F]R** | **[¹⁸F]T** | **[¹⁸F]Y** | **[¹⁸F]U** | **[¹⁸F]-9** |
|---|---|---|---|---|---|---|---|---|
| RCC^{[a]} (%) | 30±5 | 28±1 | 30±5 | 31±2 | -^{[d]} | 18±4 | -^{[d]} | 11±1 |
| RCY^{[b]} (%) | 23±1 | 26±2 | 23±2 | 24±3 | -^{[d]} | | -^{[d]} | 11±3 |
| k₂ [M⁻¹s⁻¹]^{[c]} | 2.25 | nd | 18 | 30 | 106 | 159 | 206 | 222 |
| RCP^{[a]} (%) | ≥99 | ≥99 | ≥99 | ≥99 | -^{[d]} | 99 | -^{[d]} | 99 |

| | | | | | | | | |
|---|---|---|---|---|---|---|---|---|
| [a] Radiochemical conversion (RCC) and Radiochemical purity (RCP) were determined by radio-HPLC (n=3). [b] Radiochemical yield (RCY) was decay corrected to the starting amount of radioactivity received from the cyclotron and the isolated product without formulation step (n=3). [c] Second order rate constants estimated from stopped-flow measurements of the alkyne-Tz building block with TCO at 25°C in ACN. [d] No product formed or could not be isolated. | | | | | | | | |

**Table 2. Product scope in respect to the substitution pattern when including different linkers in the aromatic ring, relative to the Cu-mediated ¹⁸F-fluorination reaction.**

| | | | | | | |
|---|---|---|---|---|---|---|
| 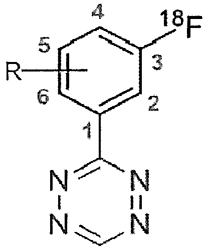 | | | | | | |

| **Substituents** | **Position** | | | **Position** | | |
|---|---|---|---|---|---|---|
| | **4** | **5** | **6** | **4** | **5** | **6** |
| -CH₃ | -^{[a]} | 14±3^{[d]} | -^{[c]} | UB-007 | UB-052 | RGV_117 |
| -OCH₃ | 4±1 | 17±3^{[d]} | -^{[c]} | UB-008 | UB-048 | RGV_116 |
| -NHCOCH₃ | -^{[a]} | 31±3^{[d]} | -^{[b]} | UB-148 | UB-150 | - |
| -CONHCH₃ | -^{[a]} | -^{[d]} | -^{[b]} | UB-198 | UB-200 | - |
| -CONH₂ | -^{[a]} | -^{[d]} | -^{[b]} | UB-22 | UB-70 | - |

| | | | | | | |
|---|---|---|---|---|---|---|
| [a] Stannate precursor could not be synthesized. [b] No tetrazine formation detected. [c] Decomposition during Cu-mediated ¹⁸F-fluorination reaction. [d] RCCs were determined by radio-HPLC (n = 3). | | | | | | |

### References

[1] J. M. Baskin, J. A. Prescher, Proceedings of the National Academy of Sciences 2007 104, 16793-16797.
[2] D. Thomae, A. Waldron, Nucl Med Biol 2014 41, 513-523.
[3] E. J. L. Steen, P. E. Edem, Biomaterials 2018**.**
[4] S. S. Gambhir, Nature Reviews Cancer 2002 2, 683-693.
[5] M. M. Herth, M. Barz, Biomacromolecules 2009 10, 1697-1703.
[6] R. Rossin, P. Renart Verkerk, Angewandte Chemie 2010 122, 3447-3450.
[7] M. M. Herth, V. L. Andersen, Chemical Communications 2013 49, 3805-3807.
[8] C. Denk, D. Svatunek, Angewandte Chemie International Edition 2014 53, 9655-9659.
[9] J. Steen, C. Denk, Journal of Nuclear Medicine 2018 59, 1144.
[10] K. J. Makaravage, A. F. Brooks, Organic Letters 2016 18, 5440-5443.
[11] I. N. Petersen, J. Villadsen, Organic & biomolecular chemistry 2017 15, 4351-4358.
[12] S. A. Albu, S. A. Al-Karmi, Bioconjugate Chemistry 2016 27, 207-216.
[13] J. Yang, M. R. Karver, Angewandte Chemie International Edition 2012 51, 5222-5225.
[14] J. W. Mclntee, C. Sundararajan, The Journal of Organic Chemistry 2008 73, 8236-8243.
[15] Yangyang Qu, FranÅois-Xavier Sauvage, AngewandteChemie 2018 130*.*
[16] M. Sun, C. Zhao, J Med Chem 2005 48, 6482-6490.
[17] I. K. Khanna, R. M. Weier, J Med Chem 1997 40, 1634-1647.
[18] M. Esfahanizadeh, K. Omidi, Iranian journal of pharmaceutical research: IJPR 2014 13, 115.
[19] Z. Zhu, J. Wang, Biomaterials science 2015 3, 842-851.
[20] M. E. Kieffer, K. V. Chuang, Chemical science 2012 3, 3170-3174.

## Claims

1. A H-tetrazine compound, having the following formula I: 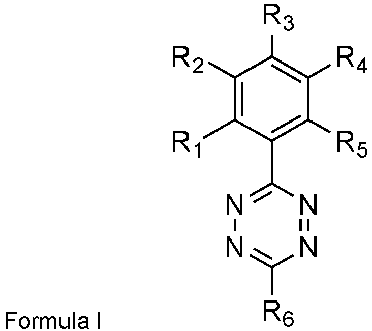 wherein R₁-R₅ are independently selected from: a radionuclide selected from ¹⁸F, ¹²³I, ¹²⁴I, ¹³¹I or ²¹¹At; one or more group(s) providing a lipophilicity of clogD_{7.4} < -0.5 to the compound of Formula I independently selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine; and
wherein R₆ is H;
wherein one of R₁-R₅ is a radionuclide and one or more of the remaining R₁-R₅ is a/are group(s) providing a lipophilicity of clogD_{7.4} < - 0,5, and any R₁-R₅ remaining thereafter is/are H.

2. A compound according to claim 1, wherein said one or more group(s) providing a lipophilicity of clogD_{7.4} < - 0,5 to the compound of Formula I; provides a lipophilicity of clogD_{7.4} < -2, such as clogD_{7.4} < -3 or such as clogD_{7.4} < -6.

3. A compound according to any of the preceding claims, wherein said one or more group(s) providing a lipophilicity of clogD_{7.4} < -0.5 to the compound of Formula I is selected from: -OH, NR₇R₈, CH₂N(CH₂COOH)₂, CH₂NHCH₂COOH, CH₂NRCH₂COOH, COOH, CONR₇R₈, SO₃H, SO₂NH₂, and SO₂NH, wherein R is H, CH₃, CH₂CH₃, CH₂CH₂CH₃ or CH₂COOH, R₇ is H, CH₃, CH₂CH₃, CH₂CH₂CH₃ or CH₂COOH; and R₈ is H, CH₃, CH₂CH₃, CH₂CH₂CH₃ or CH₂COOH.

4. A compound according to any of the preceding claims, wherein said radionuclide is situated at position R₄ in formula I.

5. A compound according to claims 1 to 3, wherein said group providing lipophilicity of clogD_{7.4} is selected from CH₂N(CH₂COOH)₂, CH₂NHCH₂COOH, CH₂NRCH₂COOH, COOH, wherein R is H, CH₃, CH₂CH₃, CH₂CH₂CH₃ or CH₂COOH, and is situated at position R₂ or R₄ in formula I; and wherein said radionuclide is situated at the other position R₂ or R₄ in Formula I.

6. A compound according to any of the preceding claims, wherein said group providing a lipophilicity of clogD_{7.4} < -0.5 to the compound of Formula I is selected from the polar groups (PG): 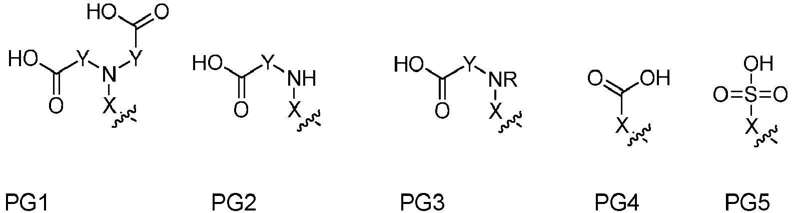 Wherein X is CO(CH₂)ₙ, SO₂(CH₂)ₙ, (CH₂)ₙ or (OCH₂CH₂)ₙ and n = 0, 1, 2 or 3; and wherein Y is (CH₂)ₙ, (OCH₂CH₂)ₙ or CO(CH₂)ₙ and n = 0, 1, 2 or 3; and wherein R is (CH₂)ₙCH₃, (OCH₂CH₂)ₙOH, CO(CH₂)ₙCOOH, (OCH₂CH₂)ₙOCH₂COOH and n = 0, 1, 2 or 3; and wherein the curly sign indicates the link to the aromatic ring.

7. A compound according to claim 1 selected from: 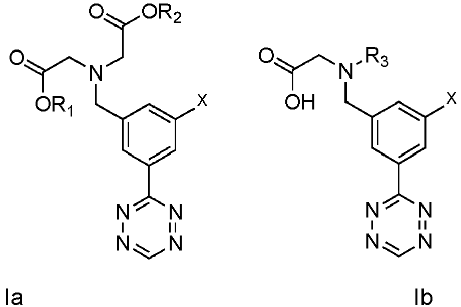 wherein X is selected from ¹⁸F, ¹²³I, ¹²⁴I, ¹³¹I or ²¹¹At, and R₁, R₂ and R₃ is independently selected from H, (CH₂)ₙCH₃, (OCH₂CH₂)ₙOH, CO(CH₂)ₙCOOH, (OCH₂CH₂)ₙOCH₂COOH and n = 0, 1, 2 or 3.

8. A tetrazine according to claim 1 selected from: 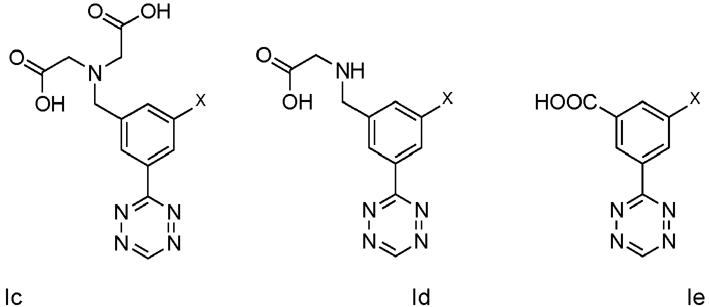 wherein X is selected from ¹⁸F, ¹²³I, ¹²⁴I, ¹³¹I or ²¹¹At.

9. A tetrazine precursor having the following formula II: 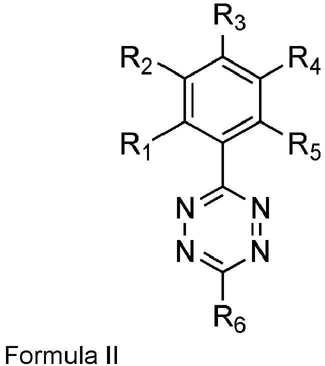 wherein R₁-R₅ are independently selected from the group consisting of: SnR₃, B(OR)₂, I⁺-Ar, I double-bonded to R or SiR₃, wherein R is a linear or branched C1-C6 alkyl, cyclic C1-C6 alkyl, optionally substituted with -OH, -NH₂ or halogen; one or more group(s) providing a lipophilicity of clogD_{7.4} < -0.5 to the compound of Formula II selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, an hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine,
and wherein R₆ is a H; and
wherein one of R₁-R₅ is selected from the group consisting of: SnR₃, B(OR)₂, I⁺-Ar, I double-bonded to R or SiR₃; and one or more of the remaining R₁-R₅ is a/are group(s) providing a lipophilicity of clogD_{7.4} < - 0,5; and any R₁-R₅ remaining thereafter is/are H.

10. A tetrazine precursor according to claim 9, wherein one of R₁-R₅ is selected from SnR₃ or B(OR)₂; and one or more of the remaining R₁-R₅ is a/are group(s) providing a lipophilicity of clogD_{7.4} < - 0,5; and any R₁-R₅ remaining thereafter is/are H.

11. A tetrazine precursor according to claim 9, wherein one of R₁-R₅ is selected from SnR₃ or SiR₃ wherein R is a linear methyl, ethyl, propyl or butyl and all R's are the same.

12. A tetrazine precursor according to claims 9 or 11, wherein one of R₁-R₅ is SiR₃ and wherein the radionuclide in the final compound of formula I provided by this precursor is ²¹¹At.

13. A tetrazine precursor according to claim 9, wherein one of R₁-R₅ is I⁺-Ar, I double-bonded to R, and wherein the radionuclide in the final compound of formula I provided by this precursor is ¹⁸F.

14. A tetrazine precursor according to any of claims 9 to 13 wherein said group selected from SnR₃, B(OR)₂, I⁺-Ar, I double-bonded to R or SiR₃, is situated at position R₄ in Formula II and one or more of the remaining R₁-R₅ is a/are group(s) providing a lipophilicity of clogD_{7.4} < - 0,5; and any R₁-R₅ remaining thereafter is/are H.

15. A tetrazine precursor according to claim 9 selected from: 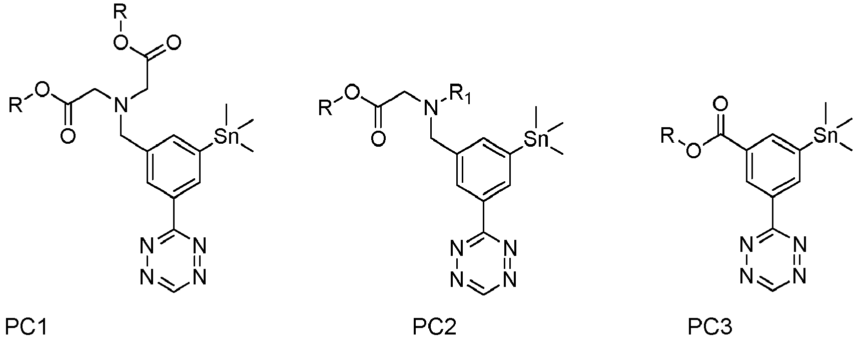 wherein the protective group R is selected from butyl groups, methylthiomethyl (MTM) groups, tetrahydropyranyl (THP) groups or benzyloxymethyl (BOM) esters, and R₁ is selected from a Boc group, trityl group, acetamide, carbamate group, (CH₂)ₙCH₃, (OCH₂CH₂)ₙOH, CO(CH₂)ₙCOOtBu, (CH₂CH₂O)ₙCH₂CO(CH₂)ₙCOOtBu wherein n = 0, 1, 2 or 3.

16. A tetrazine precursor according to claim 9 selected from: 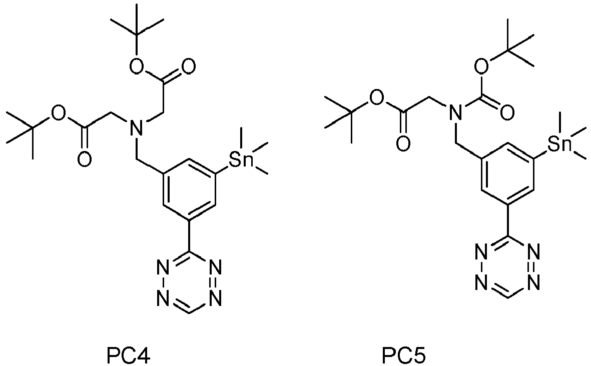 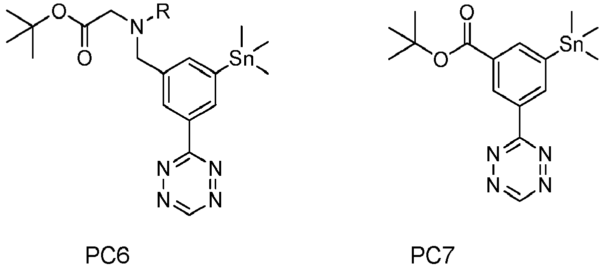 wherein R is selected from a Boc group, trityl group, acetamide, carbamate_group, (CH₂)ₙCH₃, (OCH₂CH₂)ₙOH, CO(CH₂)ₙCOOtBu, or (CH₂CH₂O)ₙCH₂CO(CH₂)ₙCOOtBu wherein n = 0, 1, 2 or 3.

17. A compound as defined in any of claims 1-8 for use in bioorthogonal chemistry.

18. A compound as defined in any of claims 1-8 for use in diagnostics.

19. A compound according to claim 18, wherein said diagnostics is cancer diagnostics

20. A compound as defined in any of claims 1-8 wherein the radionuclide is ¹⁸F or ¹²⁴I use in PET imagining.

21. A compound as defined in any of claims 1-8 wherein the radionuclide is ¹²³I for use in SPECT imagining.

22. A compound for use as defined in any of claims 20-21, wherein the imaging is of cancer tissue

23. A compound as defined in any of claims 1-8 wherein the radionuclide is ²¹¹At or ¹³¹I for use in radionuclide therapy.

24. A compound for use as defined in claim 23, wherein the radionuclide therapy is of a cancer disease.

25. A compound for use as defined in claim 24, wherein the radionuclide therapy is applied to target vectors which do not internalize, such as pathogen targets and/or cells infected with pathogens.

26. A compound for use as defined in any of claims 17-25, wherein the compound does not penetrate cell membranes.

27. benzonitrile to the corresponding tetrazine.

28. A method according to claim 27 comprising reacting 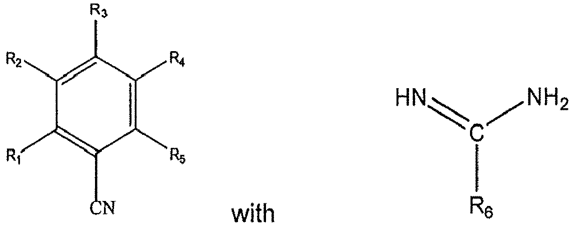 wherein R₁-R₅ are independently selected from the group consisting of: SnR₃, B(OR)₂, I⁺-Ar, I double-bonded to R or SiR₃, wherein R is a linear or branched C1-C6 alkyl, cyclic C1-C6 alkyl, optionally substituted with -OH, -NH₂ or halogen; one or more group(s) providing a lipophilicity of clogD_{7.4} < -0.5 to the compound of Formula II selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, an hydroxy group, a sulfonamide, a carboxyl group, a sulfonyl group, amine, an substituted amine; with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine,
and wherein R6 is a H;
and wherein one of R₁-R₅ is selected from: SnR₃, B(OR)₂, I⁺-Ar, I double-bonded to R or SiR₃; and one or more of the remaining R₁-R₅ is a/are group(s) providing a lipophilicity of clogD_{7.4} < - 0,5; and any R₁-R₅ remaining thereafter is/are H.

29. A method according to claim 27 comprising reacting 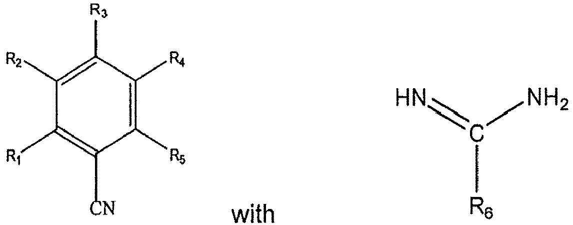 wherein R₁-R₅ are independently selected from I or F and one or more group(s) providing a lipophilicity of clogD_{7.4} < -0.5 to the compound of Formula II, selected from the group consisting of a hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-C10)cycloalkyl, (C3-C10)heterocycloalkyl, (C3-C10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, an hydroxy group, a sulfonamide, a carboxyl group, a sulfonyl group, amine, a substituted amine with 1-5 polyethylene glycol unit(s), a -(O-CH₂-CH₂)₁₋₅-OCH₂-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine,
and wherein R6 is a H,
and wherein one of R₁-R₅ is selected from: I or F; and one or more of the remaining R₁-R₅ is a/are group(s) providing a lipophilicity of clogD_{7.4} < - 0,5; and any R₁-R₅ remaining thereafter is/are H.

30. A method according to claim 28 or 29, wherein the reaction is carried out at a temperature range of from 50 to 70 °C.

31. A method according to any of claims 28 to 30, wherein water is added after cooling to room temperature followed by addition of HCI and extraction with EtOAc.
